(19) Europäisches Patentamt
European Patent Office
Office européen des brevets

(11) EP 0 537 742 B1

(12) EUROPEAN PATENT SPECIFICATION

(45) Date of publication and mention of the grant of the patent:
**21.08.1996 Bulletin 1996/34**

(21) Application number: **92117632.7**

(22) Date of filing: **15.10.1992**

(51) Int. Cl.$^6$: **C07C 255/36**, C07C 255/41, C07C 317/32, A61K 31/275, C07C 255/40, C07C 255/42, C07C 255/43, C07C 255/34, C07C 255/35

# (54) Styrene derivatives

Styrol-Derivate

Dérivés de styrène

(84) Designated Contracting States:
**DE ES FR GB IT**

(30) Priority: **15.10.1991 JP 266461/91**
**05.10.1992 JP 266027/92**

(43) Date of publication of application:
**21.04.1993 Bulletin 1993/16**

(73) Proprietor: **Mitsubishi Chemical Corporation**
**Chiyoda-ku Tokyo (JP)**

(72) Inventors:
- **Kitano, Yasunori**
**Yokohama-shi, Kanagawa-ken (JP)**
- **Takayanagi, Hisao**
**Yamato-shi, Kanagawa-ken (JP)**
- **Sugawara, Koichi**
**Yokohama-shi, Kanagawa-ken (JP)**
- **Hara, Hiroto**
**Machida-shi, Tokyo-to (JP)**
- **Nakamura, Hideo**
**Suginami-ku, Tokyo-to (JP)**
- **Oshino, Toshiko**
**Kawasaki-shi, Kanagawa-ken (JP)**

(74) Representative: **Hansen, Bernd, Dr. Dipl.-Chem. et al**
**Hoffmann, Eitle & Partner**
**Patent- und Rechtsanwälte,**
**Postfach 81 04 20**
**81904 München (DE)**

(56) References cited:
**EP-A- 0 322 738**
**EP-A- 0 323 631**
**EP-A- 0 426 468**

- **CHEMICAL ABSTRACTS, vol. 69, 1968, Columbus, Ohio, US; abstract no. 106149v,**
- **JOURNAL OF MEDICINAL CHEMISTRY vol. 34, no. 6, 1991, pages 1896 - 1907 A. GAZIT ET AL**
- **JOURNAL OF MEDICINAL CHEMISTRY vol. 32, no. 10, 1989, pages 2344 - 2352 A. GAZIT ET AL**
- **CHEMICAL & PHARMACEUTICAL BULLETIN vol. 36, 1988, pages 974 - 981 T. SHIRAISHI ET AL**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 537 742 B1

Printed by Rank Xerox (UK) Business Services
2.13.3/3.4

## Description

The present invention relates to novel styrene derivatives. In more particular, it relates to styrene derivatives having an inhibitory activity on tyrosine-specific protein kinase (hereinafter referred to as "tyrosine kinase") and also inhibitory activity on cancer cell proliferation.

A lot of substances have been used for cancer chemotherapy, but in most cases they are not necessarily satisfactory, since they exhibit unsatisfactory pharmacological effect, and their inhibitory activity is not limited to cancer cells, which causes great side effects.

It is known that the receptor of proliferation factor controls differentiation and proliferation of cells and that a certain abnormal event causes abnormal proliferation and differentiation of cells which results in canceration. Above all, it has been evident that tyrosine kinase type receptors have big participation in formation of cancer. It was found that these receptors show particular protein kinase activity specific to tyrosine and that this activity is great especially in cancer cells. On the basis of these findings, it has been proposed that the drugs which can inhibit specifically the tyrosine kinase activity of proliferation factor receptor would be anticancer agents having novel mechanism and less side effects. Examples of such anticancer agents derived from microorganisms are Erbstatin, Lavendustin, Herbimycin A, Genistein and the like, and examples of synthesized agents are benzylidenemalonitrile derivatives (Japanese Patent Publication (not examined) No. 138238/1990, Journal of Medicinal Chemistry, 32, 2344 (1989); ibid. 34, 1896 (1991)), α-cyanocinnamide derivatives [Japanese Patent Publication (not examined) No. 22153/1988], 3, 5-diisopropyl-4-hydroxystrene derivatives [Japanese Patent Publication (not examined) No. 39522/1987], 3, 5-di-t-butyl-4-hydroxystyrene derivatives [Japanese Patent Publication (not examined) No. 39523/1987], Erbstatin analogs [Japanese Patent Publication (not examined) No. 277347/1987] and the like.

EP-A-0 426 468 discloses that N,N-diethyl-2-cyano-3-(3,4-dihydroxy-5-nitrophenyl)-acrylamide is a potent inhibitor of catechol O-methyl transferase and may be used pharmaceutically in the treatment of Parkinson disease.

A short summary in the Chemical Abstracts, Vol. 69, 106149v (1968) discloses the structure of several 5-[2-cyano-2-[N-(chlorophenyl)carbamoyl]vinyl)-salicylic acids/esters

Known tyrosine kinase inhibitors have all insufficient inhibition and cannot be satisfactorily used as anticancer agents. It is an object of the present invention to provide novel anticancers which are easily available, which are specifically and highly active as tyrosine kinase inhibitors of proliferation factor receptor, and which have less side effects accordingly.

In view of the fact that there is an interaction between the phenolic hydroxy group and tyrosine kinase active site of said enzyme during the process of phosphorylation of tyrosine residue of protein and that there is a nucleophilic attack of the phenolic hydroxy group to adenosine triphosphate, the present inventors have presumed that introduction of a substituent, in particular electrophilic substitutent, near the phenol (or catechol) moiety of tyrosine derivatives would increase the acidity of phenol (catechol) moiety in comparison with original tyrosine derivatives and would simultaneously inhibit the nucleophilic attack, thereby enhancing inhibition of the enzyme activity. Based on such presumption, the inventors have made extensive study, and found a series of particular compounds having potent enzyme inhibition as never seen before. The present invention is based on such finding. Thus, the present invention provides styrene derivatives of the following general formula (I):

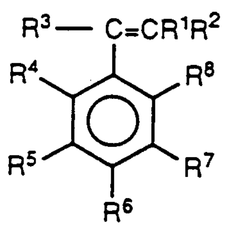

(I)

wherein, $R^1$ and $R^2$ independently each represent cyano group, $CONR^9R^{10}$ [wherein $R^9$ and $R^{10}$ independently each represent hydrogen atom, $C_1$ - $C_5$ alkyl group or

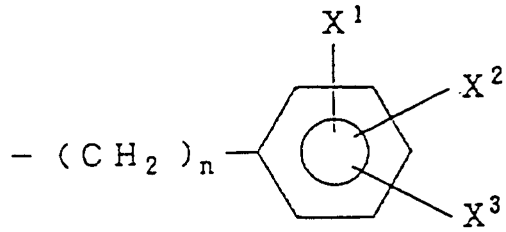

(in which n represents 0 or an integer of from 1 to 5, and $X^1$, $X^2$ and $X^3$ independently each represent hydrogen atom, halogen atom, $C_1$ - $C_5$ alkyl group, $C_1$ - $C_5$ alkoxy group, hydroxy group, nitro group or cyano group) with the proviso that $R^9$ and $R^{10}$ don't represent hydrogen atom at a time] or $-COR^{11}$ [wherein $R^{11}$ represents

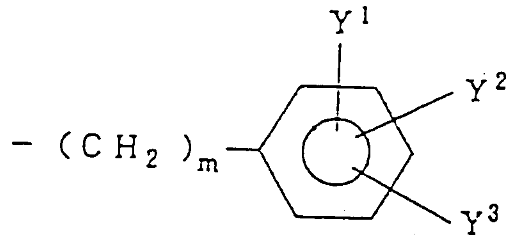

(in which m represents 0 or an integer of from 1 to 5, and $Y^1$, $Y^2$ and $Y^3$ independently each represent hydrogen atom, $C_1$ - $C_5$ alkyl group, $C_1$ - $C_5$ alkoxy group, hydroxy group, or halogen atom)] but $R^1$ and $R^2$ don't represent cyano group at a time;

$R^3$ represents hydrogen atom, $C_1$ - $C_5$ alkyl group or hydroxy group,

$R^4$, $R^5$, $R^6$, $R^7$ and $R^8$ independently each represent hydrogen atom, hydroxy group, halogen atom, $C_1$ - $C_5$ alkoxy group, nitro group, cyano group;

$-NR^{12}R^{13}$ [wherein $R^{12}$ and $R^{13}$ independently each represent hydrogen atom, $C_1$ - $C_5$ alkyl group or benzoyl group];

$-SOpR^{14}$ [wherein p represents 0, 1 or 2, and $R^{14}$ represents $C_1$ - $C_5$ alkyl group or phenyl group];

$-COR^{15}$ [wherein $R^{15}$ represents hydrogen atom, $C_1$ - $C_5$ alkyl group, $C_1$ - $C_5$ alkoxy group, hydroxy group, phenyl group, phenoxy group or $-NHR^{16}$ (in which $R^{16}$ represents $C_1$ - $C_5$ alkyl group or phenyl group)] or $C_1$ - $C_5$ alkyl group optionally substituted by halogen atom,

with the first proviso that at least one of $R^4$ to $R^8$ is hydroxy group, and that when $R^3$ is hydrogen atom and $R^4$ to $R^8$ independently each represent hydrogen atom, hydroxy group, $C_1$ - $C_5$ alkoxy group, $-NR^{12}R^{13}$ (in which $R^{12}$ and $R^{13}$ are as defined above), $C_1$ - $C_5$ alkyl group, then $R^1$ represents cyano group, $R^2$ represents $-CONR^9R^{10}$ [wherein $R^9$ represents hydrogen atom or $C_1$ - $C_5$ alkyl group, $R^{10}$ represents

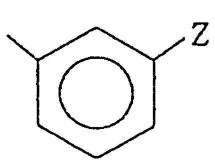

or

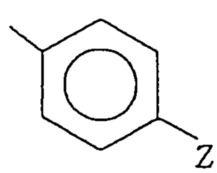

(in which Z represents $C_1$ - $C_5$ alkyl group, halogen atom, nitro group or cyano group)]

or $-COR^{11}$ [wherein $R^{11}$ represents

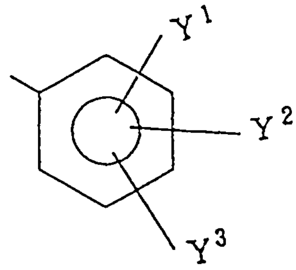

(in which $Y^1$, $Y^2$ and $Y^3$ are as defined above), provided that $R^{11}$ is not

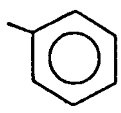
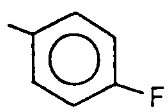
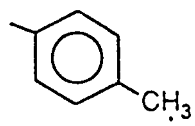
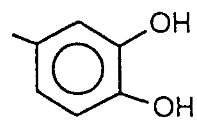

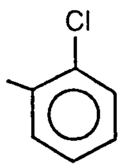

and

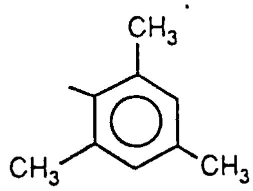

]]

with the second proviso that when $R^4$, $R^7$ and $R^8$ are H, $R^6$ is hydroxy, $R^5$ is $COR^{15}$, wherein $R^{15}$ represents a hydroxy group or $C_1$ to $C_5$ alkoxy group, $R^3$ is hydrogen, $R^1$ is cyano and $R^2$ is $-CONR^9R^{10}$ wherein $R^9$ is hydrogen, then $R^{10}$ is not

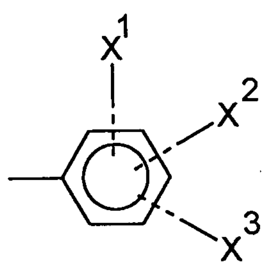

in which $X^1$, $X^2$ and $X^3$ independently represent chlorine or $C_1$ to $C_5$ alkyl group,

and with the third proviso that the styrene of the formula I is not N,N-Diethyl-2-cyano-3-(3,4-dihydroxy-5-nitrophenyl)-acrylamide or pharmaceutically acceptable salts thereof.

The compounds of the present invention will be defined in more detail. Thus, the invention provides styrene derivatives of the following general formula (I)

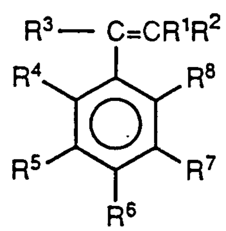

(I)

wherein, $R^1$ and $R^2$ represent cyano group, $-CONR^9R^{10}$ [wherein $R^9$ and $R^{10}$ independently each represent hydrogen atom, $C_1$ - $C_5$ alkyl group (e.g. methyl group, propyl group, pentyl group, etc.) or

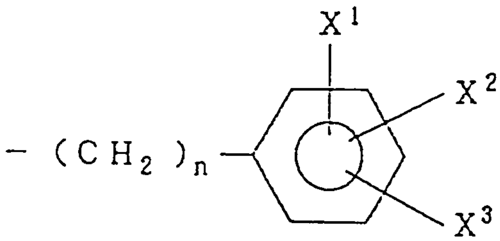

(in which n is 0 or an integer of from 1 to 5, and $X^1$, $X^2$ and $X^3$ independently each represent hydrogen atom, halogen atom (e.g, fluorine atom, chlorine atom, bromine atom, iodine atom, etc.), $C_1$ - $C_5$ alkyl group (e.g. methyl group, propyl group, pentyl group, etc.), $C_1$ - $C_5$ alkoxy group (e.g. methoxy group, propoxy group, pentyloxy group, etc.), hydroxy group, nitro group or cyano group), with the proviso that $R^9$ and $R^{10}$ don't represent hydrogen atom at a time] or $-COR^{11}$ [wherein $R^{11}$ represents

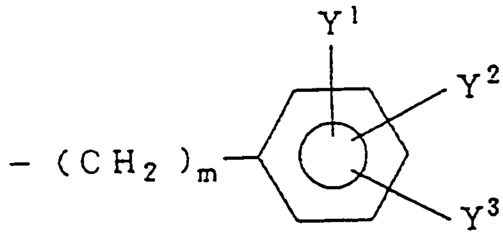

(in which m represents 0 or an integer of 1 to 5, and $Y^1$, $Y^2$ and $Y^3$ independently each represents hydrogen atom, $C_1$ - $C_5$ alkoxy group (e.g. methoxy group, propoxy group, pentyloxy group, etc.), hydroxy group, $C_1$ - $C_5$ alkyl group (methyl group, propyl group, pentyl group, etc.) or halogen atom (e.g. fluorine atom, chlorine atom, bromine atom, iodine atom etc.)] with the proviso that $R^1$ and $R^2$ don't represent cyano group at a time;

$R^3$ represents hydrogen atom, $C_1$ - $C_5$ alkyl group (e.g. methyl group, propyl group, pentyl group, etc.) or hydroxy group;

$R^4$, $R^5$, $R^6$, $R^7$ and $R^8$ independently each represent hydrogen atom, hydroxy group, halogen atom (e.g. fluorine atom, chlorine atom, bromine atom, iodine atom, etc.), $C_1$ - $C_5$ alkoxy group (e.g. methoxy group, propoxy group, pentyloxy group, etc.), nitro group, cyano group;

$-NR^{12}R^{13}$ [wherein $R^{12}$ and $R^{13}$ independently each represent hydrogen atom, $C_1$ - $C_5$ alkyl group (e.g. methyl group, propyl group, pentyl group, etc.) or benzoyl group];

$-SOpR^{14}$ [wherein p represents 0, 1 or 2, and $R^{14}$ represents $C_1$ - $C_5$ alkyl group (e.g. methyl group, propyl group, pentyl group, etc.) or phenyl group];

$-COR^{15}$ [wherein $R^{15}$ represents hydrogen atom, $C_1$ - $C_5$ alkyl group (e.g. methyl group, propyl group, pentyl group, etc.), $C_1$ - $C_5$ alkoxy group (e.g. methoxy group, propoxy group, pentyloxy group, etc.), hydroxy group, phenyl group, phenoxy group or $-NHR^{16}$ (in which $R^{16}$ represents $C_1$ - $C_5$ alkyl group (e.g. methyl group, propyl group, pentyl group, etc.) or phenyl group)] or $C_1$ - $C_5$ alkyl group (e.g. methyl group, propyl group, pentyl group, etc.) optionally substituted by halogen atom (e.g. fluorine atom, chlorine atom, bromine atom, iodine atom, etc.), with the proviso that at least one of $R^4$ to $R^8$ is hydroxy group, and that, when $R^3$ is hydrogen atom and $R^4$ to $R^8$ each represent hydrogen atom, hydroxy group, $C_1$ - $C_5$ alkoxy group (e.g. methoxy group, propoxy group, pentyloxy group, etc.) $-NR^{12}R^{13}$ (in which $R^{12}$ and $R^{13}$ are as defined above) or unsubstituted $C_1$ - $C_5$ alkyl group (e.g. methyl group, propyl group, pentyl group, etc.), then $R^1$ represents cyano group, $R^2$ represents $-CONR^9R^{10}$ [wherein $R^9$ represents hydrogen atom or $C_1$ - $C_5$ alkyl group (e.g. methyl group, propyl group, pentyl group, etc.), and $R^{10}$ represents

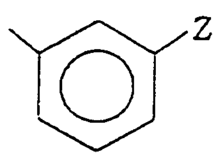

or

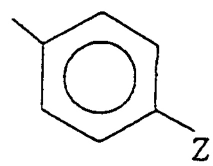

(in which Z represents $C_1$ - $C_5$ alkyl group (e.g. methyl group, propyl group, pentyl group, etc.), halogen atom (e.g. fluorine atom, chlorine atom, bromine atom, iodine atom, etc.), nitro group or cyano group.)]

or $-COR^{11}$ [wherein $R^{11}$ represents

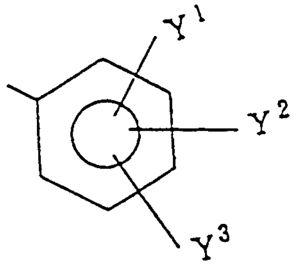

(in which $Y^1$, $Y^2$ and $Y^3$ are as defined above), provided that $R^{11}$ is not

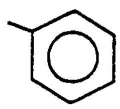
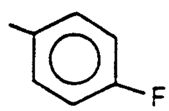
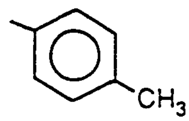
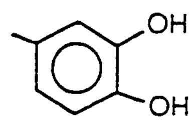

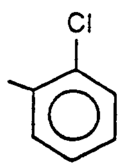
and
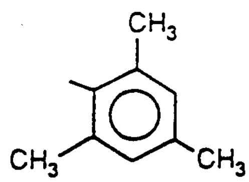

or pharmaceutically acceptable salts thereof.

Preferred are the compounds of the general formula (I) in which $R^1$ represents cyano group, $R^2$ represents $-CONR^9R^{10}$ [wherein $R^9$ represents hydrogen atom or $C_1$ - $C_5$ alkyl group, $R^{10}$ represents

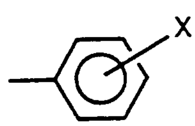

(in which X represents hydrogen atom, halogen atom, $C_1$ - $C_5$ alkyl group, nitro group or cyano group)] or $-COR^{11}$ [wherein $R^{11}$ represents

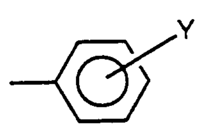

(in which Y represents hydrogen atom or halogen atom)],

$R^3$ represents hydrogen atom, $C_1$ - $C_5$ alkyl group or hydroxy group, $R^4$, $R^5$, $R^6$, $R^7$ and $R^8$ independently each represents hydrogen atom, hydroxy group, halogen atom, $C_1$ - $C_5$ alkoxy group, nitro group, cyano group, $-SO_2R^{14}$ (in which $R^{14}$ represents $C_1$ - $C_5$ alkyl group), carboxy group or $C_1$ - $C_5$ alkyl group optionally substituted by halogen atom, provided that at least one of $R^4$ to $R^8$ is hydroxy group and that when $R^3$ is hydrogen atom and $R^4$ to $R^5$ independently each represent hydrogen atom, hydroxy group, $C_1$ - $C_5$ alkyl group or unsubstituted $C_1$ - $C_5$ alkyl group, then $R^2$ represents $-CONR^9R^{10}$

[wherein $R^9$ represents hydrogen atom and $R^{10}$ represents

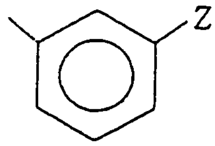

or

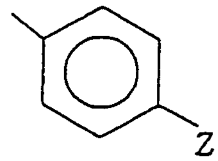

(in which Z represents $C_1$ - $C_5$ alkyl group, halogen atom, nitro group or cyano group)] or -$COR^{11}$ [wherein $R^{11}$ represents

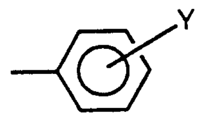

(in which Y represents halogen atom) provided that $R^{11}$ is not

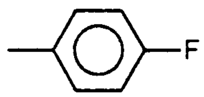

and

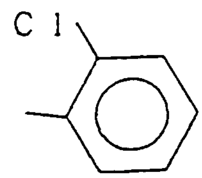

More preferable are the compounds of the general formula (I) above, in which $R^1$ represents cyano group, $R^2$ represents -$CONR^9R^{10}$ [wherein $R^9$ represents hydrogen atom and $R^{10}$ represents

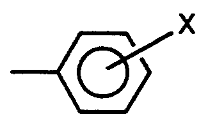

(in which X represents hydrogen atom, halogen atom, $C_1$ - $C_5$ alkyl group, nitro group or cyano group)] or -$COR^{11}$ [wherein $R^{11}$ represents

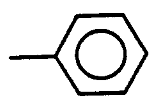

$R^3$ represents hydrogen atom or hydroxy group, $R^4$ and $R^8$ independently each represent hydrogen atom, halogen atom, or nitro group, $R^5$ and $R^7$ independently each represent hydrogen atom, hydroxy group, halogen atom, $C_1$ - $C_5$ alkoxy group, nitro group, cyano group, $SO_2R^{14}$ (in which $R^{14}$ represents $C_1$ - $C_5$ alkyl group), carboxy group or $C_1$ - $C_5$ alkyl group optionally substituted by halogen atom and $R^6$ represents hydroxy group with the proviso that when $R^3$, $R^4$ and $R^8$ are hydrogen atom and $R^5$ and $R^7$ independently each hydroxy group, $C_1$ - $C_5$ alkoxy group or $C_1$ - $C_5$ alkyl group, then $R^2$ represents -$CONR^9R^{10}$ [wherein $R^9$ represents hydrogen atom and $R^{10}$ represents

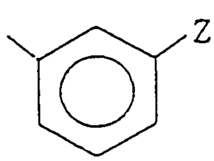

or

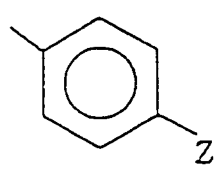

(in which Z represents $C_1$ - $C_5$ alkyl group, halogen atom, nitro group or cyano group)].

Particularly preferable are the compounds of the general formula (I) in which $R^1$ represents cyano group, $R^2$ represents -$CONR^9R^{10}$ [wherein $R^9$ represents hydrogen atom and $R^{10}$ represents

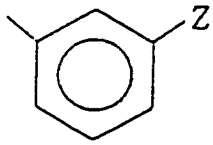

(in which Z represents hydrogen atom or halogen atom)], $R^3$ represents hydrogen atom or hydroxy group, $R^4$ represents hydrogen atom, $R^5$ represents hydroxy group, halogen atom, $C_1$ - $C_5$ alkoxy group or $C_1$ - $C_5$ alkyl group, $R^6$ represents hydroxy group, $R^7$ represents hydrogen atom, halogen atom, cyano group or $C_1$ - $C_5$ alkyl group and $R^8$ represents hydrogen atom, halogen atom or nitro group with the proviso that when $R^3$ and $R^8$ represent hydrogen atom, $R^5$ represents hydroxy group, $C_1$ - $C_5$ alkoxy group or $C_1$ - $C_5$ alkyl group, and $R^7$ represents hydrogen atom or $C_1$ - $C_5$ alkyl group, then Z represents halogen atom.

Table 1 exemplifies practical embodiments of the compounds of the present invention.

Table 1

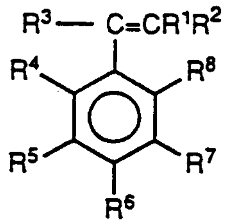

(I)

| Compd. No. | $R^1$ | $R^2$ | $R^3$ | $R^4$ | $R^5$ | $R^6$ | $R^7$ | $R^8$ |
|---|---|---|---|---|---|---|---|---|
| 1 | -CN | -CONHPh | -H | -H | -OMe | -OH | -Cl | -H |
| 2 | -CN | -CONHPh | -H | -H | -OH | -OH | -Cl | -H |
| 3 | -CN | 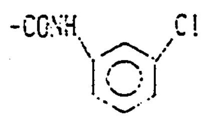 | -H | -H | -OMe | -OH | -Cl | -H |
| 4 | -CN | 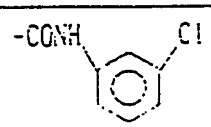 | -H | -H | -OH | -OH | -Cl | -H |
| 5 | -CN | 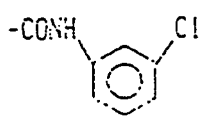 | -H | -H | -OMe | -OH | -Br | -H |
| 6 | -CN | 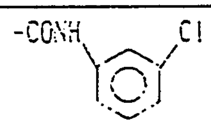 | -H | -H | -OH | -OH | -Br | -H |
| 7 | -CN | 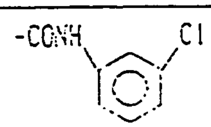 | -H | -H | -OMe | -OH | -F | -H |
| 8 | -CN | 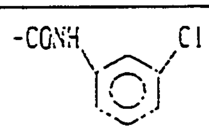 | -H | -H | -OH | -OH | -F | -H |

Table 1 (continued)

| Compd. No. | $R^1$ | $R^2$ | $R^3$ | $R^4$ | $R^5$ | $R^6$ | $R^7$ | $R^8$ |
|---|---|---|---|---|---|---|---|---|
| 9 | -CN | 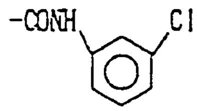 | -H | -H | -OH | -OH | -Br | -Br |
| 10 | -CN | 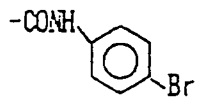 | -H | -H | -OH | -OH | -H | -H |
| 11 | -CN | 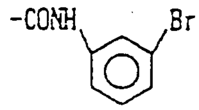 | -H | -H | -OH | -OH | -H | -H |
| 12 | -CN | 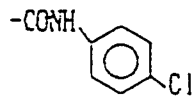 | -H | -H | -OH | -OH | -H | -H |
| 13 | -CN | 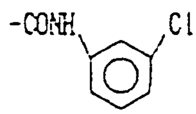 | -H | -H | -OH | -OH | -H | -H |
| 14 | -CN | 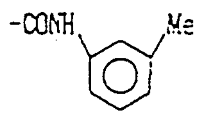 | -H | -H | -OH | -OH | -H | -H |
| 15 | -CN | 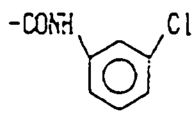 | -H | -H | -OH | -OH | -CN | -H |
| 16 | -CN | 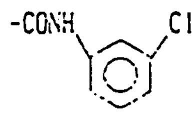 | -H | -H | -OH | -OH | -H | $-NO_2$ |
| 17 | -CN | 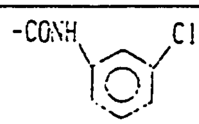 | -H | -H | -OMe | -OH | $-SO_2Me$ | -H |
| 18 | -CN | -COPh | -H | -H | -OH | -OH | -Br | -Br |
| 19 | -CN | -CONHPh | -H | -H | -OH | -OH | -I | -H |

Table 1 (continued)

| Compd. No. | $R^1$ | $R^2$ | $R^3$ | $R^4$ | $R^5$ | $R^6$ | $R^7$ | $R^8$ |
|---|---|---|---|---|---|---|---|---|
| 20 | -CN | 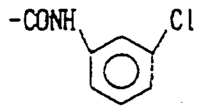 | -H | -F | -OH | -OH | -F | -H |
| 21 | -CN | 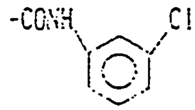 | -H | -Cl | -OH | -OH | -Cl | -H |
| 22 | -CN | 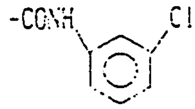 | -H | -Cl | -OH | -OH | -Cl | -Cl |
| 23 | -CN | 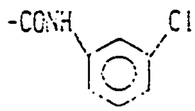 | -H | -H | -Me | -OH | -Me | -H |
| 24 | -CN | 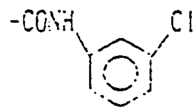 | -H | -tBu | -OH | -tBu | -F | -H |
| 25 | -CN | 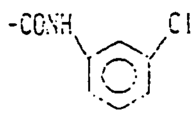 | -H | -OH | -OH | -H | $-CF_3$ | -H |
| 26 | -CN | 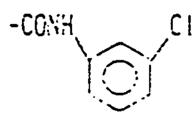 | -H | -H | -OH | -OH | -OH | -H |
| 27 | -CN | 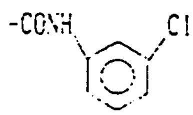 | -H | -H | -OH | -OH | -OH | -Cl |
| 28 | -CN | 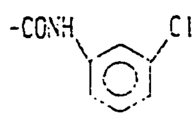 | -H | -H | -OH | -OH | -NHCOPh | -H |
| 29 | -CN | 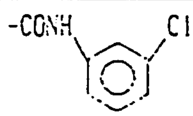 | -H | -H | -OH | -OH | $-SO_2Ph$ | -H |
| 30 | -CN | 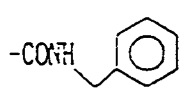 | -H | -H | -OH | -OH | -Cl | -H |

Table 1 (continued)

| Compd. No. | $R^1$ | $R^2$ | $R^3$ | $R^4$ | $R^5$ | $R^6$ | $R^7$ | $R^8$ |
|---|---|---|---|---|---|---|---|---|
| 31 | -CN | 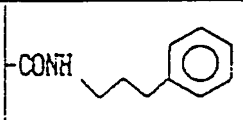 | -H | -H | -OH | -OH | -Cl | -H |
| 32 | -CN | 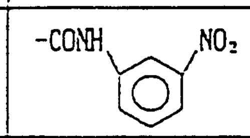 | -H | -H | -OH | -OH | -Cl | -H |
| 33 | -CN | 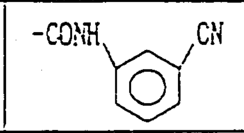 | -H | -H | -OH | -OH | -Cl | -H |
| 34 | -CN | 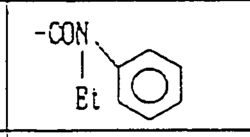 | -H | -H | -OH | -OH | -Cl | -H |
| 35 | -CN | 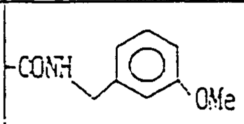 | -H | -H | -OH | -OH | -Cl | -H |
| 36 | -CN | -CONHPr | -H | -H | -OH | -OH | -Cl | -H |
| 37 | -CN | 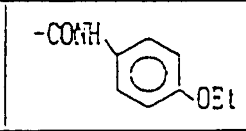 | -H | -H | -OH | -OH | -Cl | -H |
| 38 | -CN | 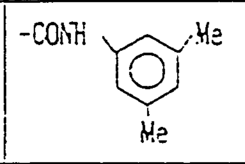 | -H | -H | -OH | -OH | -Cl | -H |
| 39 | -CN | 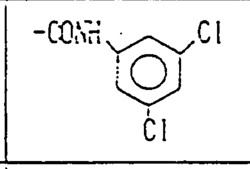 | -H | -H | -OH | -OH | -Cl | -H |
| 40 | -CN | 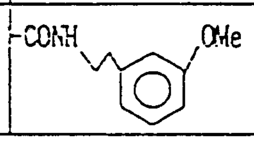 | -H | -H | -OH | -OH | -Br | -Br |

Table 1 (continued)

| Compd. No. | $R^1$ | $R^2$ | $R^3$ | $R^4$ | $R^5$ | $R^6$ | $R^7$ | $R^8$ |
|---|---|---|---|---|---|---|---|---|
| 41 | -CN | 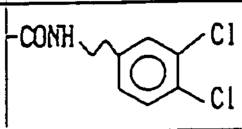 | -H | -H | -OH | -OH | -Cl | -H |
| 42 | -CN | 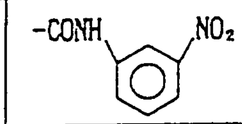 | -H | -H | -OH | -OH | -H | -H |
| 43 | -CN | 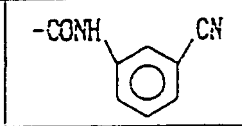 | -H | -H | -OH | -OH | -H | -H |
| 44 | -CN | 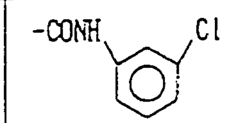 | -H | -H | -OH | -OH | -COPh | -H |
| 45 | -CN | 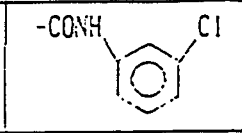 | -H | -H | -OH | -OH | $-CO_2Me$ | -H |
| 46 | -CN | 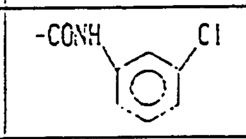 | -H | -H | -OH | -OH | -CONHPh | -H |
| 47 | -CN | 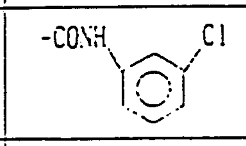 | -H | -Cl | -OH | -OH | -CONHPh | -H |
| 48 | -CONHPh | -CONHPh | -H | -H | -OH | -OH | -CONHPh | -H |
| 49 | 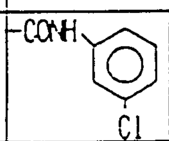 | 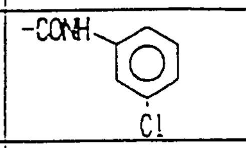 | -H | -H | -OH | -OH | -Cl | -H |
| 50 | 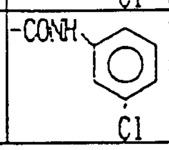 | -CN | -H | -H | -OH | -OH | -Cl | -H |

Table 1 (continued)

| Compd. No. | $R^1$ | $R^2$ | $R^3$ | $R^4$ | $R^5$ | $R^6$ | $R^7$ | $R^8$ |
|---|---|---|---|---|---|---|---|---|
| 51 | 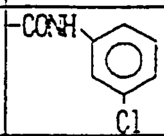 | -CN | -H | -H | -OH | -OH | -Br | -Br |
| 52 | -COPh | -COPh | -H | -H | -OH | -OH | -Cl | -H |
| 53 | -CN | -COPh | -H | -H | -OH | -OH | -Cl | -H |
| 54 | -CN | 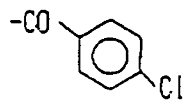 | -H | -H | -OH | -OH | -Cl | -H |
| 55 | -CN | 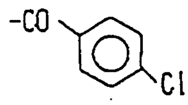 | -H | -H | -OH | -OH | -Br | -Br |
| 56 | -CN | 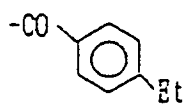 | -H | -H | -OH | -OH | -Br | -H |
| 57 | -CN | 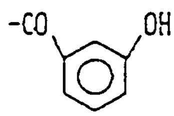 | -H | -H | -OH | -OH | -Cl | -H |
| 58 | -CN | 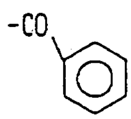 | -H | -F | -OH | -OH | -F | -H |
| 59 | -CN | 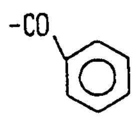 | -H | $-NO_2$ | -OH | -OH | -Br | -H |
| 60 | -CN | 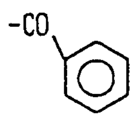 | -H | -H | -Me | -OH | -Me | -Cl |

Table 1 (continued)

| Compd. No. | $R^1$ | $R^2$ | $R^3$ | $R^4$ | $R^5$ | $R^6$ | $R^7$ | $R^8$ |
|---|---|---|---|---|---|---|---|---|
| 61 | -CN | 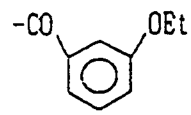 | -H | -H | -OH | -OH | -Cl | -Cl |
| 62 | -CN | 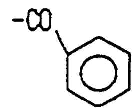 | -Me | -H | -OH | -OH | -Cl | -Cl |
| 63 | -CN | 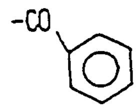 | -Bu | -H | -OH | -OH | -Cl | -Cl |
| 64 | -CN | 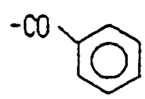 | -OH | -H | -OH | -OH | -Cl | -Cl |
| 65 | -CN | 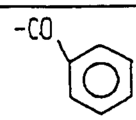 | -Me | -OMe | -OH | -OH | -Cl | -Cl |
| 66 | -CN | 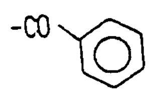 | -Me | -H | -tBu | -OH | -tBu | -Cl |
| 67 | -CN | 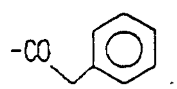 | -H | -H | -OH | -OH | -Br | -H |
| 68 | -CN | 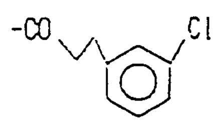 | -H | -H | -OH | -OH | -Cl | -H |
| 69 | -CN | 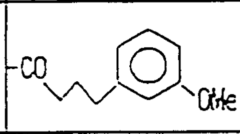 | -H | -H | -OH | -OH | -Cl | -H |
| 70 | -CN | 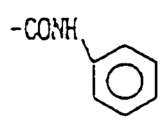 | -OH | -H | -OH | -OH | -Cl | -H |

Table 1 (continued)

| Compd. No. | $R^1$ | $R^2$ | $R^3$ | $R^4$ | $R^5$ | $R^6$ | $R^7$ | $R^8$ |
|---|---|---|---|---|---|---|---|---|
| 71 | -CN | 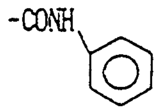 | -OH | -H | -OH | -OH | -OH | -Cl |
| 72 | -CN | 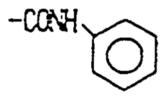 | -OH | -H | -OH | -OH | -CN | -H |
| 73 | -CN | 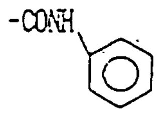 | -OH | -H | -Me | -OH | -Me | -Cl |
| 74 | -CN | 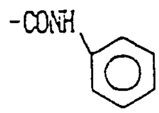 | -OH | -H | -OH | -OH | -H | $-NO_2$ |
| 75 | -CN | 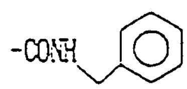 | -OH | -H | -OH | -OH | -Cl | -H |
| 76 | -CN | 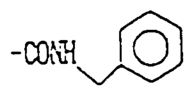 | -OH | -Cl | -OH | -OH | -Cl | -H |
| 77 | -CN | 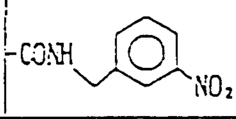 | -OH | -Cl | -OH | -OH | -Cl | -H |
| 78 | -CN | 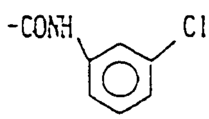 | -OH | -H | -OH | -OH | -Cl | -H |
| 79 | -CN | 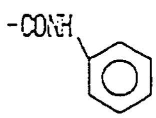 | -Me | -H | -OH | -OH | -Cl | -H |
| 80 | -CN | 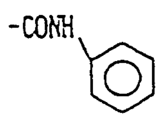 | -Me | -F | -OH | -OH | -F | -H |

Table 1 (continued)

| Compd. No. | $R^1$ | $R^2$ | $R^3$ | $R^4$ | $R^5$ | $R^6$ | $R^7$ | $R^8$ |
|---|---|---|---|---|---|---|---|---|
| 81 | -CN | 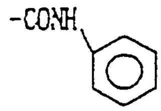 | -Me | -H | -OH | -OH | -F | $-NO_2$ |
| 82 | -CN | 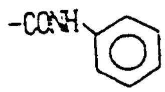 | -Me | -H | -OH | -OH | -CN | -H |
| 83 | -CN | 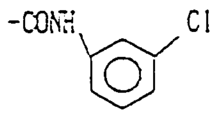 | -Me | -H | -OMe | -OH | $-SO_2Me$ | -H |
| 84 | -CN | 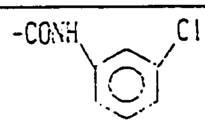 | -Me | -H | -OH | -OH | -Br | -Br |
| 85 | 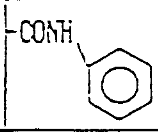 | -CN | -Me | -H | -OH | -OH | -Cl | -H |
| 86 | -CN | 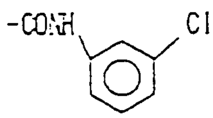 | -Bu | -H | -OH | -OH | -Cl | -H |
| 87 | -CN | 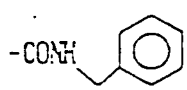 | -Bu | -H | -OH | -OH | -Cl | -H |
| 88 | -CN | 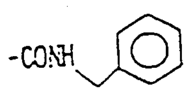 | -Bu | -H | -OH | -OH | -Cl | $-NO_2$ |
| 89 | -CN | 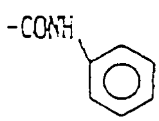 | -iPr | -H | -OH | -OH | -Cl | -H |
| 90 | 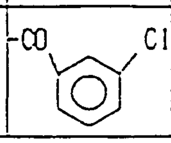 | 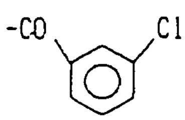 | -H | -H | -OH | -OH | -Cl | -H |

Table 1 (continued)

| Compound No. | $R^1$ | $R^2$ | $R^3$ | $R^4$ | $R^5$ | $R^6$ | $R^7$ | $R^8$ |
|---|---|---|---|---|---|---|---|---|
| 91 | 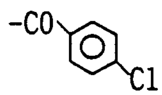 | 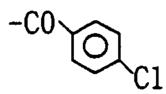 | -Me | -H | -OH | -OMe | -Cl | -H |
| 92 | 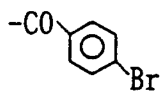 | 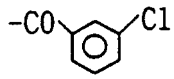 | -Me | -H | -OH | -OH | -F | -H |
| 93 | 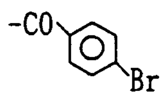 | 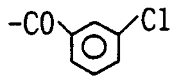 | -H | -H | -OH | -OH | -F | -H |
| 94 | -COPh | 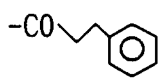 | -H | -H | -OH | -OH | -F | -H |
| 95 | -CN | 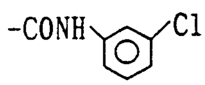 | -H | -H | -OMe | -OH | $-NO_2$ | -H |
| 96 | -CN | 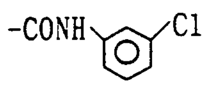 | -H | -H | -OH | -OH | $-NO_2$ | -H |
| 97 | -CN | 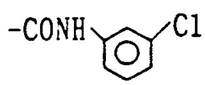 | -H | -H | -OMe | -OH | $-CF_3$ | -H |
| 98 | -CN | 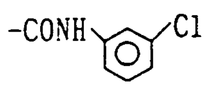 | -H | -H | -OH | -OH | $-CF_3$ | -H |
| 99 | -CN | -CONHPh | -H | -F | -OH | -OH | -F | -H |
| 100 | -CN | 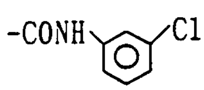 | -H | -H | -Br | -OH | -Br | -H |
| 101 | -CN | 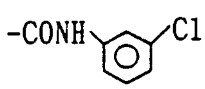 | -H | -H | -OMe | -OH | -Me | -H |

Table 1 (continued)

| Compound No. | $R^1$ | $R^2$ | $R^3$ | $R^4$ | $R^5$ | $R^6$ | $R^7$ | $R^8$ |
|---|---|---|---|---|---|---|---|---|
| 102 | -CN | 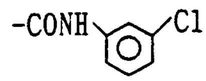 | -H | -H | $-CO_2H$ | -OH | -H | -H |
| 103 | -CN | 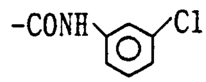 | -H | -H | -OMe | -OH | -H | -H |
| 104 | -CN | 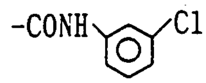 | -H | -H | -H | -OH | -Cl | -H |
| 105 | -CN | -CONHPh | -H | -H | -OMe | -OH | Br | -H |
| 106 | -CN | -CONHPh | -H | -H | -OMe | -OH | -F | -H |
| 107 | -CN | 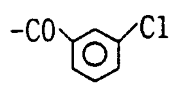 | -H | -H | -OH | -OH | -H | -H |
| 108 | -CN | 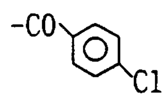 | -H | -H | -OH | -OH | -H | -H |
| 109 | -CN | 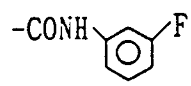 | -H | -H | -OH | -OH | -H | -H |
| 110 | -CN | 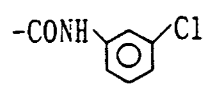 | -H | -H | -OH | -OH | -Me | -H |
| 111 | -CN | -CONHPh | -H | -H | -OH | -OH | -F | -H |
| 112 | -CN | -CONHPh | -H | -H | -OH | -OH | -Br | -H |

Table 1 (continued)

| Compound No. | $R^1$ | $R^2$ | $R^3$ | $R^4$ | $R^5$ | $R^6$ | $R^7$ | $R^8$ |
|---|---|---|---|---|---|---|---|---|
| 113 | -CN | 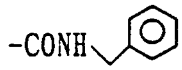 | -H | -H | -OH | -OH | -F | -H |
| 114 | -CN | 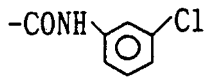 | -H | -H | -O$^n$Bu | -OH | -F | -H |
| 115 | -CN | -CONHPh | -H | -H | -O$^n$Bu | -OH | -F | -H |
| 116 | -CN | 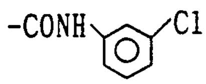 | -H | -H | -OiPr | -OH | -F | -H |
| 117 | -CN | -CONHPh | -H | -H | -OiPr | -OH | -F | -H |
| 118 | -CN | 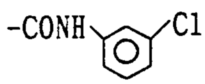 | -H | -H | -O$^n$Bu | -OH | -H | -H |
| 119 | -CN | 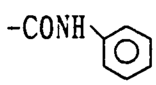 | -OH | -H | -OH | -OH | -H | -H |
| 120 | -CN | 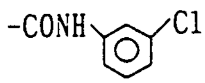 | -OH | -H | -OH | -OH | -H | -H |
| 121 | -CN | 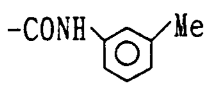 | -OH | -H | -OH | -OH | -H | -H |
| 122 | -CN | 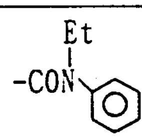 | -Me | -H | -OH | -OH | -H | -H |

In Table 1, Me means methyl group, Et means ethyl group, Pr means propyl group, Bu means buthyl group and Ph means phenyl group.

The compounds of the general formula (I) can be converted to their salts with a base. The bases may be those which can make a salt with the compound of the general formula (I) above. Illustrative examples of such salts are metallic salts such as sodium salt, magnesium salt, aluminum salt and the like, and amine salts such as ammonium salt, methylamine salt, ethylamine salt, diethylamine salt, triethylamine salt, pyrrolidine salt, piperidine salt, morpholine salt, pyridine salt, aniline salt, and the like.

The compounds of the general formula (I) above can be prepared, for example, through the following route:

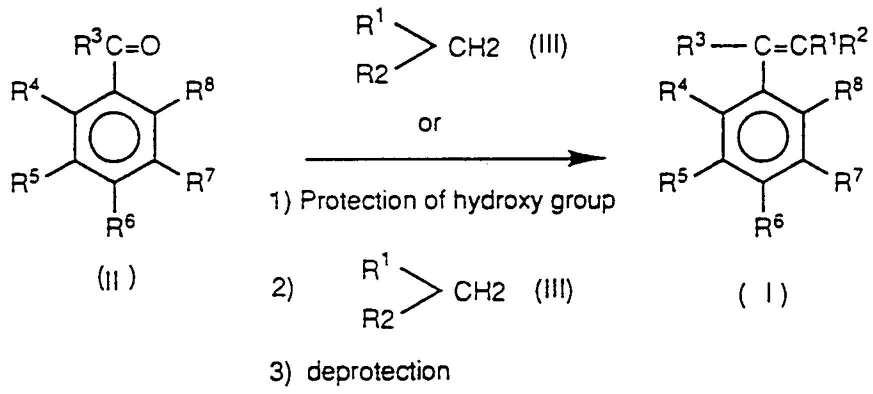


In the above formulae, $R^1$ to $R^8$ are as defined in the general formula (I) above, and $R^3$ represents hydrogen atom, chlorine atom, hydroxy group or $C_1$ - $C_5$ alkyl group.

(1) Production of the compounds wherein $R^3$ is hydrogen atom or $C_1$ - $C_5$ alkyl group:
The compounds can be prepared by condensing the compound of the general formula (II) above (in which $R^3$ represents hydrogen atom or $C_1$ - $C_5$ alkyl group) with the active methylene compound of the general formula (III) above with or without acid or base as a catalyst in appropriate solvent such as ethanol or benzene. Examples of the acids used as catalyst are protic acid such as sulfuric acid, and p-toluenesulfonic acid, and Lewis acid such as boron trifluoride, or the like. Examples of the bases usable as catalyst are ammonia or its salts; organic bases such as piperidine, pyridine, morpholine, 1, 8-diazabicyclo [5, 4, 0] undeca-7-ene or salts thereof; alkyl metal hydroxides such as sodium hydroxide, potassium hydroxide or the like; alkali metal amide such as lithium diisopropylamide or the like; metal alkoxide such as sodium methoxide or the like; alkali metal hydride such as sodium hydride, potassium hydride or the like.

The compounds of the general formula (I) wherein $R^1$ is cyano group and $R^2$ is $-CONR^9R^{10}$
(in which $R^9$ and $R^{10}$ are as defined above)
can be also prepared, for example, by condensing the compound of the general formula (II) above with cyanoacetic acid to give the compound of the following general formula (IV):

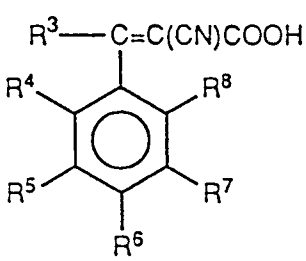

(IV)

(in which $R^3$ to $R^8$ are as defined in the general formula (I) above) and then reacting said compound (IV) with the amine of the following general formula (V):

$$R^9R^{10}NH \qquad (V)$$

(in which $R^9$ and $R^{10}$ are as defined in the general formula (I) above) in an appropriate solvent such as tetrahydrofuran, benzene, dimethylformamide or the like or without solvent in the presence or absence of a condensing agent. Said acids and bases are usable as a condensing agent, and moreover inorganic condensing agents such as phosphoryl oxychloride, thionyl chloride or the like, and organic condensing agents such as dicyclohexylcarbodiimide, carbonyl

diimidazole or the like can be used.

(2) Production of the compounds of wherein $R^3$ is hydroxy group:

Of the compounds of the general formula (II) above, for example, the compound in which $R^3$ is hydroxy group is subjected to reaction with trialkylsilane chloride such as trimethylsilane chloride, dimethyl-t-butylsilane chloride or the like or organic acid anhydride such as acetic anhydride, propionic anhydride or the like in the presence of nitrogen-containing compound such as triethylamine, pyridine, imidazole or the like in a hydrocarbon solvent such as benzene, toluene or the like or in a halogen type solvent (e.g. methylene chloride) or in an aprotic solvent (e.g. dimethylformamide) or to reaction with an organic acid anhydride such as acetic anhydride, propionic anhydride or the like also for a sole of solvent in the presence of a catalytic amount of mineral acid such as sulfuric acid, hydrochloric acid or the like, protonic acid such as p-toluenesulfonic acid or the like or Lewis acid such as boron trifluoride or the like, whereby said compound (II) in which at least one of $R^4$ to $R^8$ is acyloxy group or trialkylsiloxy group can be obtained. This product is allowed to react with thionyl chloride or oxalyl chloride in hydrocarbon solvent such as benzene, toluene or the like or halogen type solvent such as methylene chloride or the like to give the compound of the general formula (II) above in which $R^3$ is chlorine atom and at least one of $R^4$ to $R^8$ is acyloxy group or trialkylsiloxy group. This compound is allowed to react with the compound of the general formula (III) above or its alkali metal salt in hydrocarbon solvent such as benzene, toluene or the like or halogen type solvent such as methylene chloride or the like in the presence or absence of a base such as triethylamine, pyridine or the like to give the compound of the general formula (I) above. When the acyloxy group or trialkylsiloxy group remains in the product, these groups can be hydrolyzed if necessary. For example, acyloxy group may be hydrolyzed with alkali metal hydroxide such as sodium hydroxide or the like in a solvent such as water, methanol, tetrahydrofuran or their mixture, and trialkylsiloxy group may be hydrolyzed with an acid, fluorine ion or the like in methanol, tetrahydrofuran or their mixture.

In particular, the compound of the general formula (I) in which $R^3$ is OH and either of $R^1$ and $R^2$ is $-CONR^9R^{10}$ (wherein $R^9$ and $R^{10}$ are as defined in the general formula (I) above) can be prepared through the following route.

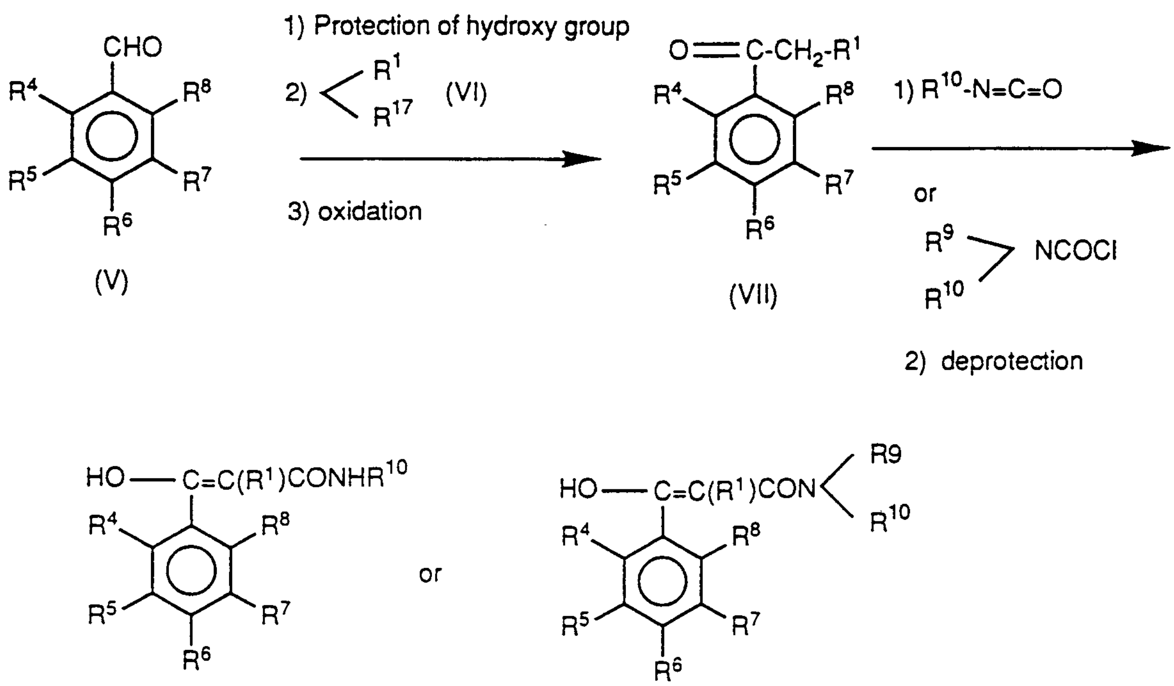


In the above formulae, $R^1$ and $R^4$ to $R^8$ are as defined in the general formula (I) above, and $R^{17}$ represents hydrogen atom or carboxy group. $R^{10}$ represents

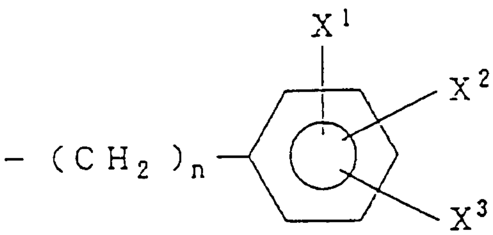

(in which $x^1$ to $x^3$ are as defined in the above general formula) and $R^9$ represents $C_1$ - $C_5$ alkyl group.

The compound of the general formula (V) is properly treated for protecting hydroxy group if necessary, allowed to react with the compound of the general formula (VI) in the presence of a base in a solvent such as diethyl ether, tetrahydrofuran or the like to give its adduct, and the latter is subjected to oxidation to give the compound of the general formula (VII). As bases, there are exemplified alkali metal amide such as lithium diisopropylamide or the like, organic metal compound such as butyllithium or the like, metal hydride such as sodium hydride or the like, and metal alcoholate such as sodium methylate or the like. Examples of the oxidizing agent are metallic oxidizing agent such as chromic acid, permanganic acid or the like, and organic oxidizing agent such as dimethyl sulfoxideoxalyl chloride or the like. Preferable protecting groups are those which can endure the reaction conditions, and include silyl group such as t-butyldimethylsilyl group or the like, substituted methyl group such as methoxymethyl group or the like, and benzyl group or the like.

The compounds of the general formula (I) or the salts thereof are useful as tyrosine kinase inhibitors as described below, and on the basis of such an activity, utility such as anticancer agents, immunosuppressive agents, platelet aggregation inhibitors, antiarteriosclerosis agents, antiinflammatory agents or the like can be expected therefrom.

Pharmaceutical formulation for the tyrosine kinase inhibitors and anticancer agents may be made in the manner suited for oral, enteral or parenteral administration. Examples of the formulation are tablets, capsules, granules, syrups, suppositories, ointments, injections and the like.

Carriers for the pharmaceutical formulation as tryrosine kinase inhibitors and anticancer agents may be organic or inorganic, solid or liquid, ordinarily inert pharmaceutical carrier suited for oral, enteral or parenteral administration. Examples of such carriers are crystalline cellulose, gelatin, lactose, starch, magnesium stearate, talc, vegetable or animal type fats and oils, gum, polyalkylene glycol, etc. Ratio of the tyrosine kinase inhibitor, or anticancer agent of the present invention to the carrier in the formulation may be changed from 0.2% to 100%.

Formulations of the tyrosine kinase inhibitor or anticancer agent of the present invention may include other tyrosine kinase inhibitor, anticancer agent and other medicines miscible therewith. The tyrosine kinase inhibitor or anticancer agent of the present invention is not necessarily the main ingredient in such formulation.

The tyrosine kinase inhibitor or anticancer agent of the present invention may be administered in a dosage which can attain generally the desired effect without side effect. Such a dosage should be decided by practioner's judgement, but in general, daily dosage for adult is 10 mg to 10 g, preferably 20 mg to 5 g. Daily dosage of the compound of the present invention for adult may be 1 mg to 5 g, preferably 3 mg to 1 g.

In order to evaluate tyrosine kinase inhibition and cancer cell proliferation inhibition of the compounds of the present invention, assay was effected on the partially purified human EGF (epidermal cell growth factor) receptor tyrosine kinase activity assaying system and cell culture system using human cancer cells. Further, of known inhibitors disclosed in patents and literatures, comparatively potent compounds were assayed simultaneously for comparing the inhibitory activity.

Test on Tyrosine Kinase Activity

Tyrosine kinase activity inhibition was assayed by improving the tyrosine kinase activity assaying method as described in Linda J. Pike at al., Proceedings of the National Academy of Sciences of the U.S.A. 79, 1443 (1982), using EGF receptor partially purified from A431 cell line derived from human squamous cell carcinoma.

Detailed explanation of the assaying method will be given below.

A431 cells were cultivated in Dulbecco's modified Eagle's medium (DMEM) containing 10% fetal calf serum (FCS) at 37°C in a stream of 5% carbon dioxide, and the resultant cells were homogenized in a solution containing 10 mM N-2-hydroxyethylpiperazino-N'-2-ethanesulfonic acid (Hepes) buffer (pH 7.4), 0.25M sucrose and 0.1 mM EDTA and centrifuged at 1000 g for 5 minutes. The supernatant was centrifuged at 27500 x g for 30 minutes to give A431 cell membrane fraction, which was provided for the assay as the partially purified EGF receptor, enzyme source.

To a mixture of said A431 cell membrane fraction (10 to 15 μg), 15 mM Hepes buffer (pH 7.7), 2 mM $MnCl_2$, 10 μM $ZnSO_4$ and a test material (1% final concentration, DMSO) dissolved in dimethyl sulfoxide (DMSO) was added 100 ng EGF, and the resultant mixture was subjected to preincubation in ice for 10 minutes, and the reaction was initiated by

adding 75 µg synthetic substrate RR-SRC peptide (Arg-Arg-Leu-Ile-Glu-Asp-Ala-Glu-Tyr-Ala-Ala-Arg-Gly) and 10 µMγ-$^{32}$P-adenosine triphosphate (55.5 kBq/assay). At that time the volume was 60 µl. The reaction was allowed to perform in ice for 30 minutes and stopped by adding 10 mg/ml bovine serum albumin (6 µl) and 20% trichloroacetic acid (25 µl). The mixture was allowed to stand in ice for 30 minutes.

Then the mixture was centrifuged at 5000 x g for 2 minutes, and the supernatant (40 µl) was sampled and adsorbed on P81 phosphocellulose paper. The paper was fixed by dipping in 30% aqueous acetic acid for 15 minutes, washed by dipping in 15% aqueous acetic acid for 15 minutes (the washing was repeated 4 times), dipped in acetone for 5 minutes, dried, and the count of $^{32}$P attached on P81 phosphocellulose paper was assayed with a liquid scintillation counter. The count obtained was defined as "A". At the same time, counts of the reaction not containing the test material and the reaction not containing both the test material and EGF were assayed, and the resulted counts were defined as "B" and "C" respectively.

Tyrosine kinase inhibitory rate was obtained according to the following equation:

$$\text{Inhibitory Rate (\%)} = (B\text{-}A/B\text{-}C) \times 100$$

Further, $IC_{50}$ value (50% Inhibitory Concentration) was calculated from the inhibitory rate obtained by changing the concentration of the test material.

Test on Cancer Cells in Tissue Culture

SKOV3 cell line, which is from human ovary cancer, possesses excessive c-erbB2 onco-genes on the surface of the cell.

c-erbB2 gene product is a proliferation factor receptor type membrane protein having homology with EGF receptor and it shows tyrosine kinase activity as EGF receptor. Effect of the test material against the proliferation of cultivated cancer cells was examined with this SKOV3 cell line as follows:

SKOV3 cell was inoculated at a rate of 1 x $10^3$ cell/well on a 96 well dish, and cultivated on a DMEM: F12 (1 : 1) medium containing 10% FCS and 0.1 mg/ml kanamycin at 37°C in a stream of 5% carbon dioxide for one day. Test material dissolved in DMSO was added to the medium (final concentration of DMSO was below 0.1%), which was cultivated for 3 days under the conditions mentioned above. Test material was replaced every 24 hours together with the medium.

Counting the number of living cells was effected by making colorimetric analysis at two wave length, 550 nm and 610 nm, in reference to the assaying method as described in Michael C. Alley et al., Cancer Research, 48, 589 (1988) using MTT reagent, and the number obtained was defined as "a".

At the same time, the count of the number of living cells obtained without adding the test material was assayed, and the number obtained was defined as "b".

Cell proliferation inhibitory rate was determined according to the following equation:

$$\text{Inhibitory Rate (\%)} = (b\text{-}a)/b \times 100$$

The results are shown in Tables 2 - 5. Compound No. in the table corresponds to Compound No. in Table 1.

Table 2: Tyrosine Kinase Activity Inhibitory Effect

| Compound No. | Inhibitory Rate (%) in the presence of 10 μM | Reference* |
|---|---|---|
| 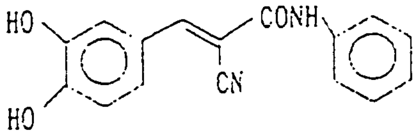 | 41 | 1) |
| 10 | 47 | - |
| 11 | 77 | - |
| 12 | 52 | - |
| 13 | 100 | - |
| 14 | 77 | - |
| 1 | 63 | - |
| 2 | 74 | - |

Table 3

| Compound No. | $IC_{50}$ (μM) | Reference* |
|---|---|---|
| 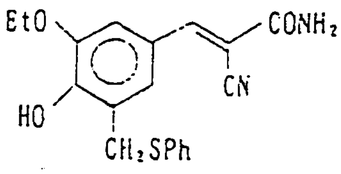 | 2.6 | 2) |
| 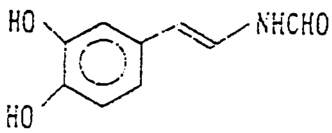 | 12 | 3) |
| 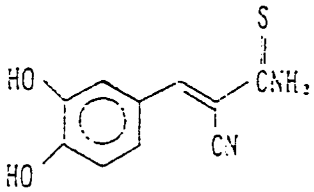 | 11 | 4) |
| 1 | 1.6 | - |
| 2 | 1.2 | - |
| 3 | 0.68 | - |
| 4 | 0.68 | - |
| 5 | 1.4 | - |
| 6 | 0.49 | - |
| 7 | 1.6 | - |
| 8 | 0.60 | - |
| 9 | 0.28 | - |
| 13 | 1.4 | - |
| 14 | 2.1 | - |
| 15 | 1.0 | - |
| 16 | 0.28 | - |
| 17 | 1.4 | - |

Table 3 (Continued)

| Compound No. | $IC_{50}$ (μM) | Reference* |
|---|---|---|
| 23 | 0.9 | - |
| 95 | 1.8 | - |
| 98 | 2.3 | - |
| 100 | 1.1 | - |
| 101 | 0.42 | - |
| 103 | 2.1 | - |
| 105 | 1.8 | - |
| 110 | 1.3 | - |
| 119 | 0.96 | - |

Table 4

| Compound No. | Inhibitory Rate (%) in the presence of 1 μM |
|---|---|
| 13 | 46 |
| 7 | 41 |
| 8 | 78 |

Table 5: Proliferation Inhibitory Effect Against Cultivated Cells

| Compound No. | $IC_{50}$ (μM) | Reference* |
|---|---|---|
| 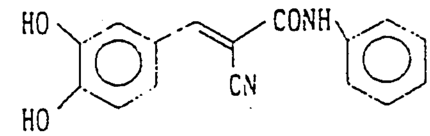 | 35 | 1) |
| 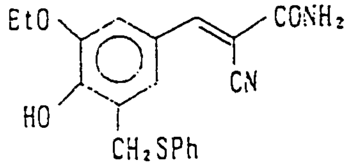 | 78 | 2) |
| 1 | 17.3 | - |
| 2 | 20.4 | - |
| 4 | 21.6 | - |
| 6 | 21 | - |
| 8 | 23.4 | - |
| 9 | 17 | - |
| 13 | 30 | - |
| 15 | 34 | - |
| 16 | 24 | - |
| 98 | 16 | - |
| 100 | 19 | - |
| 105 | 13 | - |
| 110 | 14 | - |
| 119 | 24 | - |

Reference

1) A. Gazit, N. Osheroy, I. Posner, P. Yaish, E. Pradosu, C. Gilon, and A. Levit zki; J. Med. Chem., 34, 1896 (1991).

2) T. Shiraishi, K. Kameyama, N. Imai, T. Domoto, I. Kasumi, and K. Watanabe; Chem. P harm. Bull., 36, (1988).

3) H. Umezawa, M. Imoto, T. Sawa, K. Isshiki, N. Matsuda, T. Uchida, H. Iinuma, M. Hamada, T. Takeuchi, J. Antibiot. 39, 170 (1986).

4) A. Gozit, P. Yaish, C. Gilon, A. Levitzki, J. Med. Chem. 32, 2344 (1989).

It was evident from the results of Tables 2 - 5 that the compounds of the present invention show particularly excellent enzyme inhibitory activity and cell proliferation inhibitory activity in comparison with the tyrosine kinase inhibitors already known.

Toxicity Test

Toxicity test for the compounds of the present invention was examined in the manner as shown below.

Test compound Compound No. 4
Test animal Mouse ddy (male) 16 - 20 g

Dosage and Method of Measurement:

Test compound was suspended in a mixture of 0.25% carboxymethyl cellulose, physiological saline, and a small amount of Tween 80 to give a test solution. This solution was administered intraperitoneally at dose of 0 mg/kg (control), 500 mg/kg and 1000 mg/kg to the test animal. Change of the body weight was measured 3 days later.

Test results:

No dead case was observed in both dosages of 500 mg/kg and 1000 mg/kg. Change of the body weight was as follows:

| Dosage | Change of the body weight |
|---|---|
| 500 mg/kg | +5.6 g |
| 1000 mg/kg | +6.1 g |
| Control (0 mg/kg) | +6.2 g |

Any substantial difference on the change of the body weight was not observed among the groups, and it was concluded that the compound of the present invention would show very low toxicity.

Presently preferred and practical embodiments of the present invention will be illustratively shown in the following examples.

Example 1

Production of 2-cyano-3-(3-chloro-4-hydroxy-5-methoxyphenyl) propeneanilide (Compound No. 1 in Table 1):

To a solution of 5-chlorovanillin (728 mg, 3.9 mmol) and cyanoacetanilide (625 mg, 3.9 mmol) in ethanol (20 ml) were added 3 drops of piperidine, and the resulting mixture was refluxed for 2 hours. After cooling to room temperature, the resulting solid was filtered and washed with ethanol: water (1 : 1) to give the objective 2-cyano-3-(3-chloro-4-hydroxy-5-methoxyphenyl) propenanilide (950 mg, 74% in yield).
mp. 273°C
$^1$HNMR (DMSO-$d_6$, 250 MHz) $\delta$ ppm: 3.89 (s, 3H), 7.12 (t, J = 7.5 Hz, 1H), 7.36 (t, J = 7.5 Hz, 2H), 7.60 - 7.75 (m, 4H) 8.14 (s, 1H), 10.28 (s, 1H), 10.74 (brs, 1H)

Example 2

Production of 2-cyano-3-(3-chloro-4, 5-dihydroxyphenyl)propenanilide (Compound No. 2 in Table 1):

To a solution of 2-cyano-3-(3-chloro-4-hydroxy-5-methoxyphenyl) propenanilide (340 mg, 1.03 mmol) in methylene chloride (20 ml) chilled at -70°C was dropwise added 1 M solution of boron tribromide in methylene chloride (2.50 ml, 2.5 mmol). The reaction mixture was gradually allowed to warm to room temperature and poured into water. The product was extracted with ethyl acetate, dried over $MgSO_4$ and concentrated. The resulting solid was suspended in diethyl ether, refluxed for 10 minutes and the resultant solid was filtered to give the objective 2-cyano-3-(3-chloro-4, 5-dihydroxyphenyl) propenanilide (260 mg, 80% in yield).
mp, 227 - 234°C
$^1$HNMR (DMSO-$d_6$, 250 MHz) $\delta$ ppm: 7.12 (t, J = 7.8 Hz, 1H), 7.35 (t, J = 7.8 Hz, 2H), 7.50 (d, J = 1.9 Hz, 1H), 7.57 (t, J = 1.9 Hz, 1H), 7.65 (d, J = 7.8 Hz, 2H), 8.04 (s, 1H), 10.25 (s, 1H), 10.43 (brs, 1H), 10.54 (brs, 1H).

Example 3

Production of N-(3-chlorophenyl)-2-cyano-3-(3-chloro-4-hydroxy-5-methoxyphenyl) propenamide (Compound No. 3 in Table 1):

In the same manner as in Example 1 were condensed 5-chlorovanillin (1.23 g, 6.59 mmol) and N-(3-chlorophenyl) cyanoacetamine (1.28 g, 6.59 mmol) suspended in diethyl ether (40 ml), refluxed for 10 minutes, allowed to cool to room temperature and filtered to give N-(3-chlorophenyl)-2-cyano-3-(3-chloro-4-hydroxy-5-methoxyphenyl) propenamide (1.30 g, 54% in yield).
mp. 225 - 226°C
$^1$HNMR (DMSO-$d_6$, 250 MHz) $\delta$ ppm: 4.77 (s, 3H), 8.07 (d, J = 8.1 Hz, 1H), 8.28 (t, J = 8.1 Hz, 1H), 8.48 (d, J = 8.1 Hz, 1H), 8.56 (d, J = 2.0 Hz, 2H), 8.60 (d, J = 1.8 Hz, 1H), 8.69 (d, J = 2.0 Hz, 1H), 9.04 (s, 1H), 11.33 (brs, 1H), 11.67 (brs, 1H).

Example 4

Production of N-(3-chlorophenyl)-2-cyano-3-(3-chloro-4, 5-dihydroxyphenyl) propenamide (Compound No. 4 in Table 1):

Demethylation was effected in the same manner as in Example 2 by using N-(3-chlorophenyl)-2-cyano-3-(3-chloro-4-hydroxy-5-methoxyphenyl) propenamide (587 mg, 1.62 mmol) and 1 M solution of boron tribromide in methylene chloride. The resulting solid was suspended in ethanol, refluxed for 10 minutes, allowed to cool to room temperature and filtered to give the objective N-(3-chlorophenyl)-2-cyano-3-(3-chloro-4, 5-dihydroxyphenyl) propenamide (250 mg, 44% in yield).
mp. > 300°C
$^1$HNMR (DMSO-$d_6$, 250 MHz) $\delta$ ppm: 7.18 (m, 1H), 7.39 (t, J = 8.0 Hz, 1H), 7.45 - 7.65 (m, 3H), 7.81 (t, J = 1.8 Hz, 1H), 8.05 (s, 1H), 10.39 (brs, 1H), 10.49 (brs, 2H).

Example 5

Production of N-(3-chlorophenyl)-2-cyano-3-(3-bromo-4-hydroxy-5-methoxyphenyl) propenamide (Compound No. 5 in Table 1):

The reaction was performed in the same manner as in Example 1 with 5-bromovanillin (1.38 g, 5.97 mmol) and N-(3-chlorophenyl) cyanoacetamide (1.16 g, 5.97 mmol) to give the objective N-(3-chlorophenyl)-2-cyano-3-(3-bromo-4-hydroxy-5-methoxyphenyl) propenamide (1.58 g, 65% in yield).
mp. 217 - 219°C

$^1$HNMR (DMSO-$d_6$, 250 MHz) δ ppm: 3.89 (s, 3H), 7.19 (d, J = 8.0 Hz, 1H), 7.39 (t, J = 8.0 Hz, 1H), 7.60 (d, J = 8.0 Hz, 1H), 7.71 (s, 1H), 7.81 (s, 1H), 7.84 (s, 1H), 8.15 (s, 1H), 10.43 (s, 1H), 10.85 (brs, 1H).

Example 6

Production of N-(3-chlorophenyl)-2-cyano-3-(3-bromo-4, 5-dihydroxyphenyl) propenamide (Compound No. 6 in Table 1):

Demethylation was effected in the same manner as in Example 2 with N-(3-chlorophenyl)-2-cyano-3-(3-bromo-4-hydroxy-5-methoxyphenyl) propenamide (1.38 g, 3.39 mmol) and 1 M solution of boron tribromide in methylene chloride (8.5 ml, 8.5 mmol). The resultant solid was mixed with ethanol, refluxed for 10 minutes, allowed to cool to room temperature and filtered to give the objective N-(3-chlorophenyl)-2-cyano-3-(3-bromo-4, 5-dihydroxyphenyl) propenamide (360 mg, 27% in yield).
mp. 266 - 270°C

$^1$HNMR (DMSO-$d_6$, 250 MHz) δ ppm; 7.18 (m, 1H), 7.39 (t, J = 8.1 Hz, 1H), 7.55 - 7.68 (m, 3H), 7.81 (t, J = 1.9 Hz, 1H), 8.05 (s, 1H), 10.38 (s, 1H), 10.47 (brs, 1H), 10.59 (brs, 1H).

Example 7

Production of N-(3-chlorophenyl)-2-cyano-3-(3-fluoro-4-hydroxy-5-methoxyphenyl) propenamide (Compound No. 7 in Table 1):

The reaction was effected in the same manner as in Example 1 with 5-fluorovanillin (410 mg, 2.41 mmol) and N-(3-chlorophenyl) cyanoacetamide (469 mg, 2.41 mmol) to give the objective N-(3-chlorophenyl)-2-cyano-3-(3-fluoro-4-hydroxy-5-methoxyphenyl) propenamide (476 mg, 57% in yield) as yellow powders.
mp. 235°C

$^1$HNMR (DMSO-$d_6$, 250 MHz) δ ppm: 3.88 (s, 3H), 7.20 (m, 1H), 7.40 (t, J = 8.1 Hz, 2H), 7.52 - 7.63 (m, 3H), 7.83 (t, J = 1.8 Hz, 1H), 8.18 (s, 1H), 10.48 (s, 1H).

Example 8

Production of N-(3-chlorophenyl)-2-cyano-3-(3-fluoro-4, 5-dihydroxyphenyl) propenamide (Compound No. 8 in Table 1):

Demethylation was effected in the same as in Example 2 with N-(3-chlorophenyl)-2-cyano-3-(3-fluoro-4-hydroxy-5-methoxyphenyl) propenamide (470 mg, 1.36 mmol) and 1M solution of boron tribromide in methylene chloride (3.50 ml, 34.50 mmol). The resulting solid was recrystallized from ethanol-water to give the objective N-(3-chlorophenyl)-2-cyano-3-(3-fluoro-4, 5-dihydroxyphenyl) propenamide (160 mg, 35% in yield) as yellow powder.
mp. 269 - 270°C

$^1$HNMR (DMSO-$d_6$, 250 MHz) δ ppm: 7.20 (m, 1H), 7.34 - 7.46 (m, 3H), 7.60 (m, 1H), 7.82 (t, J = 1.8 Hz, 1H), 8.06 (s, 1H), 10.42 (s, 1H).

Example 9

Production of N-(3-chlorophenyl)-2-cyano-3-(2, 3-dibromo-4, 5-dihydroxyphenyl) propenamide (Compound No. 9 in Table 1):

The reaction was effected in the same manner as in Example 1 with 3, 4-dihydroxy-5, 6-dibromobenzaldehyde (1.01 g, 3.41 mmol) and N-(3-chlorophenyl) cyanoacetalmide (644 mg, 3.41 mmol) to give the objective N-(3-chlorophenyl)-2-cyano-3-(2, 3-dibromo-4, 5-dihydroxyphenyl) propenamide (1.26 g, 78% in yield) as yellow needles.
mp. 260 - 265°C (dec.)

$^1$HNMR (DMSO-$d_6$, 250 MHz) δ ppm: 7.22 (m, 1H), 7.41 (t, J = 8.1 Hz, 1H), 7.62 (m, 1H), 7.66 (s, 1H), 7.84 (t, J = 1.9 Hz, 1H), 8.35 (s, 1H), 10.54 (s, 1H), 10.81 (brs, 2H).

IR (KBr) $cm^{-1}$: 3420, 3300, 3220, 2220, 1660, 1580, 1515, 1455, 1262, 1195, 774.

Example 10

Production of N-(4-bromophenyl)-2-cyano-3-(3, 4-dihydroxyphenyl) propenamide (Compound No. 10 in Table 1):

To 2N aqueous sodium hydroxide solution (175 ml, 350 mmol) heated at 60°C were added cyanoacetic acid (7.2 g, 86.7 mmol) and 3, 4-dihydroxybenzaldehyde (12.0 g, 86.9 mmol), and the resulting mixture was stirred for 30 minutes. The reaction mixture was allowed to cool at room temperature and gradually mixed with dilute hydrochloric acid until the reaction mixture became acidic. The product was filtered and dried to give α-cyano-3, 4-dihydroxycinnamic acid (hereinafter referred to as "Compound A" (10.0 g, 56% in yield).

A mixture of the resulting compound A (400 mg, 1.9 mmol) and a solution of p-bromoaniline (654 mg, 3.8 mmol) in tetrahydrofuran (20 ml) was stirred in ice bath. Triethylamine (0.3 ml, 2.2 mmol) and phosphoryl oxychloride (0.8 ml, 8.6 mmol) were added to the mixture, which was stirred at room temperature for 4 hours. The reaction mixture was concentrated in vacuo to remove the solvent, and the residue was partitioned between water and ethylacetate. The organic layer was separated, dried and concentrated. The residue was recrystallized from ethanol to give the objective N-(4-bromophenyl)-2-cyano-3-(3, 4-dihydroxyphenyl) propenamide (250 mg, 36% in yield).

mp. 277 - 278°C

$^1$HNMR (DMSO-$d_6$, 250 MHz) δ ppm: 6.90 (d, J = 8.3 Hz, 1H), 7.33 (dd, J = 2.0, 8.3 Hz, 1H), 7.53 (d, J = 8.9 Hz, 2H), 7.58 (d, J = 2.0 Hz, 1H), 7.63 (d, J = 8.9 Hz, 2H), 8.03 (s, 1H), 9.50 (brs, 2H), 10.29 (s, 1H).

Example 11

Production of N-(3-bromophenyl)-2-cyano-3-(3, 4-dihydroxyphenyl) propenamide (Compound No. 11 in Table 1):

Condensation of Compound A (996 mg, 4,85 mmol) and m-bromoaniline (0.63 ml, 5.82 mmol) was effected in the same manner as in Example 10. The resulting solid was recrystallized from ethanol-water to give the objective N-(3-bromophenyl)-2-cyano-3-(3, 4-dihydroxyphenyl) propenamide (300 mg, 17% in yield).

$^1$HNMR (DMSO-$d_6$, 250 MHz) δ ppm: 6.91 (d, J = 8.3 Hz, 1H), 7.20 - 7.40 (m, 3H), 7.58 (d, J = 2.3 Hz, 1H), 7.60 - 7.70 (m, 1H), 7.95 (s, 1H), 8.05 (s, 1H), 9.63 (brs, 1H), 10.22 (brs, 1H), 10.32 (brs, 1H).

Example 12

Production of N-(4-chlorophenyl)-2-cyano-3-(3, 4-dihydroxyphenyl) propenamide (Compound No. 12 in Table 1):

Condensation of Compound A (400 mg, 1.9 mmol) and p-chloroaniline (482 mg, 3.8 mmol) was effected in the same manner as in Example 10. The resulting solid was recrystallized from ethanol to give the objective N-(4-chlorophenyl)-2-cyano-3-(3, 4-dihydroxyphenyl) propenamide (110 mg, 18% in yield).

mp. 290°C

$^1$HNMR (DMSO-$d_6$, 250 MHz) δ ppm: 6.89 (d, J = 8.3 Hz, 1H), 7.33 (dd, J = 1.8, 8.3 Hz, 1H), 7.40 (d, J = 9.0 Hz, 2H), 7.58 (d, J = 1.8 Hz, 1H), 7.68 (d, J = 9.0 Hz, 2H), 8.03 (s, 1H), 9.85 (brs, 3H), 10.29 (brs. 3H).

Example 13

Production of N-(3-chlorophenyl)-2-cyano-3-(3, 4-dihydroxyphenyl) propenamide (Compound No. 13 in Table 1):

Condensation of Compound A (400 mg, 1.9 mmol) and m-chloroaniline (482 mg, 3.8 mmol) was effected in the same manner as in Example 10. The resulting solid was suspended in diethyl ether and refluxed for 10 minutes. The precipitate obtained was filtered to give the objective N-(3-chlorophenyl)-2-cyano-3-(3, 4-dihydroxyphenyl) propenamide (280 mg, 46% in yield).

$^1$HNMR (DMSO-$d_6$, 250 MHz) δ ppm: 6.90 (d, J = 8.3 Hz, 1H), 7.17 (dd, J = 8.0, 1.9 Hz, 1H), 7.34 (dd, J = 2.0, 8.3 Hz, 1H), 7.37 (t, J = 8.0 Hz, 1H), 7.53 - 7.65 (m, 2H), 7.81 (t, J = 1.9 Hz, 1H), 8.04 (s, 1H), 9.92 (brs, 3H), 10.33 (brs, 3H).

Example 14

Production of N-(3-methylphenyl)-2-cyano-3-(3, 4-dihydroxyphenyl) propenamide (Compound No. 14 in Table 1):

condensation of Compound A (400 mg, 1.9 mmol) and m-toluidine (418 mg, 3.8 mmol) was effected in the same manner as in Example 10. The resulting solid was suspended in hexane-ether (5 : 1) and refluxed for 10 minutes. The resulting solid was filtered to give the objective N-(3-methylphenyl)-2-cyano-3-(3, 4-dihydroxyphenyl) propenamide (360 mg, 64% in yield).

$^1$HNMR (DMSO-$d_6$, 250 MHz) δ ppm: 2.29 (s, 3H), 6.86 - 6.95 (m, 2H), 7.22 (t, J = 7.6 Hz, 1H), 7.32 (dd, J = 2.0, 8.3 Hz, 1H), 7.40 - 7.50 (m, 2H), 7.57 (d, J = 2.0 Hz, 1H), 8.01 (s, 1H), 9.61 (s, 1H), 10.09 (s, 1H), 10.17 (s, 1H).

Example 15

Production of N-(3-chlorophenyl)-2-cyano-3-(3-cyano-4, 5-dihydroxyphenyl) propenamide (Compound No. 15 in Table 1):

Condensation of 3-cyano-4, 5-dimethoxybenzaldehyde (200 mg, 1.05 mmol) and N-(3-chlorophenyl) cyanoacetacide (204 mg, 1.05 mmol) was effected in the same manner as in Example 1 to give a condensate (140 mg). This was treated with 1 M solution of boron tribromide in methylene chloride in the same manner as in Example 2, and the resulting solid was recrystallized from ethanol-water to give the objective N-(3-chlorophenyl)-2-cyano-3-(3-cyano-4, 5-dihydroxyphenyl) propenamide (70 mg, 25% in yield) as brown powdery crystals.
mp. 236 - 240°C

$^1$HNMR (DMSO-$d_6$, 250 MHz) δ ppm: 7.19 (d, J = 8.0 Hz, 1H), 7.39 (t, J = 8.0 Hz, 1H), 7.59 (d, J = 8.0 Hz, 1H), 7.66 (s, 1H), 7.81 (s, 1H), 7.85 (s, 1H), 8.09 (s, 1H), 10.45 (s, 1H), 11.0 (brs, 2H).

Example 16

Production of N-(3-chlorophenyl)-2-cyano-3-(4, 5-dihydroxy-2-nitrophenyl) propenamide (Compound No. 16 in Table 1):

The reaction of 4, 5-dihydroxy-2-nitrobenzaldehyde (185 mg, 1.01 mmol) and N-(3-chlorophenyl) cyanoacetamide (197 mg, 1.01 mmol) was effected in the same manner as in Example 1 to give the objective N-(3-chlorophenyl)-2-cyano-3-(4, 5-dihydroxy-2-nitrophenyl) propenamide (100 mg, 28% in yield) as brown powdery crystals.
mp. 219 - 225°C (dec.)

$^1$HNMR (DMSO-$d_6$ , 500 MHz) δ ppm: 7.21 (s, 2H), 7.40 (t, J = 8.0 Hz, 1H), 7.61 (d, J = 8.0 Hz, 1H), 7.67 (s, 1H), 7.84 (s, 1H), 8.56 (s, 1H), 10.5 (brs, 1H).

IR (KBr) $cm^{-1}$: 3330, 3200, 2230, 1665, 1600, 1530, 1450, 1305.

Example 17

Production of N-(3-chlorophenyl)-2-cyano-3-[4-hydroxy-3-methoxy-5-(methylsulfonyl) phenyl] propenamide (Compound No. 17 in Table 1):

A solution of 4-hydroxy-3-methoxy-5-(methylsulfonyl)-benzaldehyde (345 mg, 1.50 mmol), N-(3-chlorophenyl)-cyanoacetamide (292 mg, 1.50 mmol), acetic acid (50 μl) and piperidine (50 μl) in benzene (10 ml) was refluxed for 4 hours under dehydrating with Dean Stark's dehydrating apparatus. The resulting solid was filtered and recrystallized from ethanol to give N-(3-chlorophenyl)-2-cyano-3-[4-hydroxy-3-methoxy-5-(methylsulfonyl) phenyl]-propenamide (410 mg, 67% in yield) as yellow powder.
mp. 232 - 234°C

$^1$HNMR (DMSO-$d_6$, 250 MHz) δ ppm: 3.29 (s, 3H), 3.93 (s, 3H), 7.20 (m, 1H), 7.41 (t, J = 8.1 Hz, 1H), 7.62 (m, 1H), 7.84 (t, J = 1.9 Hz, 1H), 7.96 (d, J = 2.0 Hz, 1H), 8.04 (d, J = 2.0 Hz, 1H), 8.29 (s, 1H), 10.43 (s, 1H).

Example 18

Production of 2-cyano-3-oxo-3-phenyl-l-(2, 3-dibromo-4, 5-dihydroxy) propene (Compound No. 18 in Table 1):

The reaction of 5, 6-dibromo-3, 4-dihydroxybenzaldehyde (480 mg, 1.62 mmol) and benzoylacetonitrile (235 mg, 1.62 mmol) was effected in the same manner as in Example 1, and the resultant solid was recrystallized from ethanol-water to give the objective 2-cyano-3-oxo-3-phenyl-l-(2, 3-dibromo-4, 5-dihydroxy) propene (365 mg, 53% in yield).
mp. 229 - 231°C (dec.)

$^1$HNMR (DMSO-$d_6$, 500 MHz) δppm: 7.58 (t, J = 7.6 Hz, 2H), 7.69 (t, J = 7.6 Hz, 1H), 7.83 (m, 3H), 8.21 (s, 1H), 10.86 (brs, 2H).

Example 19

Production of N-(3-chlorophenyl)-2-cyano-3-(3-methoxy-4-hydroxy-5-nitrophenyl) propenamide (Compound No. 95 in Table 1):

The reaction of 5-nitrovanillin (915 mg, 4.6 mmol) and N-(3-chlorophenyl) cyanoacetamide (903 mg, 4.6 mmol) was effected in the same manner as in Example 1 to give the objective titled compound (1.53 g, 88%).

$^1$HNMR (DMSO-$d_6$, 250 MHz) δ ppm: 7.21 (brd, J = 7.9 Hz, 1H), 7.41 (t, J = 8.2 Hz, 1H), 7.61 (brd, J = 8.2, 1H), 7.83 (t, J = 1.9Hz, 1H), 7.86 (d, J = 2.0 Hz, 1H), 8.01 (d, J = 2.0 Hz, 1H), 8.19 (s, 1H).
mp. 229 - 231°C

Example 20

Production of N-(3-chlorophenyl)-2-cyano-3-(3, 4-dihydroxy-5-nitrophenyl) propenamide (Compound No. 96 in Table 1):

The reaction of N-(3-chlorophenyl)-2-cyano-3-(3-methoxy-4-hydroxy-5-nitrophenyl) propenamide (865 mg, 2.3 mmol) and 1 M solution of boron tribromide in methylene chloride (8.0 ml, 8.0 mmol) was effected in the same manner as in Example 2. The resultant solid was suspended in ethanol-water to give the objective titled compound (240 mg, 29%) as coppery yellow powder.

$^1$HNMR (DMSO-$d_6$, 250 MHz) δ ppm: 3.95 (s, 3H), 7.21 (ddd, J = 0.8, 1.9, 8.1 Hz, 1H), 7.41 (t, J = 8.1 Hz, 1H), 7.62 (dt, J = 8.1, 0.9 7.83 (t, J = 1.9 Hz, 1H), 7.94 (d, J = 1.9 Hz, 1H), 8.19 (d, J = 1.9 Hz, 1H), 8.27 (s, 1H), 10.52 (s, 1H)
mp. 242 - 248°C (dec.)

Example 21

Production of N-(3-chlorophenyl)-2-cyano-3-[3-methoxy-4-hydroxy-5-(trifluoromethyl)]propenamide (Compound No. 97 in Table 1):

Condensation of 3-methoxy-4-hydroxy-5-(trifluoromethyl) benzaldehyde (770 mg, 3.5 mmol) and N-(3-chlorophenyl) cyanoacetamide (680 mg, 3.5 mmol) was effected in the same manner as in Example 1 to give the objective titled compound (956 mg, 76%).

$^1$HNMR (DMSO-$d_6$, 250 MHz) δ ppm: 3.93 (s, 3H), 7.19 (m, 1H), 7.39 (t, J = 8.1 Hz, 1H), 7.60 (m, 1H), 7.82 (t, J = 1.9 Hz, 1H), 7.87 (d, J = 1.7 Hz, 1H), 7.93 (1.7 Hz, 1H), 8.26 (s, 1H), 10.47 (s, 1H), 11.21 (brs, 1H).
mp. 200 - 205°C

Example 22

N-(3-chlorophenyl)-2-cyano-3-[3, 4-dihydroxy-5-(trifluoromethyl) phenyl] propenamide (Compound No. 98 in Table 1):

Reaction of N-(3-chlorophenyl)-2-cyano-[3-methoxy-4-hydroxy-5-(trifluoromethyl)]propenamide obtained in Example 21 and 1M solution of boron tribromide in methylene chloride (4.9 ml, 4.9 mmol) was effected in the same manner as in Example 2 to give the objective titled compound (576 mg, 52%).

$^1$HNMR (DMSO-$d_6$, 250 M Hz) δ ppm: 7.18 (m, 1H), 7.38 (t, J = 8.1 Hz, 1H), 7.60 (brd, J = 8.1 Hz, 1H), 7.65 (d, J = 1.8 Hz, 1H), 7.81 (t, J = 1.8 Hz, 1H), 7.86 (d, J = 1.8 Hz, 1H), 8.17 (s, 1H), 10.41 (s, 1H), 10.87 (brs, 1H).
mp. 247 - 250°C

Example 23

Production of N-(3-chlorophenyl)-2-cyano-3-(2, 5-difluoro-3, 4-dihydroxyphenyl) propenamide (Compound No. 20 in Table 1):

1, 4-Difluoro-2-methoxy-3-hydroxybenzene (1.60 g, 9.99 mmol) was converted into 2, 5-difluoro-2-methoxy-3-hydroxybenzene (610 mg, 33%) according to the method as described in L. Kirk et al., J. Org. Chem., <u>51</u>, 4073 (1986). This compound (350 mg, 1.86 mmol) was allowed to react with 1M solution of boron tribromide in methylene chloride in the same manner as in Example 2. The crude product obtained above was dissolved in methylene chloride (2 ml), and diisopropylethylamine (0.4 ml, 2.3 mmol) and methoxymethyl chloride (0.45 ml, 6.0 mmol) were added to the solution, which was stirred at room temperature for 1 hour. The reaction mixture was mixed with water, extracted with ethyl acetate-hexane and the extract was concentrated in vacuo. The residue was chromatographed on a column of silica gel to give 2, 5-difluoro-3, 4-bis (methoxymethyloxy) benzaldehyde (200 mg, 41%).

A solution of the aldehyde (97 mg, 0.37 mmol) obtained above, N-(3-chlorophenyl) acetanilide (72 mg, 0.37 mmol) and piperidine (3 µl) in ethanol (2 ml) was refluxed for 2 hours. The mixture was mixed with 3 N aqueous hydrochloric acid (0.5 ml), refluxed for 30 minutes, allowed to cool to room temperature and the resultant crystals were filtered to give the titled compound (52 mg, 41%) as yellow powders.

$^1$HNMR (DMSO-$d_6$, 250 MHz) δ ppm: 7.21 (m, 1H), 7.40 (t, J = 8.1 Hz, 1H), 7.53 - 7.65 (m, 2H), 7.83 (t, J = 2.0 Hz, 1H), 8.24 (s, 1H), 10.55 (S, 1H), 10.70 (brs, 1H).

MP. 255 - 262°C (dec.)

Example 24

Production of 2-cyano-3-(2, 5-difluoro-3, 4-dihydroxyphenyl) propenanilide (Compound No. 99 in Table 1):

The aldehyde, 2, 5-difluoro-3, 4-bis (methoxymethyloxy) benzaldehyde (115 mg, 0.44 mmol) obtained above was allowed to react with cyanoacetanilide (70 mg, 0.44 mmol) in the same manner as in Example 22 to give the objective titled compound (52 mg, 36%) as yellow powders.

$^1$HNMR (DMSO-$d_6$, 250 MHz) δ ppm: 7.14 (t, J = 7.3 Hz, 1H), 7.37 (t, J = 7.6 Hz, 2H), 7.57 (dd, J = 6.2 , 11.6 Hz, 1H), 7.66 (d, J = 8.2 Hz, 1H), 8.22 (s, 1H), 10.39 (s, 1H), 10.7 (brs, 2H).

mp. 261°C (dec.)

Examples 25 - 41

The following products were produced from the corresponding starting compounds in the same manner as in Example 1. Table 6 shows the yields and physical data.

Table 6

| Example No. (Compound No.) | Method* | Yield (%) | $^1$HNMR | mp. (°C) |
|---|---|---|---|---|
| 25 (23) | 1 | 72 | $^1$HNMR (DMSO-$d_6$, 250 MHz) δ ppm; 2.22 (S, 6H), 7.17 (m, 1H), 7.38 (t, J = 8.1 Hz, 1H), 7.60 (m, 1H), 7.68 (brs, 2H), 7.82 (t, J = 1.9 Hz, 1H), 8.07 (s, 1H), 9.54 (S, 1H), 10.34 (S, 1H). | 246 - 248 |
| 26 (100) | 1 | 34 | $^1$HNMR (DMSO-$d_6$, 250 MHz) δ ppm; 7.19 (m, 1H), 7.39 (t, J = 8.1 Hz, 1H), 7.59 (d, J = 8.1 Hz, 1H) 7.81 (t, J = 1.8 Hz, 1H), 8.15 (S, 1H), 8.23 (S, 2H), 10.47 (S, 1H). | 287 - 288 |
| 27 (101) | 1 | 73 | $^1$HNMR (DMSO-$d_6$, 250 MHz) δ ppm; 2.18 (S, 3H), 3.85 (S, 3H), 7.18 (m, 1H), 7.38 (t, J = 8.1 Hz, 1H), 7.46 (brs, 1H), 7.56 - 7.63 (m, 2H), 7.82 (t) J = 1.6 Hz, 1H), 8.12 (S, 1H), 9.90 (S, 1H), 10.37 (S, 1H). | 167 - 169 |
| 28 (102) | 1 | 26 | $^1$HNMR (DMSO-$d_6$, 250 MHz) δ ppm; 7.18 (m, 2H), 7.39 (t, J = 8.1 Hz, 1H), 7.60 (d, J = 8.1 Hz, 1H), 7.82 (S, 1H), 8.17 (dd, J = 2.0, 8.8 Hz, 1H), 8.27 (S, 1H), 8.54 (d, J = 2.0 Hz, 1H), 10.46 (S, 1H). | 265 - 268 |
| 29 (103) | 1 | 60 | $^1$HNMR ($CBCl_3$-DMSO-$d_6$ (5 : 1), 250 MHz) δ ppm; 6.98 (d, J = 8.3 Hz, 1H), 7.10 (m, 1H), 7.28 (t, J = 8.2 Hz, 1H), 7.42 (dd, J = 2.1, 8.3 Hz, 1H), 7.59 (m, 1H), 7.75 (d, J = 2.1 Hz, 1H), 7.85 (t, J = 2.0 Hz, 1H), 8.15 (S, 1H), 9.64 (S, 1H). | 194 - 196 |

Table 6 (continued)

| Example No. (Compound No.) | Method* | Yield (%) | $^1$HNMR | mp. (°C) |
|---|---|---|---|---|
| 30 (104) | 1 | 84 | $^1$HNMR (DMSO-$d_6$, 250 MHz) δ ppm; 7.15 - 7.25 (m, 2H), 7.40 (t, J = 8.1 Hz, 1H), 7.61 (m, 1H), 7.83 (t, J = 1.9 Hz, 1H), 7.89 (dd, J = 2.1, 8.6 Hz, 1H), 8.09 (d, J = 2.1 Hz, 1H), 8.17 (S, 1H), 10.44 (S, 1H), 11.50 (brs, 1H). | 265 - 267 |
| 31 (105) | 1 | 63 | $^1$HNMR (DMSO-$d_6$, 250 MHz) δ ppm; 3.89 (S, 3H), 7.12 (t, J = 7.4 Hz, 1H), 7.36 (t, J = 7.5 Hz, 2H), 7.65 (d, J = 7.5 Hz, 2H), 7.71 (d, J = 1.8 Hz, 1H), 7.84 (d, J = 1.8 Hz, 1H), 8.14 (S, 1H), 10.26 (S, 1H), 10.77 (brs, 1H). | 241 - 243 |
| 32 (106) | 1 | 70 | $^1$HNMR (DMSO-$d_6$, 200 MHz) δ ppm, 3.88 (S, 3H), 7.15 (t, J = 7.0 CHz, 1H), 7.37 (t, J = 7.0 Hz, 2H), 7.56 (m, 2H), 7.66 (d, J = 7.0 Hz, 2H), 8.16 (S, 1H), 10.30 (brs, 1H). | 234 - 235 |
| 33 (107) | 1 | 47 | $^1$HNMR (DMSO-$d_6$, 250 MHz) δ ppm; 6.90 (d, J = 8.4 Hz, 1H), 7.41 (dd, J = 2.2, 8.4 Hz, 1H), 7.57 (dd, J = 7.2, 8.9 Hz, 1H), 7.68 - 7.81 (m, 4H), 7.92 (S, 1H), 10.10 (brs, 1H). | - |
| 34 (108) | 1 | 91 | $^1$HNMR (DMSO-$d_6$, 250 MHz) δ ppm; 6.89 (d, J = 8.3 Hz, 1H), 7.40 (dd, J = 2.2, 8.3 Hz, 1H), 7.62 (d, J = 8.5 Hz, 2H), 7.70 (d, J = 2.1 Hz, 1H), 7.79 (d, J = 8.5 Hz, 2H), 7.92 (S, 1H), 10.05 (brs, 2H) | 205 - 209 (Analyze) |

Table 6 (continued)

| Example No. (Compound No.) | Method* | Yield (%) | $^1$HNMR | mp. (°C) |
|---|---|---|---|---|
| 35 (42) | 10 | 49 | $^1$HNMR (DMSO-$d_6$, 250 MHz) δ ppm; 6.91 (d, J = 8.3 Hz, 1H), 7.36 (dd, J = 2.2, 8.2 Hz, 1H), 7.60 (d, J = 2.2 Hz, 1H), 7.65 (t, J = 8.2 Hz, 1H), 7.97 (J = 2.2, 8.2 Hz, 1H), 8.05 - 8.15 (m, 2H), 8.64 (t, J = 2.2 Hz, 1H), 9.95 (brs, 2H), 10.65 (S, 1H). | 291 - 292 |
| 36 (43) | 10 | 43 | $^1$HNMR (DMSO-$d_6$, 250 MHz) δ ppm; 6.91 (d, J = 8.3 Hz, 1H), 7.35 (brd, J = 8.0 Hz, 1H), 7.50 - 7.65 (m, 3H), 7.93 (m, 1H), 8.00 - 8.15 (m, 2H), 9.94 and 10.48 (brs, 3H). | > 300 |
| 37 (109) | 10 | 24 | $^1$HNMR (DMSO-$d_6$, 250 MHz) δ ppm; 6.85 - 7.00 (m, 2H), 7.30 - 7.50 (m, 3H), 7.55 - 7.65 (m, 2H), 8.04 (S, 1H), 9.80 and 10.36 (brs, 3H). | 268 - 270 |
| 38 (110) | 2 | 18 | $^1$HNMR (DMSO-$d_6$, 250 MHz) δ ppm; 2.15 (S, 3H), 7.17 (m, 1H), 7.24 (d, J = 1.8 Hz, 1H), 7.38 (t, J = 8.1 Hz; 1H), 7.53 (d, J = 1.8 Hz, 1H), 7.59 (m, 1H), 7.82 (t, J = 1.9 Hz, 1H), 8.01 (S, 1H), 9.58, 10.02 and 10.32 (each S, each 1H). | 268 - 270 |
| 39 (111) | 2 | 46 | $^1$HNMR (DMSO-$d_6$, 200 MHz) δ ppm; 7.13 (t, J = 6.0 Hz, 1H), 7.30 - 7.50 (m, 4H), 7.66 (d, J = 6.0 Hz, 2H), 8.06 (S, 1H), 10.27 (brs, 3H). | 268 - 268.5 |

Table 6 (continued)

| Example No. (Compound No.) | Method* | Yield (%) | $^1$HNMR | mp. (°C) |
|---|---|---|---|---|
| 40 (112) | 2 | 61 | $^1$HNMR (DMSO-$d_6$, 250 MHz) δ ppm; 7.12 (t, J = 7.3 Hz, 1H), 7.35 (t, J = 7.9 Hz, 2H), 7.55 - 7.70 (m, 4H), 8.04 (S, 1H), 10.22 (S, 1H), 10.43 and 10.57 (each brs, each 1H). | 265 - 267 |
| 41 (113) | 1, 2 | 44 | $^1$HNMR (DMSO-$d_6$, 500 MHz) δ ppm; 4.41 (d, J = 6.0 Hz, 2H), 7.20 - 7.40 (m, 5H) 7.51 (M, 2H), 8.10 (S, 1H), 5.7 (t, J = 6.0 Hz, 1H), 10.48 (brs, 1H). | 180 - 184 |

Method*: The numerals in the column corresponds to the number of Example wherein synthetic method for preparing the compound is described.

Example 42

Production of N-(3-chlorophenyl)-2-cyano-3-(3-butoxy-4-hydroxy-5-fluorophenyl) propenamide (Compound No. 114);

To a suspension of sodium hydride (688 mg, 28.7 mmol) in dimethylformamide (20 ml) on ice bath was bit by bit added 3-fluorosalicylaldehyde (2.68 g, 19.1 mmol) with stirring. After dropwise adding benzyl bromide (2.73 ml, 22.9 mmol), the mixture was stirred under ice cooling for 30 minutes, stirred at room temperature for 2 hours, mixed with water and the product was shaken with diethyl ether (60 ml x 2). The extracts were washed with 10% aqueous sodium carbonate solution, dilute hydrochloric acid and water in this order, dried over anhydrous magnesium sulfate and concentrated in vacuo to remove the solvent to give 2-benzyloxy-3-fluorobenzaldehyde as an oily material.

To a solution of this aldehyde in methylene chloride (50 ml) was added m-chloroperbensoic acid (5.08 g, 25.0 mmol, 85% purity) with ice cooling, and the resulting mixture was stirred at the same temperature for 2 hours, allowed to warm to room temperature and stirred overnight. The reaction mixture was mixed with saturated aqueous sodium bicarbonate solution and hexane-ethyl acetate mixture and stirred, and the organic layer was separated. The organic layer was washed with saturated aqueous sodium bicarbonate solution and water in this order, dried over anhydrous magnesium sulfate and concentrated in vacuo. The resulting residue was dissolved in methanol (20 ml), and the solution was mixed with potassium carbonate (4.14 g, 30.0 mmol) under ice cooling. The mixture was stirred at room temperature for 2 hours, acidified with dilute hydrochloric acid and extracted with ethyl acetate (50 ml x 2). The extracts were washed with saturated aqueous brine, dried over anhydrous magnesium sulfate and concentrated. The residue was chromatographed on a column of silica gel to give 2-benzyloxy-3-fluorophenol (1.60 g, 38%). A solution of the phenol (540 mg, 2.47 mmol) obtained above, potassium carbonate (681 mg, 4.95 mmol), butyl bromide (0.4 ml, 3.71 mmol) and potassium iodide (616 mg, 3.71 mmol) in dimethylformamide (40 ml) was stirred at 100°C for 1.5 hours. The reaction mixture was poured onto ice water, acidified with dilute hydrochloric acid and extracted with diethyl ether (50 ml x 2). The extracts were washed with 10% aqueous sodium carbonate solution and water in this order, dried over anhydrous magnesium sulfate and concentrated. The resulting residue was chromatographed on a column of silica gel to give l-benzyloxy-2-butoxy-3-fluorobenzene (590 mg, 87%) as an oily material.

This oily material was dissolved in ethanol (3 ml), mixed with 5% palladium carbon (70 mg) and hydrogenated at room temperature for 2 hours. The reaction mixture was filtered, and the filtrate was concentrated in vacuo to give 2-butoxy-6-fluorophenol.

The above phenol was added to a mixture of 50% aqueous dimethylamine (360 mg), 37% aqueous formaldehyde (325 mg) and ethanol (1.5 ml) and refluxed for 3 hours. The reaction mixture was concentrated in vacuo, and the residue was dissolved in chloroform (4 ml). Methyl iodide (2 ml) was added to the solution, which was stirred at room temperature for 2 hours and concentrated in vacuo. The residue was mixed with acetic acid (2 ml), water (2 ml) and hexamethylenetetramine (520 mg, 3.71 mmol), stirred at 120°C (oil bath temperature) for 3 hours, mixed with conc. hydrochloric acid (2 ml), refluxed for 10 minutes and allowed to cool to room temperature. After adding water, the reaction mixture was extracted with diethyl ether (30 ml x 1, 20 ml x 1). The extracts were washed with water (30 ml), dried over anhydrous magnesium sulfate and concentrated to give 3-butoxy-4-hydroxy-5-fluorobenzaldehyde (380 mg, 72% in overall yield from 2-benzyloxy-3-fluorophenol).

The above aldehyde (148 mg, 0.7 mmol) was allowed to condense with N-(3-chlorophenyl) cyanoacetamide (136 mg, 0.7 mmol) in the same manner as in Example 1 to give the objective N-(3-chlorophenyl-)2-cyano-3-(3-butoxy-4-hydroxy-5-fluorophenyl) propenamide (94 mg, 34%).

$^1$HNMR ($CDCl_3$, 250 MHz) δ ppm: 1.01 (t, J = 7.3 Hz, 3H), 1.53 (m, 2H), 1.87 (m, 2H), 4.16 (t, J = 6.5 Hz, 2H), 7.18 (ddd, J = 1.0, 1.6, 7.8 Hz, 1H), 7.31 (t, J = 8.0 Hz, 1H), 7.39 (dd, J = 2.0 10.7 Hz, 1H), 7.41 - 7.47 (m, 2H), 7.77 (t, J = 2.0 Hz, 1H), 7.94 (brs, 1H), 8.26 (s, 1H).

mp. 183 - 185°C

Example 43

Production of 2-cyano-3-(3-butoxy-4-hydroxy-5-fluorophenyl) propenanilide (Compound No. 115):

Condensation of 3-butoxy-4-hydroxy-5-fluorobenzaldehyde (324 mg, 1.53 mmol) obtained in Example 42 and cyanoacetanilide (245 mg, 1.53 mmol) was effected in the same manner as in Example 1 to give the titled compound (300 mg, 58%).

$^1$HNMR (DMSO-$d_6$, 250 MHz) δ ppm: 0.95 (t, J = 7.4 Hz, 3H), 1.47 (m, 2H), 1.77 (m, 2H), 4.07 (t, J = 6.66 Hz, 2H), 7.14 (t, J = 7.3 Hz, 1H), 7.37 (t, J = 7.9 Hz, 2H), 7.50 - 7.60 (m, 2H), 7.67 (d, J = 7.5 Hz, 2H), 8.15 (s, 1H), 10.3 (s, 1H), 10.45 (brs, 1H).

mp. 177 - 179°C

Example 44

Production of N-(3-chlorophenyl)-2-cyano-3-[3-fluoro-4-hydroxy-5-(l-methylethyl) oxyphenyl] propenamide (Compound No. 116):

Alkylation of 2-benzyloxy-3-fluorophenol (1.05 g, 4.81 mmol) obtained in Example 42 was effected with isopropyl iodide (0.96 ml, 9.62 mmol) and potassium carbonate (1.66 g, 12.0 mmol) in the same manner as in Example 42, followed by debenzylation and formylation. Thus, 3-fluoro-4-hydroxy-5-(l-methylethyl) oxybenzaldehyde (325 mg, 34% in overall yield) was obtained.

The above aldehyde (149 mg, 0.75 mmol) was allowed to condense with N-(3-chlorophenyl) cyanoacetamide (146 mg, 0.75 mmol) to give the objective titled compound (185 mg, 69%) as light yellow needles.

$^1$HNMR (DMSO-$d_6$, 250 MHz) δ ppm: 1.34 (d, J = 6.0 Hz, 6H), 4.60 (sep, J = 6.0 Hz, 1H), 7.20 (m, 1H), 7.40 (t, J = 8.1 Hz, 1H), 7.49 - 7.64 (m, 3H), 7.83 (t, J = 1.8 Hz, 1H), 8.16 (s, 1H), 10.37 and 10.47 (each s, 2H).
mp. 181 - 183°C

Example 45

Production of 2-cyano-3-[3-fluoro-4-hydroxy-5-(1-methylethyl)oxyphenyl] propenanilide (Compound No. 117):

Condensation of 3-fluoro-4-hydroxy-5-(1-methylethyl) oxybenzaldehyde (168 mg, 0.85 mmol) obtained in the foregoing Example with cyanoacetanilide (136 mg, 0.85 mmol) in the same manner as in Example 1 to give the objective titled compound (180 mg, 60%) as yellow powdery crystals.

$^1$HNMR ($CDCl_3$, 250 MHz) δ ppm: 1.29 (d, J = 6.1 Hz, 6H), 4.70 (sep. J = 6.1 Hz, 1H), 7.20 (t, J = 7.5 Hz, 1H), 7.30 - 7.52 (m, 2H), 7.39 (t, J = 7.5 Hz, 2H), 7.62 (d, J = 7.5 Hz, 2H), 7.97 (brs, 1H), 8.26 (s, 1H).
mp. 156 - 157°C

Example 46

Production of N-(3-chlorophenyl)-2-cyano-3-(3-butoxy-4-hydroxyphenyl) propenamide (Compound No. 118):

To a solution of 4-bromophenol (7.21 g, 41.6 mmol) in acetic anhydride (25 ml) was added 4 drops of conc. sulfuric acid, and the mixture was stirred at 90°C for 1.5 hours. Ice water was added to the mixture, which was extracted with ethyl acetate (60 ml x 1; 30 ml x 1). The extracts were washed with saturated aqueous sodium bicarbonate solution and water in this order, dried over anhydrous magnesium sulfate and concentrated in vacuo to give 4-bromoacetoxybenzene (8.96 g, quantitatively).

Anhydrous aluminum chloride (11.03 g, 82.8 mmol) was added bit by bit to 4-bromoacetoxybenzene (8.90 g, 41.4 mmol) heated at 80°C with stirring. The mixture was stirred at 100°C for 2.5 hours, allowed to cool, mixed bit by bit with ice water and extracted with ethyl acetate (80 ml x 1; 20 ml x 1). The extract was dried over anhydrous magnesium sulfate and concentrated to give 2-acetyl-4-bromophenol (7.92 g, 89%).

To a solution of 2-acetyl-4-bromophenol (3.63 g, 16.9 mmol) in methylene chloride (20 ml) on ice bath were added diisopropylethylamine (5.88 ml, 33.8 mmol) and chloromethyl methyl ether (1.92 ml, 25.3 mmol) with stirring. The mixture was stirred at room temperature overnight, poured into water and extracted with ethyl acetate. The extract was dried over anhydrous magnesium sulfate and concentrated to give 4-(methoxymethyl) oxy-3-acetylbromobenzene (4.35 g, quantitatively).

To a solution of the bromobenzene (4.35 g, 16.8 mmol) obtained above in chloroform (50 ml) was added m-chloroperbenzoic acid (4.09 g, 20.2 mmol, 85% purity), and the mixture was refluxed. Five hours later, m-chloroperbenzoic acid (2.05 g, 10.1 mmol) was added, and the mixture was refluxed for 29 hours. The reaction mixture was mixed with saturated aqueous sodium bicarbonate solution and hexaneethyl acetate, and the organic layer was separated. The organic layer was washed with saturated aqueous sodium bicarbonate solution and water in this order, dried over anhydrous magnesium sulfate and concentrated. The resulting residue was dissolved in methanol (20 ml) and mixed with potassium carbonate (4.64 g, 33.6 mmol) at 5°C. Twenty minutes later, the mixture was acidified with 3N HCl and extracted with ethyl acetate. The extract was dried over anhydrous magnesium sulfate and concentrated to give 2-(methoxymethyl) oxy-5-bromophenol (3.33 g, 85%).

To a solution of the bromophenol (1.02 g, 4.38 mmol) obtained above in dimethylformamide (15 ml) were added potassium carbonate (1.21 g, 8.76 mmol), butyl bromide (0.71 ml, 6.57 mmol) and potassium iodide (1.09 g, 6.57 mmol), and the resulting mixture was stirred at 100°C with heating. Two hours later, the reaction mixture was poured onto ice water and acidified with dilute hydrochloric acid. The product was extracted with diethyl ether (50 ml x 2), and the extract was washed with 10% aqueous sodium carbonate solution and water in this order, dried over anhydrous magnesium sulfate and concentrated. The residue was chromatographed on a column of silica gel to give 3-butoxy-4-(methoxymethyl) oxybromobenzene (920 mg, 69%) as an oily material.

To a solution of 3-butoxy-4-(methoxymethyl) oxybromobenzene (910 mg, 2.97 mmol) in tetrahydrofuran (15 ml) chilled at -72°C was dropwise added n-butyllithium (2.22 ml, 3.56 mmol, 1.6 M hexane solution) under nitrogen atmosphere, and 20 minutes later dimethylformamide (0.28 ml, 3.62 mmol) was added to the mixture, which was stirred for 30 minutes at the same temperature. The reaction mixture was mixed with water, and the product was extracted with ethyl acetate. The extract was dried over anhydrous magnesium sulfate and concentrated. The residue was chromatographed on a column of silica gel to give 3-butoxy-4-(methoxymethyl) oxybenzaldehyde (556 mg, 84%).

The benzaldehyde (200 mg, 0.9 mmol) obtained above was allowed to condense with N-(3-chlorophenyl) cyanoacetanilide (175 mg, 0.9 mmol) in the same manner as in Example 1 to give N-(3-chlorophenyl)-2-cyano-3-[3-butoxy-4-(methoxymethyl) oxyphenyl] propenamide (210 mg, 57%). To a solution of the condensate (140 mg, 0.34 mmol) in ethanol (5 ml) was added 3 N hydrochloric acid (1 ml), and the mixture was stirred at 60°C for 2 hours with heating. After cooling, the resulting precipitate was filtered to give the objective titled compound (120 mg, 95%) as light yellow needles.

$^1$HNMR ($CDCl_3$, 250 MHz) δ ppm: 1.01 (t, J = 7.3 Hz, 3H), 1.40 - 1.70 (m, 2H), 1.86 (m, 2H), 4.16 (t, J n= 6.4 Hz, 2H), 6.27 (s, 1H), 7.03 (d, J = 8.3 Hz, 1H), 7.17 (m, 1H), 7.31 (t, J = 8.1 Hz, 1H), 7.40 - 7.50 (m, 2H), 7.70 (d, J = 1.9 Hz, 1H), 7.77 (t, J = 1.9 Hz, 1H), 7.95 (brs, 1H), 8.31 (s, 1H).
mp. 154 - 156°C

Example 47

Production of 2-cyano-3-hydroxy-3-(3, 4-dihydroxyphenyl) propenanilide (Compound No. 119):

To a solution of acetonitrile (2.70 ml, 51.9 mmol) in tetrahydrofuran (60 ml) chilled at -72°C was dropwise added a solution of 2 M solution of lithium diisopropylamide in cyclohexane (22.0 ml, 44.0 mmol), and 30 minutes later a solution of 3, 4-bis (t-butyldimethylsilyloxy) benzaldehyde (12.08 g, 32.95 mmol) in tetrahydrofuran (30 ml) was dropwise added in 15 minutes. After stirring at -72°C for 30 minutes, the reaction mixture was poured into saturated aqueous ammonium chloride solution, and the product was extracted with ethyl acetate (100 ml x 2). The extracts were dried over anhydrous magnesium sulfate and concentrated to give 3-hydroxy-3-[3, 4-bis (t-butyldimethylsilyloxy) phenyl] propionitrile (2.64 g, 6.48 mmol). To a solution of this compound in acetone (200 ml) was dropwise added Jones reagent (10 ml, 40 mmol) in 20 minutes under ice cooling, and the resulting mixture was stirred at room temperature for 20 minutes. To the reaction mixture was added 2-propanol until the reaction mixture turned green, and then water (200 ml) was added. The mixture was extracted with hexane-ethyl acetate (70 ml x 2), and the extract was washed with saturated aqueous brine (100 ml), dried over anhydrous magnesium sulfate and concentrated in vacuo. The crude product was chromatographed on a column of silica gel to give the corresponding ketone, 3-oxo-3-[3, 4-bis (t-butyldimethylsilyloxy) phenyl] propionitrile (7.41 g, 56%) as an oily material.

To a solution of the ketone (4.82 g, 11.89 mmol) in benzene (30 ml) were added triethylamine (2.14 ml, 15.46 mmol) and phenyl isocyanate (1.42 ml, 13.08 mmol) under ice cooling, and the resultant mixture was stirred at room temperature for 20 minutes. The mixture was concentrated in vacuo, and the residue was mixed with water (80 ml) and chloroform (80 ml). The aqueous layer was acidified with dilute hydrochloric acid, and the organic layer was separated. The organic extract was dried over anhydrous magnesium sulfate and concentrated. The residue was recrystallized from hexane-chloroform to give an adduct, 2-cyano-3-hydroxy-3-[3, 4-bis (t-butyldimethylsilyloxy)-phenyl]propenanilide (5.15 g, 88%).

To a solution of the adduct (504 mg, 0.96 mmol) in tetrahydrofuran (8 ml) was added a solution of tetrabutyl ammonium fluoride in tetrahydrofuran (2.0 ml, 2.0 mmol, 2 M), and the resulting mixture was stirred for 30 minutes. The mixture was mixed with excessive amount of 3 N aqueous hydrochloric acid and water (20 ml), stirred at room temperature for 2 hours and the product was extracted with ethyl acetate (40 ml). The extract was concentrated, and the residue was suspended in diethyl ether and filtered. The residue was dissolved in a mixture of methanol-ethanol, mixed with ion exchange resin Dowex 50 (500 mg), stirred at room temperature for 3 days and the resin was filtered off. The filtrate was concentrated and the residue was recrystallized from ethanol-water to give the objective titled compound (100 mg, 35%).

$^1$HNMR (DMSO-$d_6$, 250 MHz) δ ppm: 6.83 (d, J = 8.0 Hz, 1H), 7.11 (t, J = 7.3 Hz, 1H), 7.20 - 7.40 (m, 4H), 7.54 (d, J = 7.9 Hz, 2H), 9.50 (brs, 2H), 10.70 (brs, 1H).
mp. 218 - 222°C

Example 48

Production of N-(3-chlorophenyl)-2-cyano-3-hydroxy-3-(3, 4-dihydroxyphenyl) propenamide (Compound No. 120):

The ketone (1.02 g, 2.52 mmol) obtained in Example 47 was allowed to react with 3-chlorophenyl isocyanate (0.34

ml, 2.77 mmol) in the same manner as in Example 47, followed by desilylation, and the crude product was recrystallized from ethanol-water to give the titled compound (100 mg, 31%).

$^1$HNMR (DMSO-$d_6$, 250 MHz) δ ppm: 6.85 (d, J = 8.8 Hz, 1H), 7.19 (d, J = 7.5 Hz, 1H), 7.20 - 7.55 (m, 4H), 9.80 (brs, 3H), 10.63 (brs, 1H).

mp. 235 - 237°C

Example 49

Production of N-(3-toluyl-2-cyano-3-hydroxy-3-(3, 4-dihydroxyphenyl) propenamide (Compound No. 121):

Corresponding ketone (1.018 g, 2.51 mmol) was allowed to react with 3-methylphenyl isocyanate (0.36 ml, 2.76 mmol) in the same manner as in Example 48 to give the titled compound (145 mg, 43%).

$^1$HNMR (DMSO-$d_6$, 250 MHz) δ ppm: 2.29 (s, 3H), 6.88 (d, J = 8.9 Hz, 1H), 6.98 (d, J = 7.3 Hz, 1H), 7.23 (t, J = 7.7 Hz, 1H), 7.30 - 7.42 (m, 4H), 10.15 (s, 2H).

mp. 213 - 215°C

Example 50

Production of N-ethyl-2-cyano-3-(3, 4-dihydroxyphenyl)-2-butenanilide (Compound No. 122):

To a solution of 3, 4-dimethoxyacetophenone (5.68 g, 31.53 mmol) and cyanoacetic acid (2.95 g, 34.68 mmol) in benzene were added acetic acid (1.9 ml) and ammonium acetate (150 mg), and the resultant mixture was refluxed with Dean Stark's dehydrating apparatus for 13 hours while adding ammonium acetate (130 mg) every 4 hours. The mixture was mixed with chloroform (200 ml), 2N aqueous sodium hydroxide solution (100 ml) and water (200 ml), stirred well and the aqueous layer was separated. The aqueous layer was acidified with dilute hydrochloric acid, and the resulting precipitate was filtered off. The residue was washed with diethyl ether to give a carboxylic acid, 2-cyano-3-methyl-3-(3, 4-dimethoxyphenyl) propionic acid (2.25 g, 29%).

This carboxylic acid (234 mg, 0.95 mmol) was allowed to condense with N-ethylaniline (138 mg, 1.14 mmol) in the same manner as in Example 10, and then demethylation was effected in the same manner as in Example 2 to give the objective titled compound (226 mg, 80%).

$^1$HNMR (DMSO-$d_6$, 250 MHz) δ ppm: 1.09 (t, J = 7.1 Hz, 3H), 2.09 (s, 3H), 3.49 (8, J = 7.1 Hz, 2H), 6.40 - 6.70 (m, 1H), 7.20 - 7.60 (m, 5H), 9.15 and 9.49 (each brs, each 1H).

mp. 174 - 177°C

The compounds of the present invention can be prepared easily, and they show more potent tyrosine kinase inhibition than known tyrosine kinase inhibitors, and therefore, they are particularly useful as anticancer agents with less side effects.

## Claims

### Claims for the following Contracting States : DE, FR, GB, IT

1. Styrene derivatives of the following general formula (I):

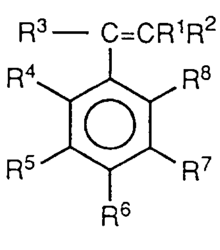

(I)

wherein, $R^1$ and $R^2$ independently each represent cyano group, $CONR^9R^{10}$ [wherein $R^9$ and $R^{10}$ independently each represent hydrogen atom, $C_1$ - $C_5$ alkyl group or

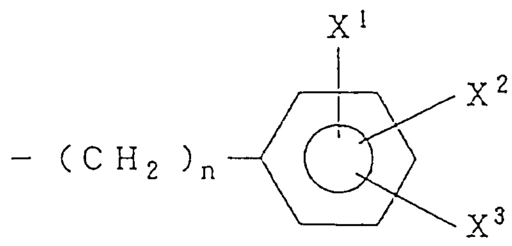

(in which n represents 0 or an integer of from 1 to 5, and $X^1$, $X^2$ and $X^3$ independently each represent hydrogen atom, halogen atom, $C_1$ - $C_5$ alkyl group, $C_1$ - $C_5$ alkoxy group, hydroxy group, nitro group or cyano group) with the proviso that $R^9$ and $R^{10}$ don't represent hydrogen atom at a time] or -$COR^{11}$ [wherein $R^{11}$ represents

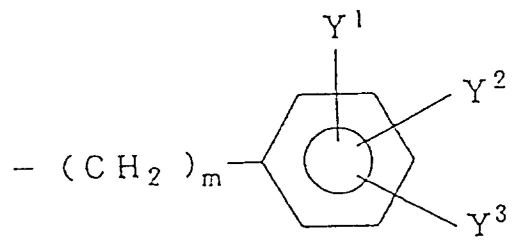

(in which m represents 0 or an integer of from 1 to 5, and $Y^1$, $Y^2$ and $Y^3$ independently each represent hydrogen atom, $C_1$ - $C_5$ alkyl group, $C_1$ - $C_5$ alkoxy group, hydroxy group, or halogen atom)] but $R^1$ and $R^2$ don't represent cyano group at a time;

$R^3$ represents hydrogen atom, $C_1$ - $C_5$ alkyl group or hydroxy group;

$R^4$, $R^5$, $R^6$, $R^7$ and $R^8$ independently each represent hydrogen atom, hydroxy group, halogen atom, $C_1$ - $C_5$ alkoxy group, nitro group, cyano group;

-$NR^{12}R^{13}$ [wherein $R^{12}$ and $R^{13}$ independently each represent hydrogen atom, $C_1$ - $C_5$ alkyl group or benzoyl group];

-$SOpR^{14}$ [wherein p represents 0, 1 or 2, and $R^{14}$ represents $C_1$ - $C_5$ alkyl group or phenyl group];

-$COR^{15}$ [wherein $R^{15}$ represents hydrogen atom, $C_1$ - $C_5$ alkyl group, $C_1$ - $C_5$ alkoxy group, hydroxy group, phenyl group, phenoxy group or -$NHR^{16}$ (in which $R^{16}$ represents $C_1$ - $C_5$ alkyl group or phenyl group)] or $C_1$ - $C_5$ alkyl group optionally substituted by halogen atom,

with the first proviso that at least one of $R^4$ to $R^8$ is hydroxy group, and that when $R^3$ is hydrogen atom and $R^4$ to $R^8$ independently each represent hydrogen atom, hydroxy group, $C_1$ - $C_5$ alkoxy group, -$NR^{12}R^{13}$ (in which $R^{12}$ and $R^{13}$ are as defined above), $C_1$ - $C_5$ alkyl group, then $R^1$ represents cyano group, $R^2$ represents -$CONR^9R^{10}$ [wherein $R^9$ represents hydrogen atom or $C_1$ - $C_5$ alkyl group, $R^{10}$ represents

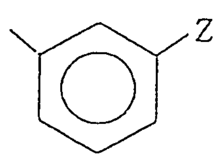

or

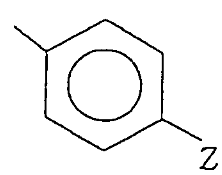

(in which Z represents $C_1$ - $C_5$ alkyl group, halogen atom, nitro group or cyano group)]

or -$COR^{11}$ (wherein $R^{11}$ represents

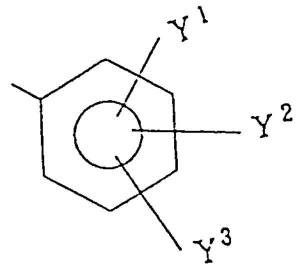

(in which $Y^1$, $Y^2$ and $Y^3$ are as defined above), provided that $R^{11}$ is not

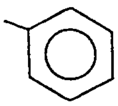 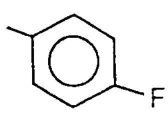 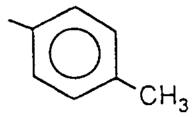 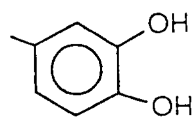

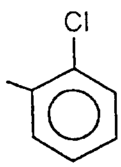 and 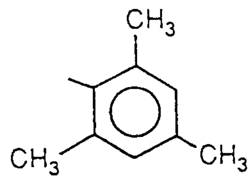 ]]

with the second proviso that when $R^4$, $R^7$ and $R^8$ are H, $R^6$ is hydroxy, $R^5$ is $COR^{15}$, wherein $R^{15}$ represents a hydroxy group or $C_1$ to $C_5$ alkoxy group, $R^3$ is hydrogen, $R^1$ is cyano and $R^2$ is $-CONR^9R^{10}$ wherein $R^9$ is hydrogen, then $R^{10}$ is not

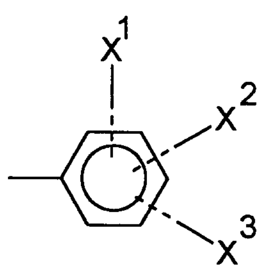

in which $X^1$, $X^2$ and $X^3$ independently represent chlorine or $C_1$ to $C_5$ alkyl group,

and with the third proviso that the styrene of the formula I is not N,N-Diethyl-2-cyano-3-(3,4-dihydroxy-5-nitrophenyl)-acrylamide

or pharmaceutically acceptable salts thereof.

2. A compound according to claim 1, wherein $R^1$ represents cyano group and $R^2$ represents $-CONR^9R^{10}$ [wherein $R^9$ is hydrogen atom or $C_1$ - $C_5$ alkyl group and $R^{10}$ represents

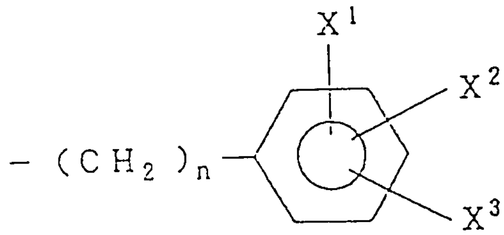

(in which $X^1$ represents hydrogen atom, halogen atom, $C_1$ - $C_5$ alkyl group, nitro group or cyano group, and $X^2$ and $X^3$ represent hydrogen atom, and n represents 0)]

or $-COR^{11}$ [wherein $R^{11}$ represents

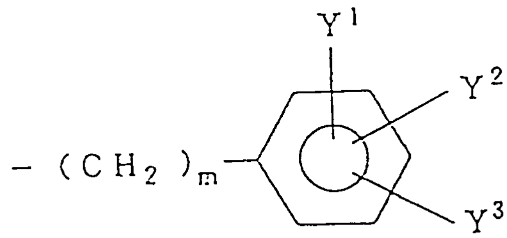

(in which $Y^1$ represents hydrogen atom or halogen atom, $Y^2$ and $Y^3$ represent hydrogen atom and m represents 0)],

$R^3$ represents hydrogen atom, $C_1$ - $C_5$ alkyl group or hydroxy group, $R^4$, $R^5$, $R^6$, $R^7$ and $R^8$ independently each represents hydrogen atom, hydroxy group, halogen atom, $C_1$ - $C_5$ alkoxy group, nitro group, cyano group, -$SO_2R_{14}$ (in which $R^{14}$ represents $C_1$ - $C_5$ alkyl group), $-COR^{15}$ (in which $R^{15}$ represents hydroxy group), or $C_1$ - $C_5$ alkyl group optionally substituted by halogen atom and,

at least one of $R^4$ to $R^5$ is hydroxy group with the proviso that when $R^3$ is hydrogen atom and $R^4$ to $R^8$ independently each represent hydrogen atom, hydroxy group, $C_1$ - $C_5$ alkoxy group or $C_1$ - $C_5$ alkyl group, then $R^2$ is -$CONR^9R^{10}$ [wherein $R^9$ represents hydrogen atom and $R^{10}$ represents

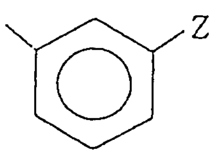

or

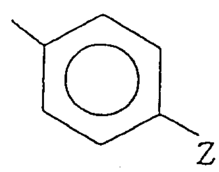

(in which Z represents $C_1$ - $C_5$ alkyl group, halogen atom, nitro group or cyano group)]

or $-COR^{11}$ [wherein $R^{11}$ is

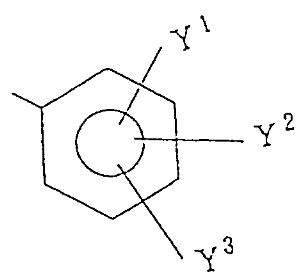

(in which $Y^1$ represents halogen atom and $Y^2$ and $Y^3$ represent hydrogen atom), provided that $R^{11}$ is not

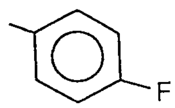

and

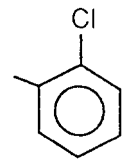

**3.** A compound according to claim 2, wherein $R^1$ represents cyano group,
$R^2$ represents $-CONR^9R^{10}$ [wherein $R^9$ represents hydrogen atom and $R^{10}$ represents

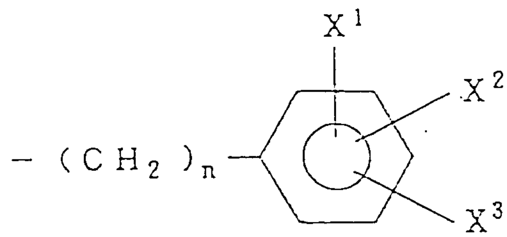

(in which $X^1$ represents hydrogen atom, halogen atom, $C_1$ - $C_5$ alkyl group, nitro group or cyano group, $X^2$ and $X^3$ represent hydrogen atom and n represents 0)]
or $-COR^{11}$ [wherein $R^{11}$ represents

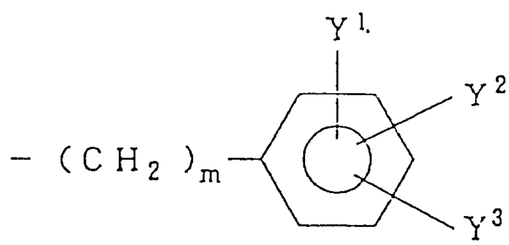

(in which $Y^1$, $Y^2$ and $Y^3$ represent hydrogen atom and m represents 0)],
$R^3$ represents hydrogen atom or hydroxy group,
$R^4$ and $R^8$ independently each represent hydrogen atom, halogen atom or nitro group,
$R^5$ and $R^7$ represent hydroxy group, halogen atom, $C_1$ - $C_5$ alkoxy group, nitro group, cyano group, $SO_2R^{14}$ (in which $R^{14}$ represents $C_1$ - $C_5$ alkyl group), $-COR^{15}$ (in which $R^{15}$ represents hydroxy group) or $C_1$ - $C_5$ alkyl group optionally substituted by halogen atom, $R^6$ represents hydroxy group, with the proviso that when $R^3$, $R^4$ and $R^8$ are hydrogen atom and $R^5$ and $R^7$ independently each represent hydrogen atom, hydroxy group, $C_1$ - $C_5$ alkoxy group or $C_1$ - $C_5$ alkyl group, then $R^2$ is $-CONR^9R^{10}$ [wherein $R^9$ represents hydrogen atom, and $R^{10}$ represents

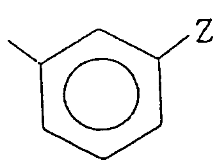

or

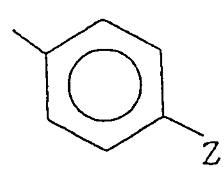

(in which Z represents $C_1$ - $C_5$ alkyl group, halogen atom, nitro group or cyano group)].

**4.** A compound according to claim 3, in which $R^1$ represents cyano group,
$R^2$ represents $-CONR^9R^{10}$ [wherein $R^9$ represents hydrogen atom and $R^{10}$ represents

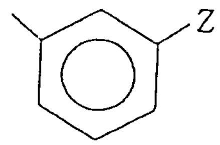

(in which Z represents hydrogen atom or halogen atom)],

$R^3$ represents hydrogen atom or hydroxy group,

$R^4$ represents hydrogen atom, $R^5$ represents hydroxy group, halogen atom, $C_1$ - $C_5$ alkoxy group or $C_1$ - $C_5$ alkyl group,

$R^6$ represents hydroxy group,

$R^7$ represents hydrogen atom, halogen atom, cyano group or $C_1$ - $C_5$ alkyl group and,

$R^8$ represents hydrogen atom, halogen atom or nitro group, with the proviso that when $R^3$ and $R^8$ are hydrogen atom and $R^5$ represents hydroxy group, $C_1$ - $C_5$ alkoxy group or $C_1$ - $C_5$ alkyl group and $R^7$ represents hydrogen atom or $C_1$ - $C_5$ alkyl group, then Z is halogen atom.

**5.** A pharmaceutical composition comprising styrene derivatives of the following general formula (I):

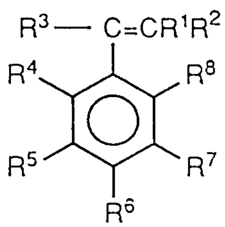

(I)

wherein, $R^1$ and $R^2$ independently each represent cyano group, $CONR^9R^{10}$ [wherein $R^9$ and $R^{10}$ independently each represent hydrogen atom, $C_1$ - $C_5$ alkyl group or

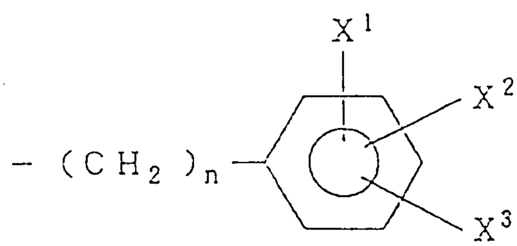

(in which n represents 0 or an integer of from 1 to 5, and $X^1$, $X^2$ and $X^3$ independently each represent hydrogen atom, halogen atom, $C_1$ - $C_5$ alkyl group, $C_1$ - $C_5$ alkoxy group, hydroxy group, nitro group or cyano group) with the proviso that $R^9$ and $R^{10}$ don't represent hydrogen atom at a time] or $-COR^{11}$ [wherein $R^{11}$ represents

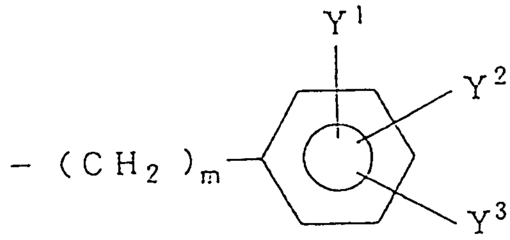

(in which m represents 0 or an integer of from 1 to 5, and $Y^1$, $Y^2$ and $Y^3$ independently each represent hydrogen atom, $C_1$ - $C_5$ alkyl group, $C_1$ - $C_5$ alkoxy group, hydroxy group, or halogen atom)] but $R^1$ and $R^2$ don't represent cyano group at a time;

$R^3$ represents hydrogen atom, $C_1$ - $C_5$ alkyl group or hydroxy group;

$R^4$, $R^5$, $R^6$, $R^7$ and $R^8$ independently each represent hydrogen atom, hydroxy group, halogen atom, $C_1$ - $C_5$ alkoxy group, nitro group, cyano group;

$-NR^{12}R^{13}$ [wherein $R^{12}$ and $R^{13}$ independently each represent hydrogen atom, $C_1$ - $C_5$ alkyl group or benzoyl group];

$-SOpR^{14}$ [wherein p represents 0, 1 or 2, and $R^{14}$ represents $C_1$ - $C_5$ alkyl group or phenyl group];

$-COR^{15}$ [wherein $R^{15}$ represents hydrogen atom, $C_1$ - $C_5$ alkyl group, $C_1$ - $C_5$ alkoxy group, hydroxy group, phenyl group, phenoxy group or $-NHR^{16}$ (in which $R^{16}$ represents $C_1$ - $C_5$ alkyl group or phenyl group)] or $C_1$ - $C_5$ alkyl group optionally substituted by halogen atom,

with the first proviso that at least one of $R^4$ to $R^8$ is hydroxy group, and that when $R^3$ is hydrogen atom and $R^4$ to $R^8$ independently each represent hydrogen atom, hydroxy group, $C_1$ - $C_5$ alkoxy group, $-NR^{12}R^{13}$ (in which $R^{12}$ and $R^{13}$ are as defined above), $C_1$ - $C_5$ alkyl group, then $R^1$ represents cyano group, $R^2$ represents $-CONR^9R^{10}$ [wherein $R^9$ represents hydrogen atom or $C_1$ - $C_5$ alkyl group, $R^{10}$ represents

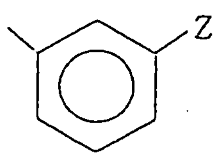

or

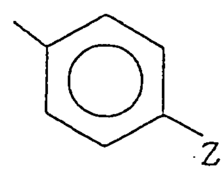

(in which Z represents $C_1$ - $C_5$ alkyl group, halogen atom, nitro group or cyano group)]

or $-COR^{11}$ [wherein $R^{11}$ represents

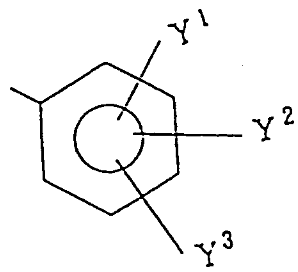

(in which $Y^1$, $Y^2$ and $Y^3$ are as defined above), provided that $R^{11}$ is not

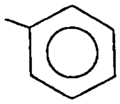
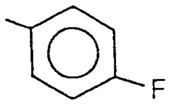
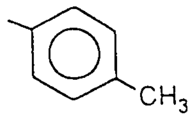
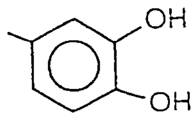

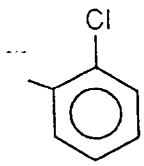

and

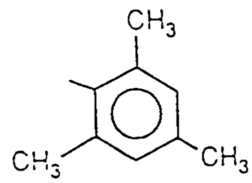 ]]

and with the second proviso that the styrene of the formula I is not N,N-Diethyl-2-cyano-3-(3,4-dihydroxy-5-nitrophenyl)acrylamide,

or pharmaceutically acceptable salts thereof,

and pharmaceutically acceptable carriers therefor.

6. Use of styrene derivatives of the following general formula (I):

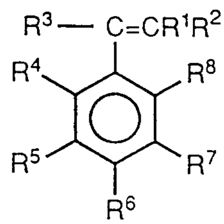

(I)

wherein, $R^1$ and $R^2$ independently each represent cyano group, $CONR^9R^{10}$ [wherein $R^9$ and $R^{10}$ independently each represent hydrogen atom, $C_1$ - $C_5$ alkyl group or

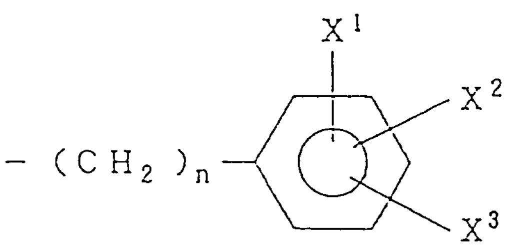

(in which n represents 0 or an integer of from 1 to 5, and $X^1$, $X^2$ and $X^3$ independently each represent hydrogen atom, halogen atom, $C_1$ - $C_5$ alkyl group, $C_1$ - $C_5$ alkoxy group, hydroxy group, nitro group or cyano group) with the proviso that $R^9$ and $R^{10}$ don't represent hydrogen atom at a time] or $-COR^{11}$ [wherein $R^{11}$ represents

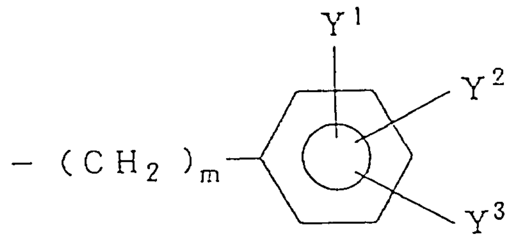

(in which m represents 0 or an integer of from 1 to 5, and $Y^1$, $Y^2$ and $Y^3$ independently each represent hydrogen atom, $C_1$ - $C_5$ alkyl group, $C_1$ - $C_5$ alkoxy group, hydroxy group, or halogen atom)] but $R^1$ and $R^2$ don't represent cyano group at a time;

$R^3$ represents hydrogen atom, $C_1$ - $C_5$ alkyl group or hydroxy group;

$R^4$, $R^5$, $R^6$, $R^7$ and $R^8$ independently each represent hydrogen atom, hydroxy group, halogen atom, $C_1$ - $C_5$ alkoxy group, nitro group, cyano group;

$-NR^{12}R^{13}$ [wherein $R^{12}$ and $R^{13}$ independently each represent hydrogen atom, $C_1$ - $C_5$ alkyl group or benzoyl group];

$-SOpR^{14}$ [wherein p represents 0, 1 or 2, and $R^{14}$ represents $C_1$ - $C_5$ alkyl group or phenyl group];

$-COR^{15}$ [wherein $R^{15}$ represents hydrogen atom, $C_1$ - $C_5$ alkyl group, $C_1$ - $C_5$ alkoxy group, hydroxy group, phenyl group, phenoxy group or $-NHR^{16}$ (in which $R^{16}$ represents $C_1$ - $C_5$ alkyl group or phenyl group)] or $C_1$ - $C_5$ alkyl group optionally substituted by halogen atom,

with the proviso that at least one of $R^4$ to $R^8$ is hydroxy group, and that when $R^3$ is hydrogen atom and $R^4$ to $R^8$ independently each represent hydrogen atom, hydroxy group, $C_1$ - $C_5$ alkoxy group, $-NR^{12}R^{13}$ (in which $R^{12}$ and $R^{13}$ are as defined above), $C_1$ - $C_5$ alkyl group, then $R^1$ represents cyano group, $R^2$ represents $-CONR^9R^{10}$ [wherein $R^9$ represents hydrogen atom or $C_1$ - $C_5$ alkyl group, $R^{10}$ represents

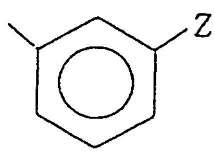

or

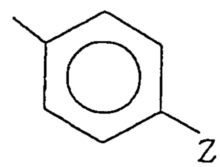

(in which Z represents $C_1$ - $C_5$ alkyl group, halogen atom, nitro group or cyano group)]

or $-COR^{11}$ [wherein $R^{11}$ represents

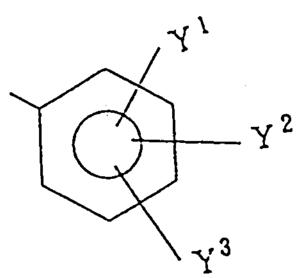

(in which $Y^1$, $Y^2$ and $Y^3$ are as defined above), provided that $R^{11}$ is not

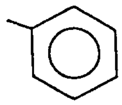
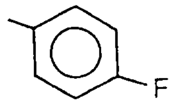
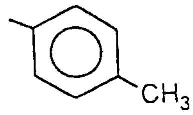
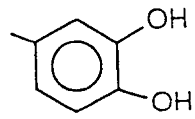

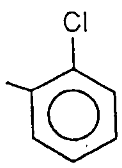
and
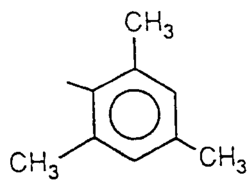
]]

or pharmaceutically acceptable salts thereof,
for the manufacture of a medicament effective in the treatment of cancer.

**Claims for the following Contracting State : ES**

1. Styrene derivatives of the following general formula (I):

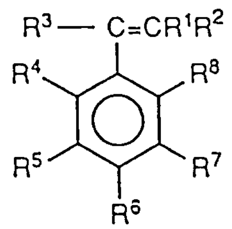

(I)

wherein, $R^1$ and $R^2$ independently each represent cyano group, $CONR^9R^{10}$ [wherein $R^9$ and $R^{10}$ independently each represent hydrogen atom, $C_1$ - $C_5$ alkyl group or

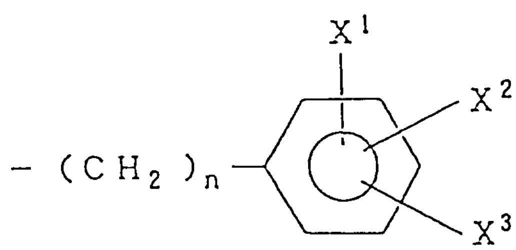

(in which n represents 0 or an integer of from 1 to 5, and $X^1$, $X^2$ and $X^3$ independently each represent hydrogen atom, halogen atom, $C_1$ - $C_5$ alkyl group, $C_1$ - $C_5$ alkoxy group, hydroxy group, nitro group or cyano group) with the proviso that $R^9$ and $R^{10}$ don't represent hydrogen atom at a time] or $-COR^{11}$ [wherein $R^{11}$ represents

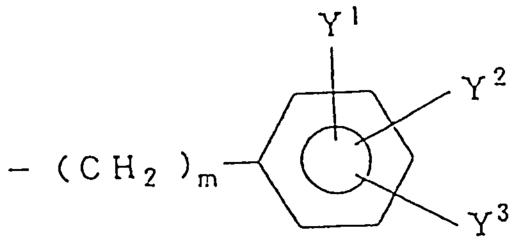

(in which m represents 0 or an integer of from 1 to 5, and $Y^1$, $Y^2$ and $Y^3$ independently each represent hydrogen atom, $C_1$ - $C_5$ alkyl group, $C_1$ - $C_5$ alkoxy group, hydroxy group, or halogen atom)] but $R^1$ and $R^2$ don't represent cyano group at a time;

$R^3$ represents hydrogen atom, $C_1$ - $C_5$ alkyl group or hydroxy group;

$R^4$, $R^5$, $R^6$, $R^7$ and $R^8$ independently each represent hydrogen atom, hydroxy group, halogen atom, $C_1$ - $C_5$ alkoxy group, nitro group, cyano group;

$-NR^{12}R^{13}$ [wherein $R^{12}$ and $R^{13}$ independently each represent hydrogen atom, $C_1$ - $C_5$ alkyl group or benzoyl group];

$-SOpR^{14}$ [wherein p represents 0, 1 or 2, and $R^{14}$ represents $C_1$ - $C_5$ alkyl group or phenyl group];

$-COR^{15}$ [wherein $R^{15}$ represents hydrogen atom, $C_1$ - $C_5$ alkyl group, $C_1$ - $C_5$ alkoxy group, hydroxy group, phenyl group, phenoxy group or $-NHR^{16}$ (in which $R^{16}$ represents $C_1$ - $C_5$ alkyl group or phenyl group)] or $C_1$ - $C_5$ alkyl group optionally substituted by halogen atom,

with the first proviso that at least one of $R^4$ to $R^8$ is hydroxy group, and that when $R^3$ is hydrogen atom and $R^4$ to $R^8$ independently each represent hydrogen atom, hydroxy group, $C_1$ - $C_5$ alkoxy group, $-NR^{12}R^{13}$ (in which $R^{12}$ and $R^{13}$ are as defined above), $C_1$ - $C_5$ alkyl group, then $R^1$ represents cyano group, $R^2$ represents $-CONR^9R^{10}$ [wherein $R^9$ represents hydrogen atom or $C_1$ - $C_5$ alkyl group, $R^{10}$ represents

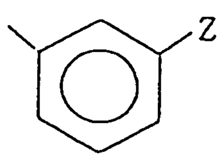

or

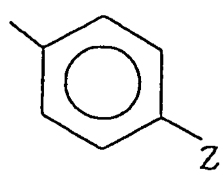

(in which Z represents $C_1$ - $C_5$ alkyl group, halogen atom, nitro group or cyano group)]

or $-COR^{11}$ (wherein $R^{11}$ represents

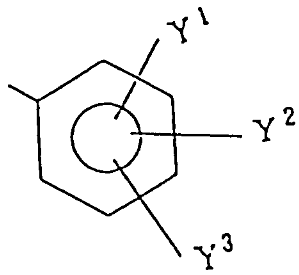

(in which $Y^1$, $Y^2$ and $Y^3$ are as defined above), provided that $R^{11}$ is not

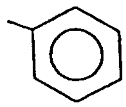 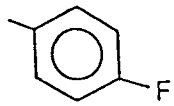 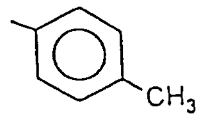 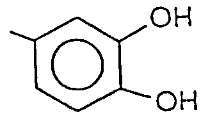

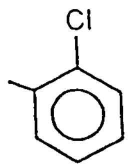 and , 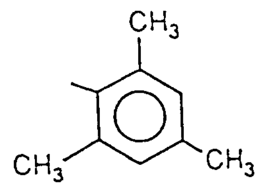 ]]

with the second proviso that when $R^4$, $R^7$ and $R^8$ are H, $R^6$ is hydroxy, $R^5$ is $COR^{15}$, wherein $R^{15}$ represents a hydroxy group or $C_1$ to $C_5$ alkoxy group, $R^3$ is hydrogen, $R^1$ is cyano and $R^2$ is $-CONR^9R^{10}$ wherein $R^9$ is hydrogen, then $R^{10}$ is not

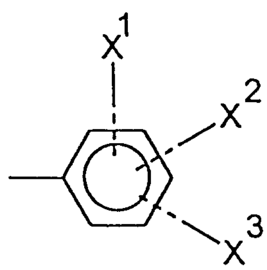

in which $X^1$, $X^2$ and $X^3$ independently represent chlorine or $C_1$ to $C_5$ alkyl group,

and with the third proviso that the styrene of the formula I is not N,N-Diethyl-2-cyano-3-(3,4-dihydroxy-5-nitrophenyl)-acrylamide,

or pharmaceutically acceptable salts thereof.

2. A compound according to claim 1, wherein $R^1$ represents cyano group and $R^2$ represents $-CONR^9R^{10}$ [wherein $R^9$ is hydrogen atom or $C_1$ - $C_5$ alkyl group and $R^{10}$ represents

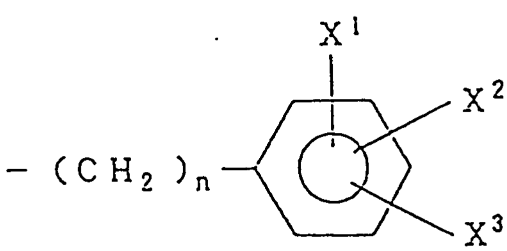

(in which $X^1$ represents hydrogen atom, halogen atom, $C_1$ - $C_5$ alkyl group, nitro group or cyano group, and $X^2$ and $X^3$ represent hydrogen atom, and n represents 0)]

or $-COR^{11}$ (wherein $R^{11}$ represents

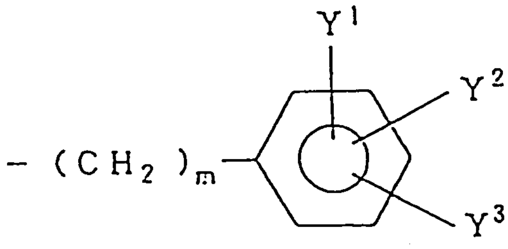

(in which $Y^1$ represents hydrogen atom or halogen atom, $Y^2$ and $Y^3$ represent hydrogen atom and m represents 0)],

$R^3$ represents hydrogen atom, $C_1$ - $C_5$ alkyl group or hydroxy group, $R^4$, $R^5$, $R^6$, $R^7$ and $R^8$ independently each represents hydrogen atom, hydroxy group, halogen atom, $C_1$ - $C_5$ alkoxy group, nitro group, cyano group, -$SO_2R_{14}$ (in which $R^{14}$ represents $C_1$ - $C_5$ alkyl group), -$COR^{15}$ (in which $R^{15}$ represents hydroxy group), or $C_1$ - $C_5$ alkyl group optionally substituted by halogen atom and,

at least one of $R^4$ to $R^5$ is hydroxy group with the proviso that when $R^3$ is hydrogen atom and $R^4$ to $R^8$ independently each represent hydrogen atom, hydroxy group, $C_1$ - $C_5$ alkoxy group or $C_1$ - $C_5$ alkyl group, then $R^2$ is -$CONR^9R^{10}$ [wherein $R^9$ represents hydrogen atom and $R^{10}$ represents

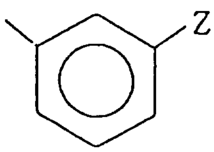

or

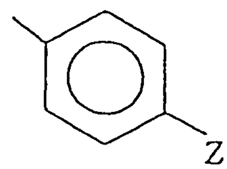

(in which Z represents $C_1$ - $C_5$ alkyl group, halogen atom, nitro group or cyano group)]

or -$COR^{11}$ [wherein $R^{11}$ is

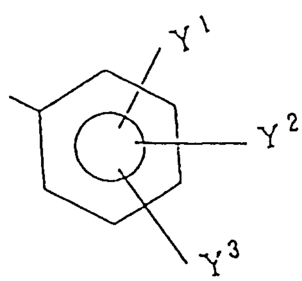

(in which $Y^1$ represents halogen atom and $Y^2$ and $Y^3$ represent hydrogen atom), provided that $R^{11}$ is not

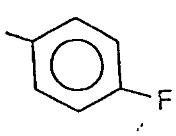

and

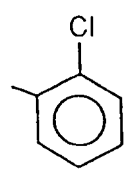

3. A compound according to claim 2, wherein $R^1$ represents cyano group,

$R^2$ represents -$CONR^9R^{10}$ [wherein $R^9$ represents hydrogen atom and $R^{10}$ represents

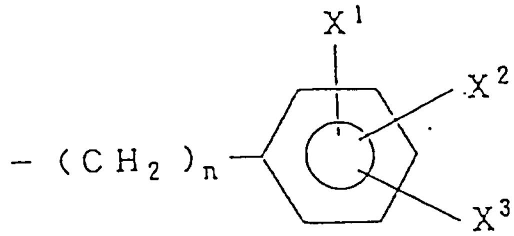

(in which $X^1$ represents hydrogen atom, halogen atom, $C_1$ - $C_5$ alkyl group, nitro group or cyano group, $X^2$ and $X^3$ represent hydrogen atom and n represents 0)]

or $-COR^{11}$ [wherein $R^{11}$ represents

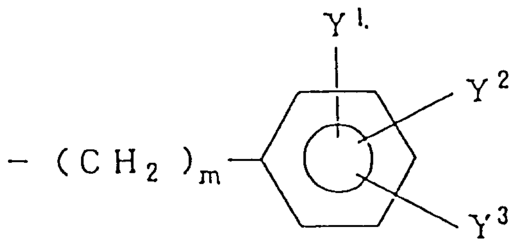

(in which $Y^1$, $Y^2$ and $Y^3$ represent hydrogen atom and m represents 0)],

$R^3$ represents hydrogen atom or hydroxy group,

$R^4$ and $R^8$ independently each represent hydrogen atom, halogen atom or nitro group,

$R^5$ and $R^7$ represent hydroxy group, halogen atom, $C_1$ - $C_5$ alkoxy group, nitro group, cyano group, $SO_2R^{14}$ (in which $R^{14}$ represents $C_1$ - $C_5$ alkyl group), $-COR^{15}$ (in which $R^{15}$ represents hydroxy group) or $C_1$ - $C_5$ alkyl group optionally substituted by halogen atom, $R^6$ represents hydroxy group, with the proviso that when $R^3$, $R^4$ and $R^8$ are hydrogen atom and $R^5$ and $R^7$ independently each represent hydrogen atom, hydroxy group, $C_1$ - $C_5$ alkoxy group or $C_1$ - $C_5$ alkyl group, then $R^2$ is $-CONR^9R^{10}$ [wherein $R^9$ represents hydrogen atom, and $R^{10}$ represents

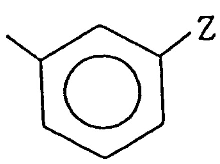

or

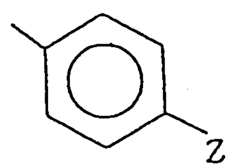

(in which Z represents $C_1$ - $C_5$ alkyl group, halogen atom, nitro group or cyano group)].

**4.** A compound according to claim 3, in which $R^1$ represents cyano group,

$R^2$ represents $-CONR^9R^{10}$ [wherein $R^9$ represents hydrogen atom and $R^{10}$ represents

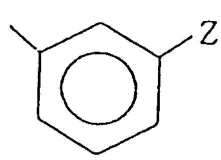

(in which Z represents hydrogen atom or halogen atom)],

$R^3$ represents hydrogen atom or hydroxy group,

$R^4$ represents hydrogen atom, $R^5$ represents hydroxy group, halogen atom, $C_1$ - $C_5$ alkoxy group or $C_1$ - $C_5$ alkyl group,

$R^6$ represents hydroxy group,

$R^7$ represents hydrogen atom, halogen atom, cyano group or $C_1$ - $C_5$ alkyl group and,

$R^8$ represents hydrogen atom, halogen atom or nitro group, with the proviso that when $R^3$ and $R^8$ are hydrogen atom and $R^5$ represents hydroxy group, $C_1$ - $C_5$ alkoxy group or $C_1$ - $C_5$ alkyl group and $R^7$ represents hydrogen atom or $C_1$ - $C_5$ alkyl group, then Z is halogen atom.

**5.** A pharmaceutical composition comprising styrene derivatives of the following general formula (I):

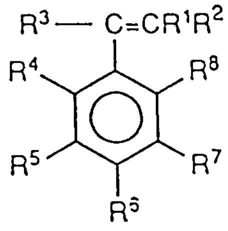

(I)

wherein, $R^1$ and $R^2$ independently each represent cyano group, $CONR^9R^{10}$ [wherein $R^9$ and $R^{10}$ independently each represent hydrogen atom, $C_1$ - $C_5$ alkyl group or

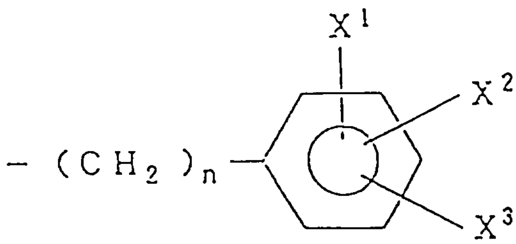

(in which n represents 0 or an integer of from 1 to 5, and $X^1$, $X^2$ and $X^3$ independently each represent hydrogen atom, halogen atom, $C_1$ - $C_5$ alkyl group, $C_1$ - $C_5$ alkoxy group, hydroxy group, nitro group or cyano group) with the proviso that $R^9$ and $R^{10}$ don't represent hydrogen atom at a time) or $-COR^{11}$ [wherein $R^{11}$ represents

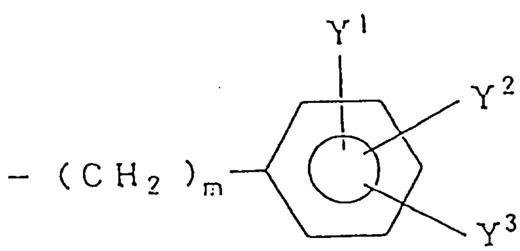

(in which m represents 0 or an integer of from 1 to 5, and $Y^1$, $Y^2$ and $Y^3$ independently each represent hydrogen atom, $C_1$ - $C_5$ alkyl group, $C_1$ - $C_5$ alkoxy group, hydroxy group, or halogen atom)] but $R^1$ and $R^2$ don't represent cyano group at a time;

$R^3$ represents hydrogen atom, $C_1$ - $C_5$ alkyl group or hydroxy group;

$R^4$, $R^5$, $R^6$, $R^7$ and $R^8$ independently each represent hydrogen atom, hydroxy group, halogen atom, $C_1$ - $C_5$ alkoxy group, nitro group, cyano group;

$-NR^{12}R^{13}$ [wherein $R^{12}$ and $R^{13}$ independently each represent hydrogen atom, $C_1$ - $C_5$ alkyl group or benzoyl group];

$-SOpR^{14}$ [wherein p represents 0, 1 or 2, and $R^{14}$ represents $C_1$ - $C_5$ alkyl group or phenyl group];

$-COR^{15}$ [wherein $R^{15}$ represents hydrogen atom, $C_1$ - $C_5$ alkyl group, $C_1$ - $C_5$ alkoxy group, hydroxy group, phenyl group, phenoxy group or $-NHR^{16}$ (in which $R^{16}$ represents $C_1$ - $C_5$ alkyl group or phenyl group)] or $C_1$ - $C_5$ alkyl group optionally substituted by halogen atom,

with the first proviso that at least one of $R^4$ to $R^8$ is hydroxy group, and that when $R^3$ is hydrogen atom and $R^4$ to $R^8$ independently each represent hydrogen atom, hydroxy group, $C_1$ - $C_5$ alkoxy group, $-NR^{12}R^{13}$ (in which $R^{12}$ and $R^{13}$ are as defined above), $C_1$ - $C_5$ alkyl group, then $R^1$ represents cyano group, $R^2$ represents $-CONR^9R^{10}$ [wherein $R^9$ represents hydrogen atom or $C_1$ - $C_5$ alkyl group, $R^{10}$ represents

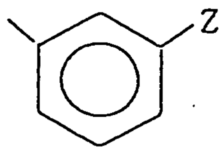

or

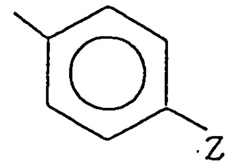

(in which Z represents $C_1$ - $C_5$ alkyl group, halogen atom, nitro group or cyano group)]

or $-COR^{11}$ [wherein $R^{11}$ represents

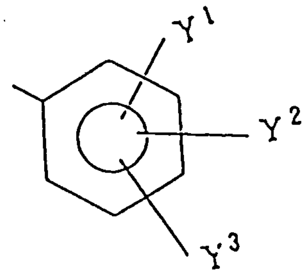

(in which $Y^1$, $Y^2$ and $Y^3$ are as defined above), provided that $R^{11}$ is not

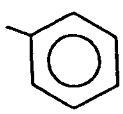

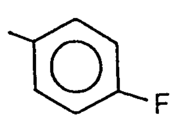

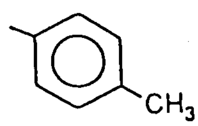

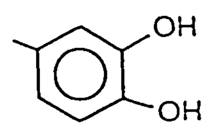

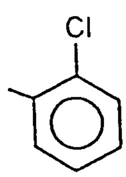

and

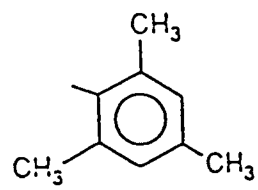

]]

and with the second proviso that the styrene of the formula I is not N,N-Diethyl-2-cyano-3-(3,4-dihydroxy-5-nitrophenyl)acrylamide,

or pharmaceutically acceptable salts thereof,

and pharmaceutically acceptable carriers therefor.

**6.** Use of styrene derivatives of the following general formula (I):

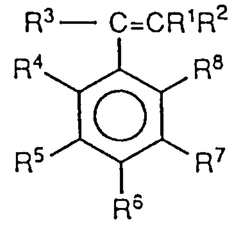

(I)

wherein, $R^1$ and $R^2$ independently each represent cyano group, $CONR^9R^{10}$ [wherein $R^9$ and $R^{10}$ independently each represent hydrogen atom, $C_1$ - $C_5$ alkyl group or

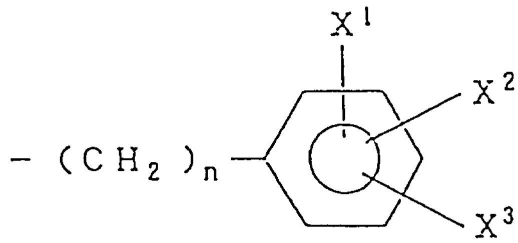

(in which n represents 0 or an integer of from 1 to 5, and $X^1$, $X^2$ and $X^3$ independently each represent hydrogen atom, halogen atom, $C_1$ - $C_5$ alkyl group, $C_1$ - $C_5$ alkoxy group, hydroxy group, nitro group or cyano group) with the proviso that $R^9$ and $R^{10}$ don't represent hydrogen atom at a time] or $-COR^{11}$ [wherein $R^{11}$ represents

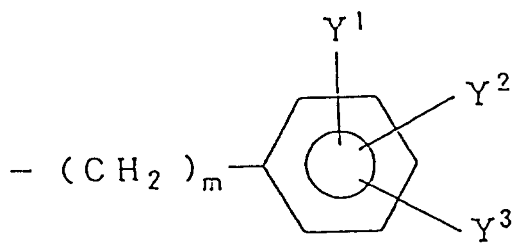

(in which m represents 0 or an integer of from 1 to 5, and $Y^1$, $Y^2$ and $Y^3$ independently each represent hydrogen atom, $C_1$ - $C_5$ alkyl group, $C_1$ - $C_5$ alkoxy group, hydroxy group, or halogen atom)] but $R^1$ and $R^2$ don't represent cyano group at a time;

$R^3$ represents hydrogen atom, $C_1$ - $C_5$ alkyl group or hydroxy group;

$R^4$, $R^5$, $R^6$, $R^7$ and $R^8$ independently each represent hydrogen atom, hydroxy group, halogen atom, $C_1$ - $C_5$ alkoxy group, nitro group, cyano group;

$-NR^{12}R^{13}$ [wherein $R^{12}$ and $R^{13}$ independently each represent hydrogen atom, $C_1$ - $C_5$ alkyl group or benzoyl group];

$-SOpR^{14}$ [wherein p represents 0, 1 or 2, and $R^{14}$ represents $C_1$ - $C_5$ alkyl group or phenyl group];

$-COR^{15}$ [wherein $R^{15}$ represents hydrogen atom, $C_1$ - $C_5$ alkyl group, $C_1$ - $C_5$ alkoxy group, hydroxy group, phenyl group, phenoxy group or $-NHR^{16}$ (in which $R^{16}$ represents $C_1$ - $C_5$ alkyl group or phenyl group)] or $C_1$ - $C_5$ alkyl group optionally substituted by halogen atom,

with the proviso that at least one of $R^4$ to $R^8$ is hydroxy group, and that when $R^3$ is hydrogen atom and $R^4$ to $R^8$ independently each represent hydrogen atom, hydroxy group, $C_1$ - $C_5$ alkoxy group, $-NR^{12}R^{13}$ (in which $R^{12}$ and $R^{13}$ are as defined above), $C_1$ - $C_5$ alkyl group, then $R^1$ represents cyano group, $R^2$ represents $-CONR^9R^{10}$ [wherein $R^9$ represents hydrogen atom or $C_1$ - $C_5$ alkyl group, $R^{10}$ represents

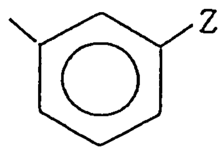

or

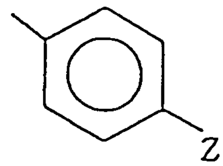

(in which Z represents $C_1$ - $C_5$ alkyl group, halogen atom, nitro group or cyano group)] or -$COR^{11}$ [wherein $R^{11}$ represents

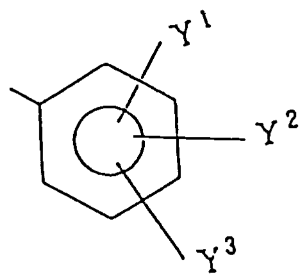

(in which $Y^1$, $Y^2$ and $Y^3$ are as defined above), provided that $R^{11}$ is not

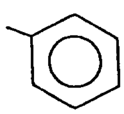 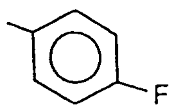 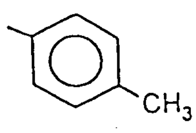 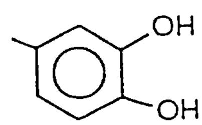

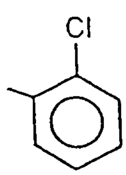 and 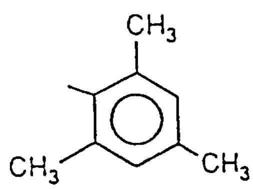 ]]

or pharmaceutically acceptable salts thereof, for the manufacture of a medicament effective in the treatment of cancer.

**7.** Process for the preparation of styrene derivatives of the following general formula (I):

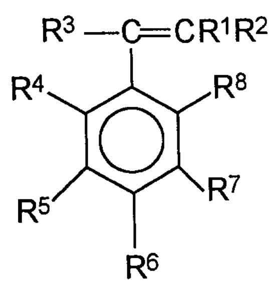

(I)

wherein $R^1$ and $R^2$ independently each represent cyano group, $CONR^9R^{10}$ [wherein $R^9$ and $R^{10}$ independently each represent hydrogen atom, $C_1$-$C_5$ alkyl group or

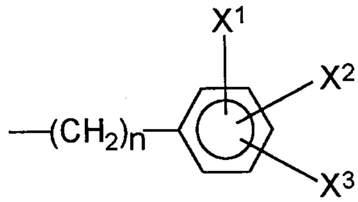

(in which n represents 0 or an integer of from 1 to 5, and $X^1$, $X^2$ and $X^3$ independently each represent hydrogen atom, halogen atom, $C_1$-$C_5$ alkyl group, $C_1$-$C_5$ alkoxy group, hydroxy group, nitro group or cyano group) with the proviso that $R^9$ and $R^{10}$ do not represent hydrogen atom at a time] or -$COR^{11}$ [wherein $R^{11}$ represents

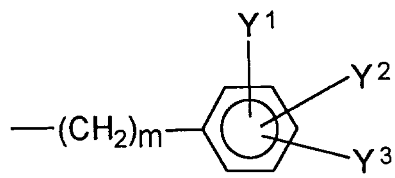

(in which m represents 0 or an integer of from 1 to 5, and $Y^1$, $Y^2$ and $Y^3$ independently each represent hydrogen atom, $C_1$-$C_5$ alkyl group, $C_1$-$C_5$ alkoxy group, hydroxy group or halogen atom)] but $R^1$ and $R^2$ do not represent cyano group at a time;

$R^3$ represents hydrogen atom or $C_1$-$C_5$ alkyl group;

$R^4$, $R^5$, $R^6$, $R^7$ and $R^8$ independently each represents hydrogen atom, hydroxy group, halogen atom, $C_1$-$C_5$ alkoxy group, nitro group, cyano group;

-$NR^{12}R^{13}$ [wherein $R^{12}$ and $R^{13}$ independently each represent hydrogen atom, $C_1$-$C_5$ alkyl group or benzoyl group];

-$SOpR^{14}$ [wherein p represents 0, 1 or 2, and $R^{14}$ represents $C_1$-$C_5$ alkyl group or phenyl group];

-$COR^{15}$ [wherein $R^{15}$ represents hydrogen atom, $C_1$-$C_5$ alkyl group, $C_1$-$C_5$ alkoxy group, hydroxy group, phenyl group, phenoxy group or -$NHR^{16}$ (in which $R^{16}$ represents $C_1$-$C_5$ alkyl group or phenyl group)] or $C_1$-$C_5$ alkyl group optionally substituted by halogen atom,

with the first proviso that at least one of $R^4$ to $R^8$ is hydroxy group, and that when $R^3$ is hydrogen atom and $R^4$ to $R^8$ independently each represent hydrogen atom, hydroxy group, $C_1$-$C_5$ alkoxy group, -$NR^{12}R^{13}$ (in which $R^{12}$ and $R^{13}$ are defined as above), $C_1$-$C_5$ alkyl group, then $R^1$ represents cyano group, $R^2$ represents -$CONR^9R^{10}$ [wherein $R^9$ represents hydrogen atom or $C_1$-$C_5$ alkyl group, $R^{10}$ represents

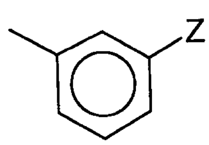 or 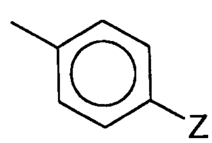

(in which Z represents $C_1$-$C_5$ alkyl group, halogen atom, nitro group or cyano group)]

or -$COR^{11}$ [wherein $R^{11}$ represents

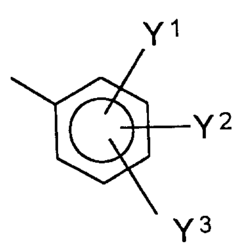

(in which $Y^1$, $Y^2$ and $Y^3$ are defined as above), provided that $R^{11}$ is not

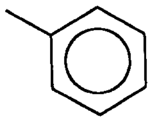 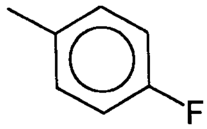 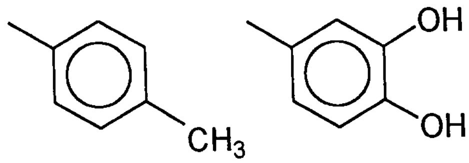

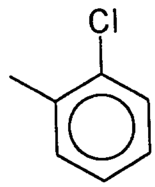

and

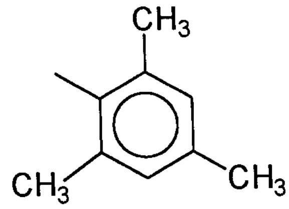

]]

with the second proviso that when $R^4$, $R^7$ and $R^8$ are H, $R^6$ is hydroxy, $R^5$ is $COR^{15}$, wherein $R^{15}$ represents a hydroxy group or $C_1$-$C_5$ alkoxy group, $R^3$ is hydrogen, $R^1$ is cyano and $R^2$ is $-CONR^9R^{10}$ wherein $R^9$ is hydrogen, then $R^{10}$ is not

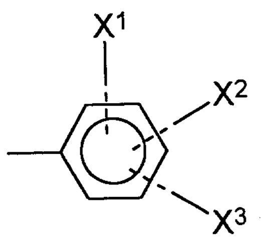

in which $X^1$, $X^2$ and $X^3$ independently represent chlorine or $C_1$-$C_5$ alkyl group,

and with the third proviso that the styrene of the formula (I) is not N,N-diethyl-2-cyano-3-(3,4-dihydroxy-5-nitrophenyl)-acrylamide,

by condensing the compound of the formula (II):

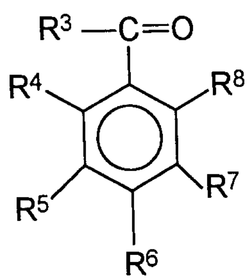

(II)

wherein $R^3$ to $R^8$ have the same meaning as above, with the compound of the formula (III):

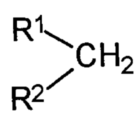

(III)

wherein $R^1$ and $R^2$ have the same meaning as above

or by protecting the hydroxygroup(s) in the above compound of formula (II), condensing the product obtained thereby with the above compound of formula (III) and deprotecting said hydroxy group(s) of the product obtained by said condensation step.

**8.** Process for the preparation of styrene derivatives of the following general formula (Ia):

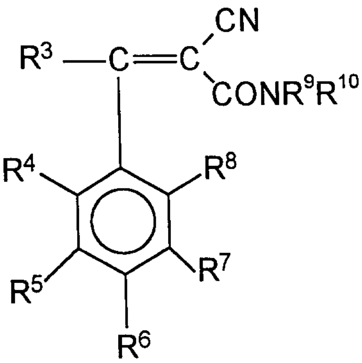

(Ia)

wherein $R^9$ and $R^{10}$ independently each represent hydrogen atom, $C_1$-$C_5$ alkyl group or

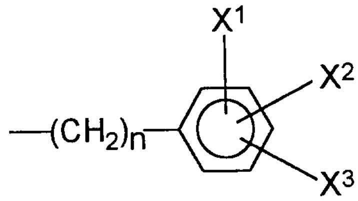

(in which n represents 0 or an integer of from 1 to 5, and $X^1$, $X^2$ and $X^3$ independently each represent hydrogen atom, halogen atom, $C_1$-$C_5$ alkyl group, $C_1$-$C_5$ alkoxy group, hydroxy group, nitro group or cyano group) with the proviso that $R^9$ and $R^{10}$ do not represent hydrogen atom at a time;

$R^3$ represents hydrogen atom or $C_1$-$C_5$ alkyl group;

$R^4$, $R^5$, $R^6$, $R^7$ and $R^8$ independently each represents hydrogen atom, hydroxy group, halogen atom, $C_1$-$C_5$ alkoxy group, nitro group, cyano group;

$-NR^{12}R^{13}$ [wherein $R^{12}$ and $R^{13}$ independently each represent hydrogen atom, $C_1$-$C_5$ alkyl group or benzoyl group];

$-SOpR^{14}$ [wherein p represents 0, 1 or 2, and $R^{14}$ represents $C_1$-$C_5$ alkyl group or phenyl group];

$-COR^{15}$ [wherein $R^{15}$ represents hydrogen atom, $C_1$-$C_5$ alkyl group, $C_1$-$C_5$ alkoxy group, hydroxy group, phenyl group, phenoxy group or $-NHR^{16}$ (in which $R^{16}$ represents $C_1$-$C_5$ alkyl group or phenyl group)] or $C_1$-$C_5$ alkyl group optionally substituted by halogen atom,

with the first proviso that at least one of $R^4$ to $R^8$ is hydroxy group, and that when $R^3$ is hydrogen atom and $R^4$ to $R^8$ independently each represent hydrogen atom, hydroxy group, $C_1$-$C_5$ alkoxy group, $-NR^{12}R^{13}$ (in which $R^{12}$ and $R^{13}$ are defined as above), $C_1$-$C_5$ alkyl group then $R^9$ represents hydrogen atom or $C_1$-$C_5$ alkyl group and $R^{10}$ represents

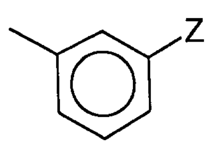

or

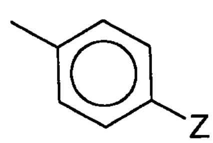

(in which Z represents $C_1$-$C_5$ alkyl group, halogen atom, nitro group or cyano group);

with the second proviso that when $R^4$, $R^7$ and $R^8$ are H, $R^6$ is hydroxy, $R^5$ is $COR^{15}$, wherein $R^{15}$ represents a hydroxy group or $C_1$-$C_5$ alkoxy group, $R^3$ is hydrogen and $R^9$ is hydrogen, then $R^{10}$ is not

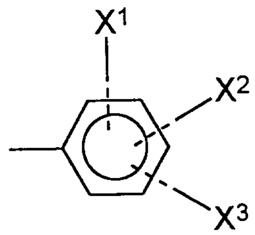

in which $X^1$, $X^2$ and $X^3$ independently represent chlorine or $C_1$-$C_5$ alkyl group,

and with the third proviso that the styrene of the formula I is not N,N-diethyl-2-cyano-3-(3,4-dihydroxy-5-nitrophenyl)-acrylamide,

comprising the following steps:

condensing the compound of the general formula (II):

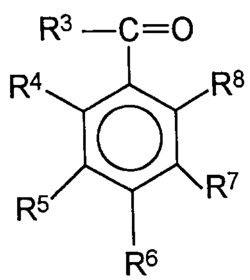

(II)

wherein $R^3$ to $R^8$ have the same meaning as above, with cyanoacetic acid to give the compound of the following general formula (IV):

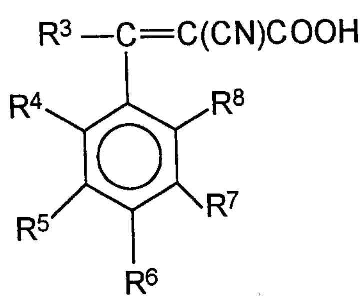

(IV)

wherein $R^3$ to $R^8$ have the same meaning as above, and condensing said compound (IV) with the amine of the following general formula (V):

$$R^9R^{10}NH \qquad (V)$$

wherein $R^9$ to $R^{10}$ have the same meaning as above.

**9.** Process for the preparation of styrene derivatives of the following general formula (Ib):

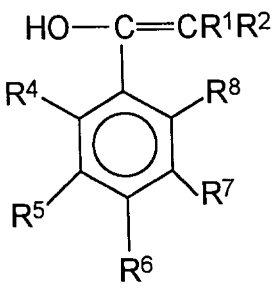

(Ib)

wherein $R^1$ and $R^2$ independently each represent cyano group, $CONR^9R^{10}$ [wherein $R^9$ and $R^{10}$ independently each represent hydrogen atom, $C_1$-$C_5$ alkyl group or

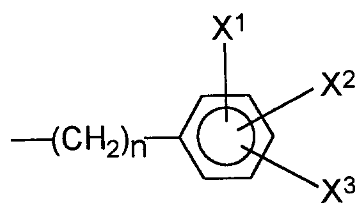

(in which n represents 0 or an integer of from 1 to 5, and $X^1$, $X^2$ and $X^3$ independently each represent hydrogen atom, halogen atom, $C_1$-$C_5$ alkyl group, $C_1$-$C_5$ alkoxy group, hydroxy group, nitro group or cyano group) with the proviso that $R^9$ and $R^{10}$ do not represent hydrogen atom at a time] or $-COR^{11}$ [wherein $R^{11}$ represents

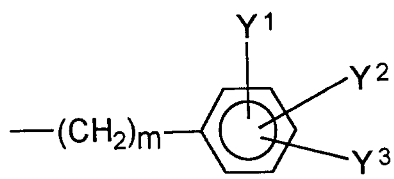

(in which m represents 0 or an integer of from 1 to 5, and $Y^1$, $Y^2$ and $Y^3$ independently each represent hydrogen atom, $C_1$-$C_5$ alkyl group, $C_1$-$C_5$ alkoxy group, hydroxy group or halogen atom)] but $R^1$ and $R^2$ do not represent cyano group at a time;

$R^4$, $R^5$, $R^6$, $R^7$ and $R^8$ independently each represents hydrogen atom, hydroxy group, halogen atom, $C_1$-$C_5$ alkoxy group, nitro group, cyano group;

$-NR^{12}R^{13}$ [wherein $R^{12}$ and $R^{13}$ independently each represent hydrogen atom, $C_1$-$C_5$ alkyl group or benzoyl group];

$-SOpR^{14}$ [wherein p represents 0, 1 or 2, and $R^{14}$ represents $C_1$-$C_5$ alkyl group or phenyl group];

$-COR^{15}$ [wherein $R^{15}$ represents hydrogen atom, $C_1$-$C_5$ alkyl group, $C_1$-$C_5$ alkoxy group, hydroxy group, phenyl group, phenoxy group or $-NHR^{16}$ (in which $R^{16}$ represents $C_1$-$C_5$ alkyl group or phenyl group)] or $C_1$-$C_5$ alkyl group optionally substituted by halogen atom,

with the proviso that at least one of $R^4$ to $R^8$ is hydroxy group,

comprising the following steps:

protecting the hydroxy group in compounds of the general formula (IIa):

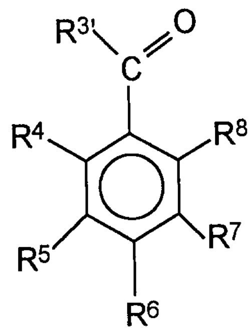

(IIa)

wherein $R^4$ to $R^8$ are defined as above and $R^{3'}$ is hydroxy;

reacting with thionyl chloride or oxalyl chloride to give the compound of the formula (IIa) wherein $R^{3'}$ is chlorine and at least one of $R^4$ to $R^8$ is acyloxy or trialkylsiloxy and the remaining substituents are defined as above;

reacting the product obtained thereby with the compound of the general formula (III):

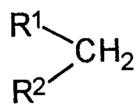

(III)

wherein $R^1$ and $R^2$ are defined as above; and

deprotecting the hydroxy groups, if necessary.

**10.** Process for the preparation of styrene derivatives of the following general formula (Ic):

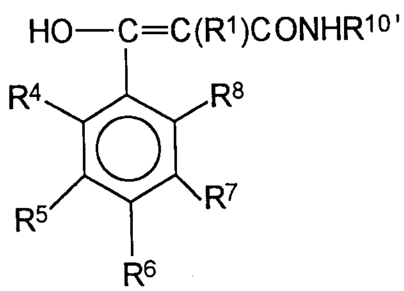

(Ic)

wherein $R^1$ represents cyano group or $CONR^9R^{10}$ [wherein $R^9$ and $R^{10}$ independently each represent hydrogen atom, $C_1$-$C_5$ alkyl group or

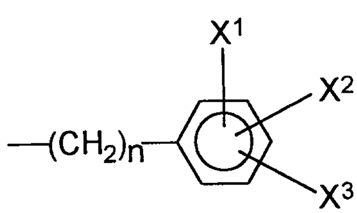

(in which n represents 0 or an integer of from 1 to 5, and $X^1$, $X^2$ and $X^3$ independently each represent hydrogen atom, halogen atom, $C_1$-$C_5$ alkyl group, $C_1$-$C_5$ alkoxy group, hydroxy group, nitro group or cyano group) with the proviso that $R^9$ and $R^{10}$ do not represent hydrogen atom at a time] or $-COR^{11}$ [wherein $R^{11}$ represents

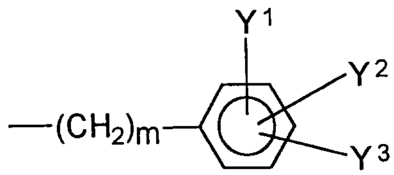

(in which m represents 0 or an integer of from 1 to 5, and $Y^1$, $Y^2$ and $Y^3$ independently each represent hydrogen atom, $C_1$-$C_5$ alkyl group, $C_1$-$C_5$ alkoxy group, hydroxy group or halogen atom)];

$R^4$, $R^5$, $R^6$, $R^7$ and $R^8$ independently each represents hydrogen atom, hydroxy group, halogen atom, $C_1$-$C_5$ alkoxy group, nitro group, cyano group;

$-NR^{12}R^{13}$ [wherein $R^{12}$ and $R^{13}$ independently each represent hydrogen atom, $C_1$-$C_5$ alkyl group or benzoyl group];

$-SOpR^{14}$ [wherein p represents 0, 1 or 2, and $R^{14}$ represents $C_1$-$C_5$ alkyl group or phenyl group];

$-COR^{15}$ [wherein $R^{15}$ represents hydrogen atom, $C_1$-$C_5$ alkyl group, $C_1$-$C_5$ alkoxy group, hydroxy group, phenyl group, phenoxy group or $-NHR^{16}$ (in which $R^{16}$ represents $C_1$-$C_5$ alkyl group or phenyl group)] or $C_1$-$C_5$ alkyl group optionally substituted by halogen atom, and

$R^{10'}$ represents

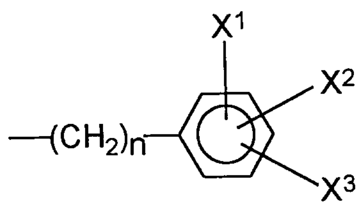

wherein n, $X^1$, $X^2$ and $X^3$ are defined as above

with the proviso that at least one of $R^4$ to $R^8$ is hydroxy group,

comprising the following steps:

protecting the hydroxy group in a compound of the general formula (V):

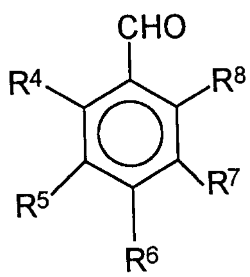

(V)

wherein $R^4$ to $R^8$ have the same meaning as above;

reacting with a compound of the following formula (VI):

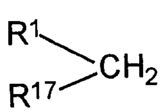

(VI)

wherein $R^1$ has the same meaning as above and $R^{17}$ represents hydrogen or carboxy group;

oxidizing the product obtained thereby to give a compound of the general formula (VII):

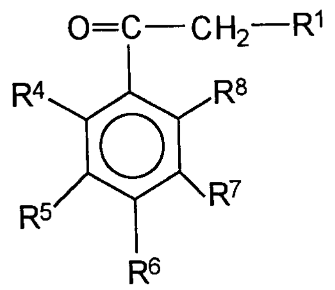

(VII)

wherein $R^4$ to $R^8$ are defined as above;

reacting compound (VII) with

$$R^{10'}—N=C=O$$

wherein $R^{10'}$ is defined as above; and

deprotecting the hydroxy groups, if necessary.

**11.** Process for the preparation of styrene derivatives of the following general formula (Id):

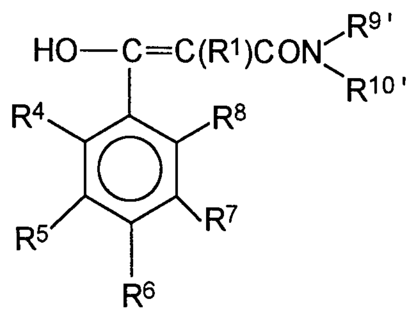

(Id)

wherein $R^1$ represents cyano group or $CONR^9R^{10}$ [wherein $R^9$ and $R^{10}$ independently each represent hydrogen atom, $C_1$-$C_5$ alkyl group or

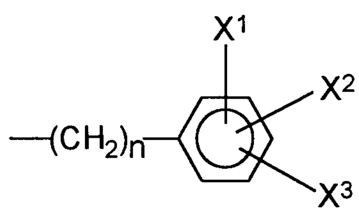

(in which n represents 0 or an integer of from 1 to 5, and $X^1$, $X^2$ and $X^3$ independently each represent hydrogen atom, halogen atom, $C_1$-$C_5$ alkyl group, $C_1$-$C_5$ alkoxy group, hydroxy group, nitro group or cyano group) with the proviso that $R^9$ and $R^{10}$ do not represent hydrogen atom at a time] or $-COR^{11}$ [wherein $R^{11}$ represents

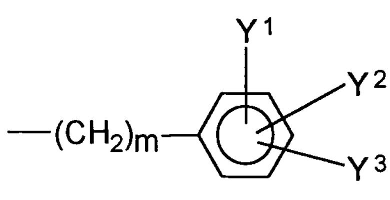

(in which m represents 0 or an integer of from 1 to 5, and $Y^1$, $Y^2$ and $Y^3$ independently each represent hydrogen atom, $C_1$-$C_5$ alkyl group, $C_1$-$C_5$ alkoxy group, hydroxy group or halogen atom)];

$R^4$, $R^5$, $R^6$, $R^7$ and $R^8$ independently each represents hydrogen atom, hydroxy group, halogen atom, $C_1$-$C_5$ alkoxy group, nitro group, cyano group;

$-NR^{12}R^{13}$ [wherein $R^{12}$ and $R^{13}$ independently each represent hydrogen atom, $C_1$-$C_5$ alkyl group or benzoyl group];

$-SOpR^{14}$ [wherein p represents 0, 1 or 2, and $R^{14}$ represents $C_1$-$C_5$ alkyl group or phenyl group];

$-COR^{15}$ [wherein $R^{15}$ represents hydrogen atom, $C_1$-$C_5$ alkyl group, $C_1$-$C_5$ alkoxy group, hydroxy group, phenyl group, phenoxy group or $-NHR^{16}$ (in which $R^{16}$ represents $C_1$-$C_5$ alkyl group or phenyl group)] or $C_1$-$C_5$ alkyl group optionally substituted by halogen atom, and

$R^{9'}$ represents $C_1$-$C_5$ alkyl group and $R^{10'}$ represents

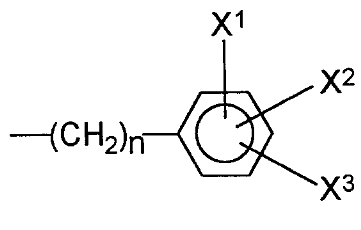

wherein n, $X^1$, $X^2$ and $X^3$ are defined as above,

with the proviso that at least one of $R^4$ to $R^8$ is hydroxy group,

comprising the following steps:

protecting the hydroxy group in a compound of the general formula (V):

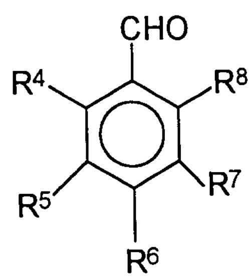

(V)

wherein $R^4$ to $R^8$ have the same meaning as above;

reacting with a compound of the following formula (VI):

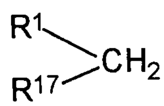

(VI)

wherein $R^1$ has the same meaning as above and $R^{17}$ represents hydrogen or carboxy group,

oxidizing the product obtained thereby to give a compound of the general formula (VII):

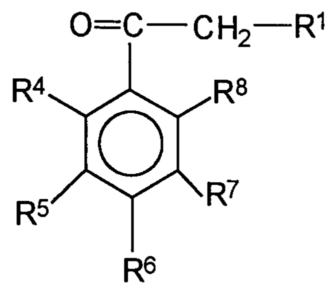

(VII)

wherein $R^4$ to $R^8$ are defined as above;
reacting compound (VII) with

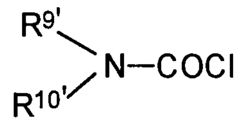

wherein $R^{9'}$ and $R^{10'}$ are defined as above; and
deprotecting the hydroxy groups, if necessary.

## Patentansprüche

### Patentansprüche für folgende Vertragsstaaten : DE, FR, GB, IT

1. Styrol-Derivate der folgenden allgemeinen Formel (I):

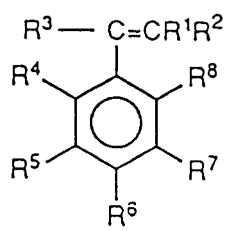

(I)

worin $R^1$ und $R^2$ jeweils unabhängig eine Cyano-Gruppe, $CONR^9R^{10}$ [worin $R^9$ und $R^{10}$ jeweils unabhängig Wasserstoffatom, $C_1$-$C_5$-Alkyl-Gruppe oder

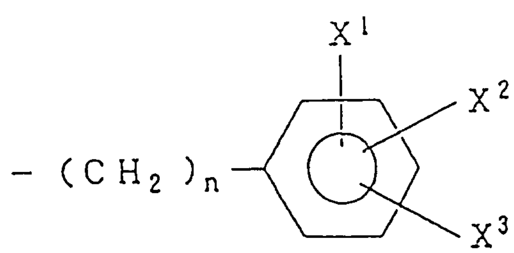

bedeuten, (worin n 0 oder eine ganze Zahl von 1 bis 5 und $X^1$, $X^2$ und $X^3$ jeweils unabhängig Wasserstoffatom, Halogenatom, $C_1$-$C_5$-Alkyl-Gruppe, $C_1$-$C_5$-Alkoxy-Gruppe, Hydroxy-Gruppe, Nitro-Gruppe oder Cyano-Gruppe sind), mit dem Vorbehalt, daß $R^9$ und $R^{10}$ nicht gleichzeitig Wasserstoffatom bedeuten] oder $-COR^{11}$ bedeuten

[worin $R^{11}$ ist:

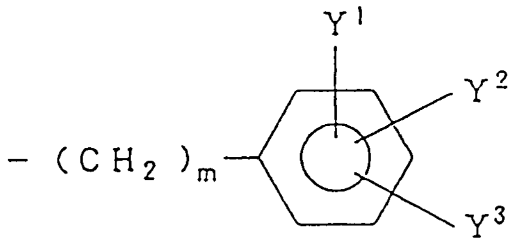

(worin m 0 oder eine ganze Zahl von 1 bis 5 und $Y^1$, $Y^2$ und $Y^3$ jeweils unabhängig Wasserstoffatom, $C_1$-$C_5$-Alkyl-Gruppe, $C_1$-$C_5$-Alkoxy-Gruppe, Hydroxy-Gruppe oder Halogenatom sind)], aber worin $R^1$ und $R^2$ nicht gleichzeitig eine Cyano-Gruppe sind;

$R^3$ Wasserstoffatom, $C_1$-$C_5$-Alkyl-Gruppe oder Hydroxy-Gruppe ist,

$R^4$, $R^5$, $R^6$, $R^7$ und $R^8$ jeweils unabhängig Wasserstoffatom, Hydroxy-Gruppe, Halogenatom, $C_1$-$C_5$-Alkoxy-Gruppe, Nitro-Gruppe, Cyano-Gruppe;

$-NR^{12}R^{13}$ [worin $R^{12}$ und $R^{13}$ jeweils unabhängig Wasserstoffatom, $C_1$-$C_5$-Alkyl-Gruppe oder Benzoyl-Gruppe sind];

$-SO_pR^{14}$ [worin p 0, 1 oder 2 und $R^{14}$ $C_1$-$C_5$-Alkyl-Gruppe oder Phenyl-Gruppe sind];

$-COR^{15}$ [worin $R^{15}$ Wasserstoffatom, $C_1$-$C_5$-Alkyl-Gruppe, $C_1$-$C_5$-Alkoxy-Gruppe, Hydroxy-Gruppe, Phenyl-Gruppe, Phenoxy-Gruppe oder $-NHR^{16}$ sind (worin $R^{16}$ $C_1$-$C_5$-Alkyl-Gruppe oder Phenyl-Gruppe ist)] oder $C_1$-$C_5$-Alkyl-Gruppe, wahlweise substituiert durch Halogenatom, bedeuten, mit dem ersten Vorbehalt, daß zumindest eines von $R^4$ bis $R^8$ eine Hydroxy-Gruppe ist und daß dann, wenn $R^3$ Wasserstoffatom und $R^4$ bis $R^8$ jeweils unabhängig ein Wasserstoffatom, Hydroxy-Gruppe, $C_1$-$C_5$-Alkoxy-Gruppe, $-NR^{12}R^{13}$ (worin $R^{12}$ und $R^{13}$ wie oben definiert sind), $C_1$-$C_5$-Alkyl-Gruppe sind, $R^1$ eine Cyano-Gruppe, $R^2$ $-CONR^9R^{10}$ [worin $R^9$ Wasserstoffatom oder $C_1$-$C_5$-Alkyl-Gruppe, $R^{10}$

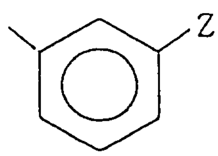

oder

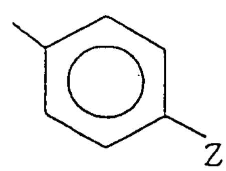

(worin Z eine $C_1$-$C_5$-Alkyl-Gruppe, Halogenatom, Nitro-Gruppe oder Cyano-Gruppe ist) sind] oder $-COR^{11}$ sind [worin $R^{11}$ bedeutet:

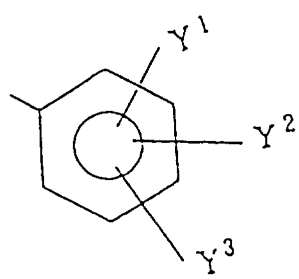

(worin $Y^1$, $Y^2$ und $Y^3$ wie oben definiert sind), vorausgesetzt, daß $R^{11}$ nicht:

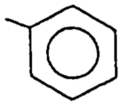 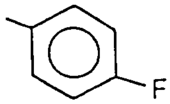 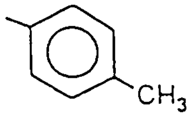 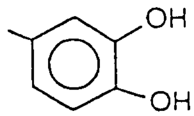

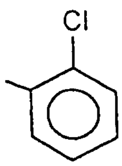 und 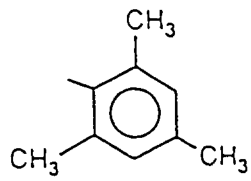

ist], mit dem zweiten Vorbehalt, daß dann, wenn $R^4$, $R^7$ und $R^8$ H, $R^6$ Hydroxy, $R^5$ $COR^{15}$, worin $R^{15}$ eine Hydroxy-Gruppe oder $C_1$-$C_5$-Alkoxy-Gruppe ist, $R^3$ Wasserstoff, $R^1$ Cyano und $R^2$ $-CONR^9R^{10}$ sind, worin $R^9$ Wasserstoff ist, $R^{10}$ nicht

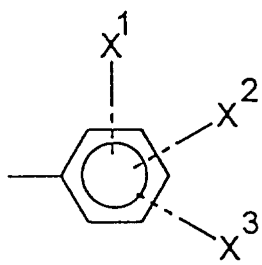

bedeutet, worin $X^1$, $X^2$ und $X^3$ jeweils unabhängig Chlor oder $C_1$-$C_5$-Alkyl-Gruppe bedeuten,
und mit dem dritten Vorbehalt, daß das Styrol der Formel I nicht N,N-Diethyl-2-cyano-3-(3,4-dihydroxy-5-nitrophenyl)-acrylamid ist, oder pharmazeutisch akzeptable Salze davon.

2. Verbindung nach Anspruch 1, worin $R^1$ Cyano-Gruppe und $R^2$ $-CONR^9R^{10}$ [worin $R^9$ Wasserstoffatom oder $C_1$-$C_5$-Alkyl-Gruppe und $R^{10}$

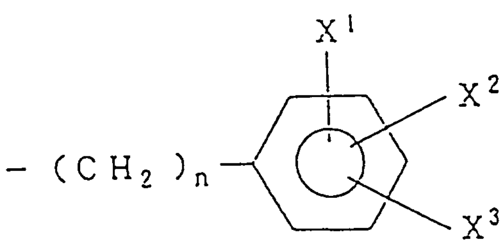

bedeuten (worin $X^1$ Wasserstoffatom, Halogenatom, $C_1$-$C_5$-Alkyl-Gruppe, Nitro-Gruppe oder Cyano-Gruppe und $X^2$ und $X^3$ Wasserstoffatom und n 0 bedeuten)]
oder $-COR^{11}$ sind [worin $R^{11}$ bedeutet:

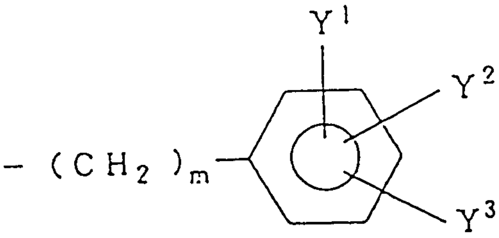

(worin $Y^1$ Wasserstoffatom oder Halogenatom, $Y^2$ und $Y^3$ Wasserstoffatom und m 0 bedeuten)],
$R^3$ Wasserstoffatom, $C_1$-$C_5$-Alkyl-Gruppe oder Hydroxy-Gruppe, $R^4$, $R^5$, $R^6$, $R^7$ und $R^8$ jeweils unabhängig Wasserstoffatom, Hydroxy-Gruppe, Halogenatom, $C_1$-$C_5$-Alkoxy-Gruppe, Nitro-Gruppe, Cyano-Gruppe, $-SO_2R^{14}$ (worin $R^{14}$ $C_1$-$C_5$-Alkyl-Gruppe ist), $-COR^{15}$ (worin $R^{15}$ Hydroxy-Gruppe ist) oder $C_1$-$C_5$-Alkyl-Gruppe, wahlweise substituiert durch Halogenatom, sind, und worin zumindest eines von $R^4$ bis $R^5$ Hydroxy-Gruppe ist, mit dem Vorbehalt, daß dann, wenn $R^3$ Wasserstoffatom und $R^4$ bis $R^8$ jeweils unabhängig Wasserstoffatom, Hydroxy-Gruppe, $C_1$-$C_5$-Alkoxy-Gruppe oder $C_1$-$C_5$-Alkyl-Gruppe sind, $R^2$ $-CONR^9R^{10}$ [worin $R^9$ Wasserstoffatom und $R^{10}$

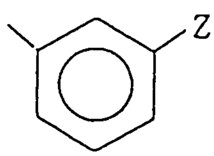

oder

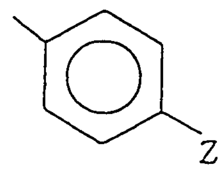

bedeuten (worin Z $C_1$-$C_5$-Alkyl-Gruppe, Halogenatom, Nitro-Gruppe oder Cyano-Gruppe ist)] oder $-COR^{11}$ ist [worin $R^{11}$ bedeutet

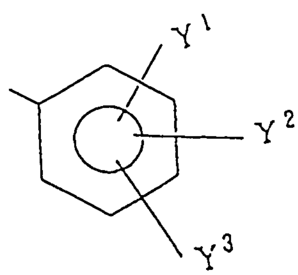

(worin $Y^1$ Halogenatom und $Y^2$ und $Y^3$ Wasserstoffatom bedeuten), vorausgesetzt, daß $R^{11}$ nicht

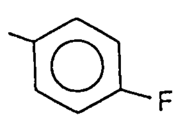

und

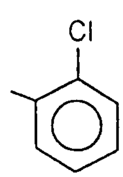

ist.

**3.** Verbindung nach Anspruch 2, worin $R^1$ Cyano-Gruppe,
$R^2$ $-CONR^9R^{10}$ [worin $R^9$ Wasserstoffatom und $R^{10}$

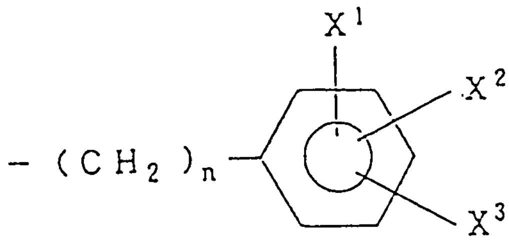

sind (worin $X^1$ Wasserstoffatom, Halogenatom, $C_1$-$C_5$-Alkyl-Gruppe, Nitro-Gruppe oder Cyano-Gruppe, $X^2$ und $X^3$ Wasserstoffatom und n 0 bedeuten)]
oder -$COR^{11}$ bedeuten [worin $R^{11}$ bedeutet:

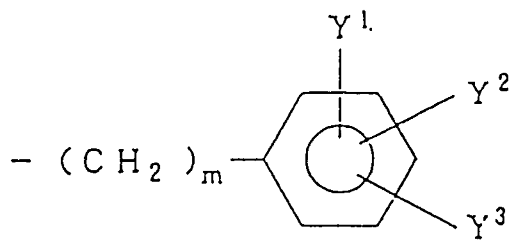

(worin $Y^1$, $Y^2$ und $Y^3$ Wasserstoffatom und m 0 bedeuten)],
worin $R^3$ Wasserstoffatom oder Hydroxy-Gruppe,
$R^4$ und $R^8$ jeweils unabhängig Wasserstoffatom, Halogenatom oder Nitro-Gruppe,
$R^5$ und $R^7$ Hydroxy-Gruppe, Halogenatom, $C_1$-$C_5$-Alkoxy-Gruppe, Nitro-Gruppe, Cyano-Gruppe, $SO_2R^{14}$ (worin $R^{14}$ $C_1$-$C_5$-Alkyl-Gruppe ist), -$COR^{15}$ (worin $R^{15}$ Hydroxy-Gruppe ist) oder $C_1$-$C_5$-Alkyl-Gruppe, wahlweise substituiert durch Halogenatom, $R^6$ Hydroxy-Gruppe bedeuten, mit dem Vorbehalt, daß dann, wenn $R^3$, $R^4$ und $R^8$ Wasserstoffatom und $R^5$ und $R^7$ jeweils unabhängig Wasserstoffatom, Hydroxy-Gruppe, $C_1$-$C_5$-Alkoxy-Gruppe oder $C_1$-$C_5$-Alkyl-Gruppe sind, $R^2$ -$CONR^9R^{10}$ ist [worin $R^9$ Wasserstoffatom und $R^{10}$

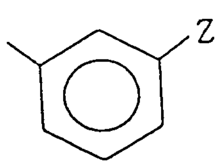

oder

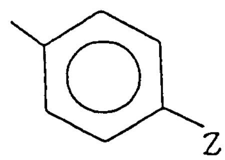

ist (worin Z $C_1$-$C_5$-Alkyl-Gruppe, Halogenatom, Nitro-Gruppe oder Cyano-Gruppe bedeutet)].

4. Verbindung nach Anspruch 3, worin $R^1$ Cyano-Gruppe,
$R^2$ -$CONR^9R^{10}$ [worin $R^9$ Wasserstoffatom und $R^{10}$

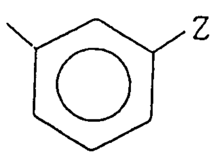

bedeuten (worin Z Wasserstoffatom oder Halogenatom ist)],
$R^3$ Wasserstoffatom oder Hydroxy-Gruppe,
$R^4$ Wasserstoffatom, $R^5$ Hydroxy-Gruppe, Halogenatom, $C_1$-$C_5$-Alkoxy-Gruppe oder $C_1$-$C_5$-Alkyl-Gruppe,
$R^6$ Hydroxy-Gruppe,

$R^7$ Wasserstoffatom, Halogenatom, Cyano-Gruppe oder $C_1$-$C_5$-Alkyl-Gruppe und
$R^8$ Wasserstoffatom, Halogenatom oder Nitro-Gruppe bedeuten, mit dem Vorbehalt, daß dann, wenn $R^3$ und $R^8$ Wasserstoffatom und $R^5$ Hydroxy-Gruppe, $C_1$-$C_5$-Alkoxy-Gruppe oder $C_1$-$C_5$-Alkyl-Gruppe und $R^7$ Wasserstoffatom oder $C_1$-$C_5$-Alkyl-Gruppe sind, Z Halogenatom ist.

**5.** Pharmazeutische Zusammensetzung, umfassend Styrol-Derivate der folgenden allgemeinen Formel (I):

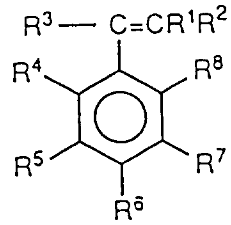

(I)

worin $R^1$ und $R^2$ jeweils unabhängig eine Cyano-Gruppe, $CONR^9R^{10}$ [worin $R^9$ und $R^{10}$ jeweils unabhängig Wasserstoffatom, $C_1$-$C_5$-Alkyl-Gruppe oder

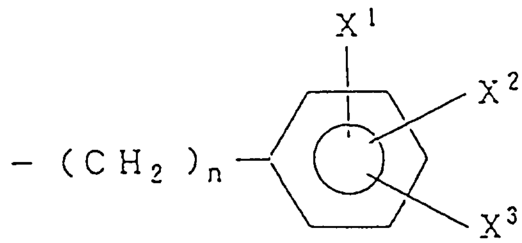

bedeuten, (worin n 0 oder eine ganze Zahl von 1 bis 5 und $X^1$, $X^2$ und $X^3$ jeweils unabhängig Wasserstoffatom, Halogenatom, $C_1$-$C_5$-Alkyl-Gruppe, $C_1$-$C_5$-Alkoxy-Gruppe, Hydroxy-Gruppe, Nitro-Gruppe oder Cyano-Gruppe sind), mit dem Vorbehalt, daß $R^9$ und $R^{10}$ nicht gleichzeitig Wasserstoffatom bedeuten] oder $-COR^{11}$ bedeuten [worin $R^{11}$ ist:

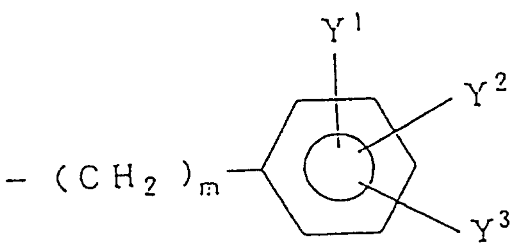

(worin m 0 oder eine ganze Zahl von 1 bis 5 und $Y^1$, $Y^2$ und $Y^3$ jeweils unabhängig Wasserstoffatom, $C_1$-$C_5$-Alkyl-Gruppe, $C_1$-$C_5$-Alkoxy-Gruppe, Hydroxy-Gruppe oder Halogenatom sind)], aber worin $R^1$ und $R^2$ nicht gleichzeitig eine Cyano-Gruppe sind;
$R^3$ Wasserstoffatom, $C_1$-$C_5$-Alkyl-Gruppe oder Hydroxy-Gruppe ist,
$R^4$, $R^5$, $R^6$, $R^7$ und $R^8$ jeweils unabhängig Wasserstoffatom, Hydroxy-Gruppe, Halogenatom, $C_1$-$C_5$-Alkoxy-Gruppe, Nitro-Gruppe, Cyano-Gruppe;
$-NR^{12}R^{13}$ [worin $R^{12}$ und $R^{13}$ jeweils unabhängig Wasserstoffatom, $C_1$-$C_5$-Alkyl-Gruppe oder Benzoyl-Gruppe sind];
$-SO_pR^{14}$ [worin P 0, 1 oder 2 und $R^{14}$ $C_1$-$C_5$-Alkyl-Gruppe oder Phenyl-Gruppe sind];
$-COR^{15}$ [worin $R^{15}$ Wasserstoffatom, $C_1$-$C_5$-Alkyl-Gruppe, $C_1$-$C_5$-Alkoxy-Gruppe, Hydroxy-Gruppe, Phenyl-Gruppe, Phenoxy-Gruppe oder $-NHR^{16}$ sind (worin $R^{16}$ $C_1$-$C_5$-Alkyl-Gruppe oder Phenyl-Gruppe ist)] oder $C_1$-$C_5$-

Alkyl-Gruppe, wahlweise substituiert durch Halogenatom, bedeuten,
mit dem ersten Vorbehalt, daß zumindest eines von $R^4$ bis $R^8$ eine Hydroxy-Gruppe ist und daß dann, wenn $R^3$ Wasserstoffatom und $R^4$ bis $R^8$ jeweils unabhängig ein Wasserstoffatom, Hydroxy-Gruppe, $C_1$-$C_5$-Alkoxy-Gruppe, -$NR^{12}R^{13}$ (worin $R^{12}$ und $R^{13}$ wie oben definiert sind), $C_1$-$C_5$-Alkyl-Gruppe sind, $R^1$ eine Cyano-Gruppe, $R^2$ -$CONR^9R^{10}$ [worin $R^9$ Wasserstoffatom oder $C_1$-$C_5$-Alkyl-Gruppe, $R^{10}$

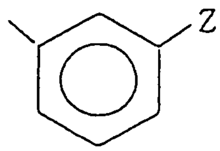

oder

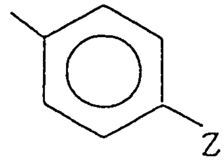

(worin Z eine $C_1$-$C_5$-Alkyl-Gruppe, Halogenatom, Nitro-Gruppe oder Cyano-Gruppe ist ) sind] oder -$COR^{11}$ bedeuten [worin $R^{11}$ bedeutet:

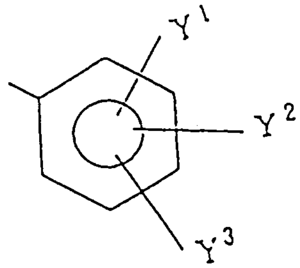

(worin $Y^1$, $Y^2$ und $Y^3$ wie oben definiert sind), vorausgesetzt, daß $R^{11}$ nicht:

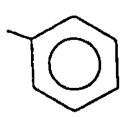

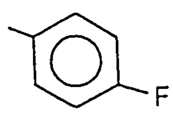

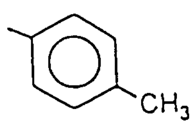

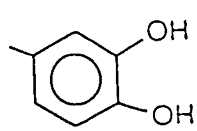

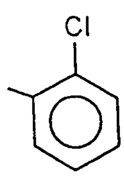

und

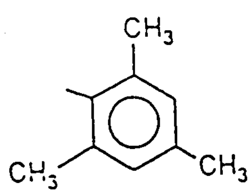

ist], mit dem zweiten Vorbehalt, daß das Styrol der Formel I nicht N,N-Diethyl-2-cyano-3-(3,4-dihydroxy-5-nitrophenyl)-acrylamid ist, oder pharmazeutisch akzeptable Salze dafür und pharmazeutisch akzeptable Träger dafür.

6. Verwendung von Styrol-Derivaten der folgenden allgemeinen Formel (I)

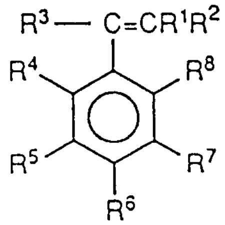

(I)

worin $R^1$ und $R^2$ jeweils unabhängig eine Cyano-Gruppe, $CONR^9R^{10}$ [worin $R^9$ und $R^{10}$ jeweils unabhängig Wasserstoffatom, $C_1$-$C_5$-Alkyl-Gruppe oder

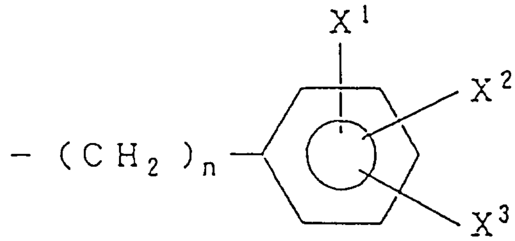

bedeuten, (worin n 0 oder eine ganze Zahl von 1 bis 5 und $X^1$, $X^2$ und $X^3$ jeweils unabhängig Wasserstoffatom, Halogenatom, $C_1$-$C_5$-Alkyl-Gruppe, $C_1$-$C_5$-Alkoxy-Gruppe, Hydroxy-Gruppe, Nitro-Gruppe oder Cyano-Gruppe sind), mit dem Vorbehalt, daß $R^9$ und $R^{10}$ nicht gleichzeitig Wasserstoffatom bedeuten] oder $-COR^{11}$ bedeuten [worin $R^{11}$ ist:

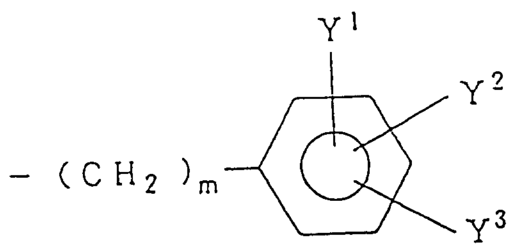

(worin m 0 oder eine ganze Zahl von 1 bis 5 und $Y^1$, $Y^2$ und $Y^3$ jeweils unabhängig Wasserstoffatom, $C_1$-$C_5$-Alkyl-Gruppe, $C_1$-$C_5$-Alkoxy-Gruppe, Hydroxy-Gruppe oder Halogenatom sind)], aber worin $R^1$ und $R^2$ nicht gleichzeitig eine Cyano-Gruppe sind;
$R^3$ Wasserstoffatom, $C_1$-$C_5$-Alkyl-Gruppe oder Hydroxy-Gruppe ist,
$R^4$, $R^5$, $R^6$, $R^7$ und $R^8$ jeweils unabhängig Wasserstoffatom, Hydroxy-Gruppe, Halogenatom, $C_1$-$C_5$-Alkoxy-Gruppe, Nitro-Gruppe, Cyano-Gruppe;
$-NR^{12}R^{13}$ [worin $R^{12}$ und $R^{13}$ jeweils unabhängig Wasserstoffatom, $C_1$-$C_5$-Alkyl-Gruppe oder Benzoyl-Gruppe sind];
$-SO_pR^{14}$ [worin p 0, 1 oder 2 und $R^{14}$ $C_1$-$C_5$-Alkyl-Gruppe oder Phenyl-Gruppe sind];
$-COR^{15}$ [worin $R^{15}$ Wasserstoffatom, $C_1$-$C_5$-Alkyl-Gruppe, $C_1$-$C_5$-Alkoxy-Gruppe, Hydroxy-Gruppe, Phenyl-Gruppe, Phenoxy-Gruppe oder $-NHR^{16}$ sind (worin $R^{16}$ $C_1$-$C_5$-Alkyl-Gruppe oder Phenyl-Gruppe ist)] oder $C_1$-$C_5$-Alkyl-Gruppe, wahlweise substituiert durch Halogenatom, bedeuten,
mit dem ersten Vorbehalt, daß zumindest eines von $R^4$ bis $R^8$ eine Hydroxy-Gruppe ist und daß dann, wenn $R^3$ Wasserstoffatom und $R^4$ bis $R^8$ jeweils unabhängig ein Wasserstoffatom, Hydroxy-Gruppe, $C_1$-$C_5$-Alkoxy-Gruppe, $-NR^{12}R^{13}$ (worin $R^{12}$ und $R^{13}$ wie oben definiert sind), $C_1$-$C_5$-Alkyl-Gruppe sind, $R^1$ eine Cyano-Gruppe, $R^2$ -

$CONR^9R^{10}$ [worin $R^9$ Wasserstoffatom oder $C_1$-$C_5$-Alkyl-Gruppe, $R^{10}$

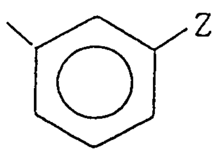

oder

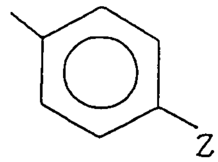

(worin Z eine $C_1$-$C_5$-Alkyl-Gruppe, Halogenatom, Nitro-Gruppe oder Cyano-Gruppe ist ) sind] oder $-COR^{11}$ [worin $R^{11}$ bedeutet:

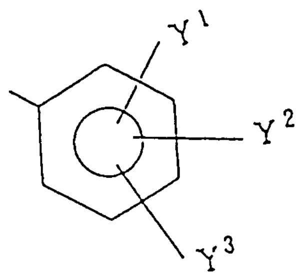

(worin $Y^1$, $Y^2$ und $Y^3$ wie oben definiert sind), vorausgesetzt, daß $R^{11}$ nicht:

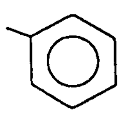

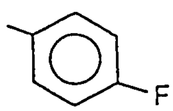

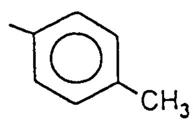

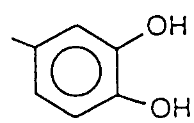

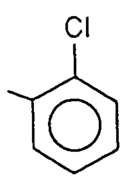

und

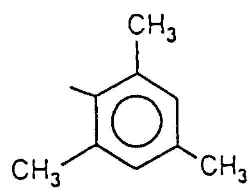

ist] bedeuten, oder von pharmazeutisch akzeptablen Salzen davon für die Herstellung eines Medikamentes, das bei der Behandlung von Krebs wirksam ist.

**Patentansprüche für folgenden Vertragsstaat : ES**

1. Styrol-Derivate der folgenden allgemeinen Formel (I):

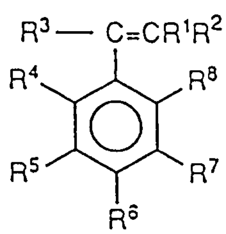

(I)

worin $R^1$ und $R^2$ jeweils unabhängig eine Cyano-Gruppe, $CONR^9R^{10}$ [worin $R^9$ und $R^{10}$ jeweils unabhängig Wasserstoffatom, $C_1$-$C_5$-Alkyl-Gruppe oder

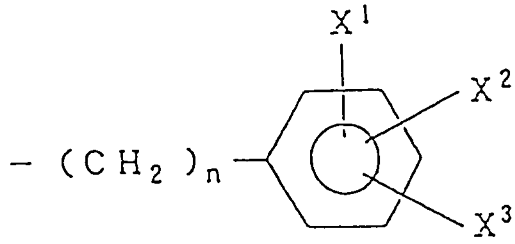

bedeuten (worin n 0 oder eine ganze Zahl von 1 bis 5 und $X^1$, $X^2$ und $X^3$ jeweils unabhängig Wasserstoffatom, Halogenatom, $C_1$-$C_5$-Alkyl-Gruppe, $C_1$-$C_5$-Alkoxy-Gruppe, Hydroxy-Gruppe, Nitro-Gruppe oder Cyano-Gruppe sind), mit dem Vorbehalt, daß $R^9$ und $R^{10}$ nicht gleichzeitig Wasserstoffatom bedeuten] oder $-COR^{11}$ bedeuten [worin $R^{11}$ ist:

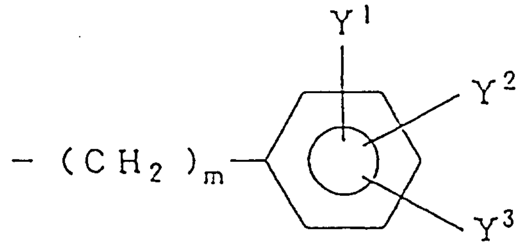

(worin m 0 oder eine ganze Zahl von 1 bis 5 und $Y^1$, $Y^2$ und $Y^3$ jeweils unabhängig Wasserstoffatom, $C_1$-$C_5$-Alkyl-Gruppe, $C_1$-$C_5$-Alkoxy-Gruppe, Hydroxy-Gruppe oder Halogenatom sind)], aber worin $R^1$ und $R^2$ nicht gleichzeitig eine Cyano-Gruppe sind;

$R^3$ Wasserstoffatom, $C_1$-$C_5$-Alkyl-Gruppe oder Hydroxy-Gruppe ist,

$R^4$, $R^5$, $R^6$, $R^7$ und $R^8$ jeweils unabhängig Wasserstoffatom, Hydroxy-Gruppe, Halogenatom, $C_1$-$C_5$-Alkoxy-Gruppe, Nitro-Gruppe, Cyano-Gruppe;

$-NR^{12}R^{13}$ [worin $R^{12}$ und $R^{13}$ jeweils unabhängig Wasserstoffatom, $C_1$-$C_5$-Alkyl-Gruppe oder Benzoyl-Gruppe sind];

$-SO_pR^{14}$ [worin p 0, 1 oder 2 und $R^{14}$ $C_1$-$C_5$-Alkyl-Gruppe oder Phenyl-Gruppe sind];

$-COR^{15}$ [worin $R^{15}$ Wasserstoffatom, $C_1$-$C_5$-Alkyl-Gruppe, $C_1$-$C_5$-Alkoxy-Gruppe, Hydroxy-Gruppe, Phenyl-Gruppe, Phenoxy-Gruppe oder $-NHR^{16}$ sind (worin $R^{16}$ $C_1$-$C_5$-Alkyl-Gruppe oder Phenyl-Gruppe ist)] oder $C_1$-$C_5$-Alkyl-Gruppe, wahlweise substituiert durch Halogenatom, bedeuten, mit dem ersten Vorbehalt, daß zumindest eines von $R^4$ bis $R^8$ eine Hydroxy-Gruppe ist und daß dann, wenn $R^3$ Wasserstoffatom und $R^4$ bis $R^8$ jeweils unabhängig ein Wasserstoffatom, Hydroxy-Gruppe, $C_1$-$C_5$-Alkoxy-Gruppe, $-NR^{12}R^{13}$ (worin $R^{12}$ und $R^{13}$ wie oben definiert sind), $C_1$-$C_5$-Alkyl-Gruppe sind, $R^1$ eine Cyano-Gruppe, $R^2$ $-CONR^9R^{10}$ [worin $R^9$ Wasserstoffatom oder $C_1$-$C_5$-Alkyl-Gruppe, $R^{10}$

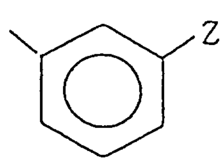

oder

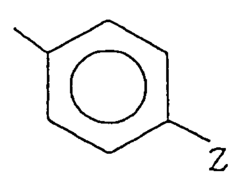

(worin Z eine $C_1$-$C_5$-Alkyl-Gruppe, Halogenatom, Nitro-Gruppe oder Cyano-Gruppe ist ) sind] oder $-COR^{11}$ bedeuten [worin $R^{11}$ bedeutet:

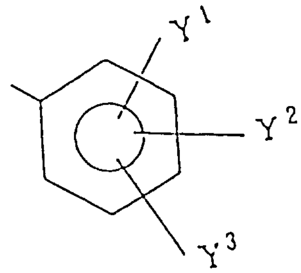

(worin $Y^1$, $Y^2$ und $Y^3$ wie oben definiert sind), vorausgesetzt, daß $R^{11}$ nicht:

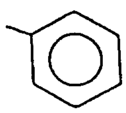 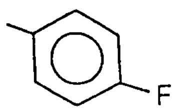 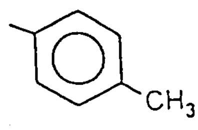 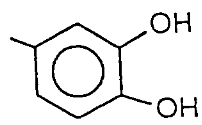

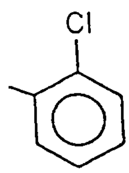 und 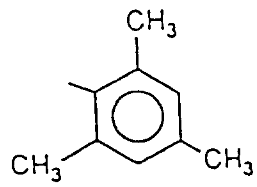

ist], mit dem zweiten Vorbehalt, daß dann, wenn $R^4$, $R^7$ und $R^8$ H sind, $R^6$ Hydroxy, $R^5$ $COR^{15}$, worin $R^{15}$ eine Hydroxy-Gruppe oder $C_1$-$C_5$-Alkoxy-Gruppe ist, $R^3$ Wasserstoff, $R^1$ Cyano und $R^2$ $-CONR^9R^{10}$ sind, worin $R^9$ Wasserstoff ist, $R^{10}$ nicht

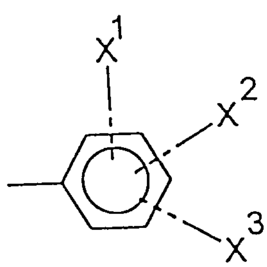

bedeutet, worin $X^1$, $X^2$ und $X^3$ jeweils unabhängig Chlor oder $C_1$-$C_5$-Alkyl-Gruppe bedeuten,
und mit dem dritten Vorbehalt, daß das Styrol der Formel I nicht N,N-Diethyl-2-cyano-3-(3,4-dihydroxy-5-nitrophenyl)-acrylamid ist, oder pharmazeutisch akzeptable Salze davon.

2. Verbindung nach Anspruch 1, worin $R^1$ Cyano-Gruppe und $R^2$ $-CONR^9R^{10}$ [worin $R^9$ Wasserstoffatom oder $C_1$-$C_5$-Alkyl-Gruppe und $R^{10}$

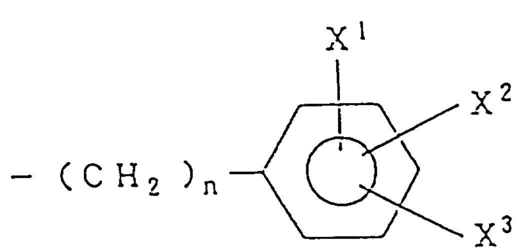

bedeuten (worin $X^1$ Wasserstoffatom, Halogenatom, $C_1$-$C_5$-Alkyl-Gruppe, Nitro-Gruppe oder Cyano-Gruppe und $X^2$ und $X^3$ Wasserstoffatom und n 0 bedeuten)]
oder -$COR^{11}$ sind [worin $R^{11}$ bedeutet:

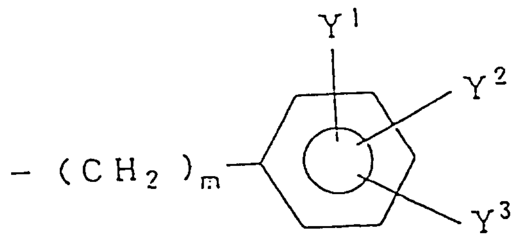

(worin $Y^1$ Wasserstoffatom oder Halogenatom, $Y^2$ und $Y^3$ Wasserstoffatom und m 0 bedeuten)],
$R^3$ Wasserstoffatom, $C_1$-$C_5$-Alkyl-Gruppe oder Hydroxy-Gruppe, $R^4$, $R^5$, $R^6$, $R^7$ und $R^8$ jeweils unabhängig Wasserstoffatom, Hydroxy-Gruppe, Halogenatom, $C_1$-$C_5$-Alkoxy-Gruppe, Nitro-Gruppe, Cyano-Gruppe, -$SO_2R^{14}$ (worin $R^{14}$ $C_1$-$C_5$-Alkyl-Gruppe ist), -$COR^{15}$ (worin $R^{15}$ Hydroxy-Gruppe ist) oder $C_1$-$C_5$-Alkyl-Gruppe, wahlweise substituiert durch Halogenatom, sind, und worin zumindest eines von $R^4$ bis $R^5$ Hydroxy-Gruppe ist, mit dem Vorbehalt, daß dann, wenn $R^3$ Wasserstoffatom und $R^4$ bis $R^8$ jeweils unabhängig Wasserstoffatom, Hydroxy-Gruppe, $C_1$-$C_5$-Alkoxy-Gruppe oder $C_1$-$C_5$-Alkyl-Gruppe sind, $R^2$ -$CONR^9R^{10}$ [worin $R^9$ Wasserstoffatom und $R^{10}$

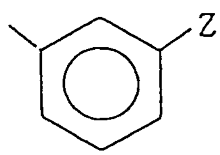

oder

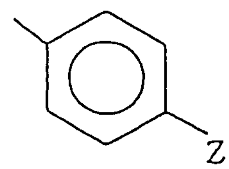

bedeuten (worin Z $C_1$-$C_5$-Alkyl-Gruppe, Halogenatom, Nitro-Gruppe oder Cyano-Gruppe ist)]
oder -$COR^{11}$ ist [worin $R^{11}$ bedeutet

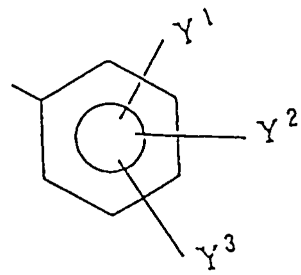

(worin $Y^1$ Halogenatom und $Y^2$ und $Y^3$ Wasserstoffatom bedeuten), vorausgesetzt, daß $R^{11}$ nicht

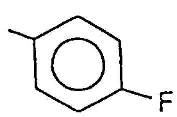

und

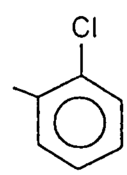

ist.

**3.** Verbindung nach Anspruch 2, worin $R^1$ Cyano-Gruppe, $R^2$ -$CONR^9R^{10}$ [worin $R^9$ Wasserstoffatom und $R^{10}$

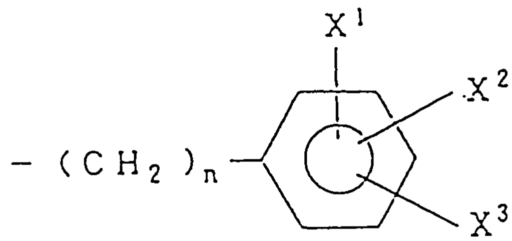

sind (worin $X^1$ Wasserstoffatom, Halogenatom, $C_1$-$C_5$-Alkyl-Gruppe, Nitro-Gruppe oder Cyano-Gruppe, $X^2$ und $X^3$ Wasserstoffatom und n 0 bedeuten)]
oder -$COR^{11}$ bedeutet [worin $R^{11}$ bedeutet:

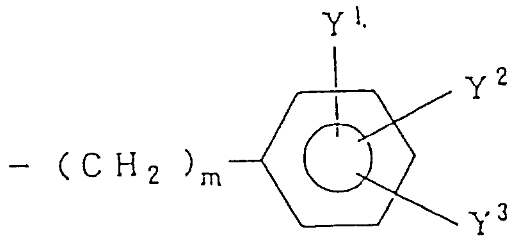

(worin $Y^1$, $Y^2$ und $Y^3$ Wasserstoffatom und m 0 bedeuten)],
worin $R^3$ Wasserstoffatom oder Hydroxy-Gruppe,
$R^4$ und $R^8$ jeweils unabhängig Wasserstoffatom, Halogenatom oder Nitro-Gruppe,
$R^5$ und $R^7$ Hydroxy-Gruppe, Halogenatom, $C_1$-$C_5$-Alkoxy-Gruppe, Nitro-Gruppe, Cyano-Gruppe, $SO_2R^{14}$ (worin $R^{14}$ $C_1$-$C_5$-Alkyl-Gruppe ist), -$COR^{15}$ (worin $R^{15}$ Hydroxy-Gruppe ist) oder $C_1$-$C_5$-Alkyl-Gruppe, wahlweise substituiert durch Halogenatom, $R^6$ Hydroxy-Gruppe bedeuten, mit dem Vorbehalt, daß dann, wenn $R^3$, $R^4$ und $R^8$ Wasserstoffatom und $R^5$ und $R^7$ jeweils unabhängig Wasserstoffatom, Hydroxy-Gruppe, $C_1$-$C_5$-Alkoxy-Gruppe oder $C_1$-$C_5$-Alkyl-Gruppe sind, $R^2$ -$CONR^9R^{10}$ ist [worin $R^9$ Wasserstoffatom und $R^{10}$

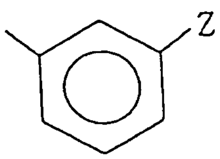 oder 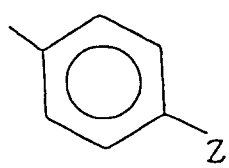

ist (worin Z $C_1$-$C_5$-Alkyl-Gruppe, Halogenatom, Nitro-Gruppe oder Cyano-Gruppe bedeutet)].

**4.** Verbindung nach Anspruch 3, worin $R^1$ Cyano-Gruppe, $R^2$ -$CONR^9R^{10}$ [worin $R^9$ Wasserstoffatom und $R^{10}$

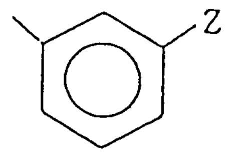

bedeutet, (worin Z Wasserstoffatom oder Halogenatom ist)], $R^3$ Wasserstoffatom oder Hydroxy-Gruppe, $R^4$ Wasserstoffatom, $R^5$ Hydroxy-Gruppe, Halogenatom, $C_1$-$C_5$-Alkoxy-Gruppe oder $C_1$-$C_5$-Alkyl-Gruppe, $R^6$ Hydroxy-Gruppe, $R^7$ Wasserstoffatom, Halogenatom, Cyano-Gruppe oder $C_1$-$C_5$-Alkyl-Gruppe und $R^8$ Wasserstoffatom, Halogenatom oder Nitro-Gruppe bedeuten, mit dem Vorbehalt, daß dann, wenn $R^3$ und $R^8$ Wasserstoffatom und $R^5$ Hydroxy-Gruppe, $C_1$-$C_5$-Alkoxy-Gruppe oder $C_1$-$C_5$-Alkyl-Gruppe und $R^7$ Wasserstoffatom oder $C_1$-$C_5$-Alkyl-Gruppe sind, Z Halogenatom ist.

**5.** Pharmazeutische Zusammensetzung, umfassend Styrol-Derivate der folgenden allgemeinen Formel (I):

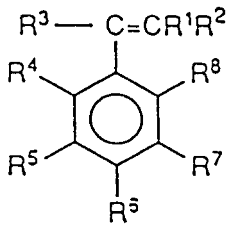

(I)

worin $R^1$ und $R^2$ jeweils unabhängig eine Cyano-Gruppe, $CONR^9R^{10}$ [worin $R^9$ und $R^{10}$ jeweils unabhängig Wasserstoffatom, $C_1$-$C_5$-Alkyl-Gruppe oder

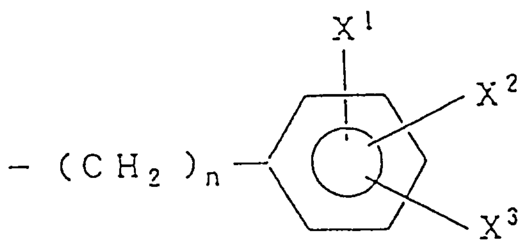

bedeuten, (worin n 0 oder eine ganze Zahl von 1 bis 5 und $X^1$, $X^2$ und $X^3$ jeweils unabhängig Wasserstoffatom, Halogenatom, $C_1$-$C_5$-Alkyl-Gruppe, $C_1$-$C_5$-Alkoxy-Gruppe, Hydroxy-Gruppe, Nitro-Gruppe oder Cyano-Gruppe sind), mit dem Vorbehalt, daß $R^9$ und $R^{10}$ nicht gleichzeitig Wasserstoffatom bedeuten] oder $-COR^{11}$ bedeuten [worin $R^{11}$ ist:

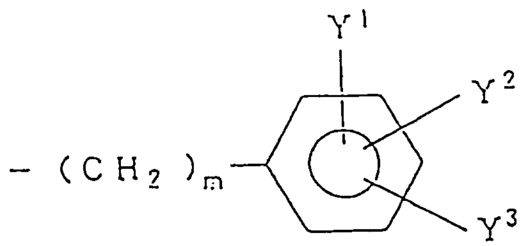

(worin m 0 oder eine ganze Zahl von 1 bis 5 und $Y^1$, $Y^2$ und $Y^3$ jeweils unabhängig Wasserstoffatom, $C_1$-$C_5$-Alkyl-Gruppe, $C_1$-$C_5$-Alkoxy-Gruppe, Hydroxy-Gruppe oder Halogenatom sind)], aber worin $R^1$ und $R^2$ nicht gleichzeitig eine Cyano-Gruppe sind;
$R^3$ Wasserstoffatom, $C_1$-$C_5$-Alkyl-Gruppe oder Hydroxy-Gruppe ist,
$R^4$, $R^5$, $R^6$, $R^7$ und $R^8$ jeweils unabhängig Wasserstoffatom, Hydroxy-Gruppe, Halogenatom, $C_1$-$C_5$-Alkoxy-Gruppe, Nitro-Gruppe, Cyano-Gruppe;
-$NR^{12}R^{13}$ [worin $R^{12}$ und $R^{13}$ jeweils unabhängig Wasserstoffatom, $C_1$-$C_5$-Alkyl-Gruppe oder Benzoyl-Gruppe sind];
-$SO_pR^{14}$ [worin p 0, 1 oder 2 und $R^{14}$ $C_1$-$C_5$-Alkyl-Gruppe oder Phenyl-Gruppe sind];
-$COR^{15}$ [worin $R^{15}$ Wasserstoffatom, $C_1$-$C_5$-Alkyl-Gruppe, $C_1$-$C_5$-Alkoxy-Gruppe, Hydroxy-Gruppe, Phenyl-Gruppe, Phenoxy-Gruppe oder -$NHR^{16}$ sind (worin $R^{16}$ $C_1$-$C_5$-Alkyl-Gruppe oder Phenyl-Gruppe ist)] oder $C_1$-$C_5$-Alkyl-Gruppe, wahlweise substituiert durch Halogenatom, bedeuten,
mit dem ersten Vorbehalt, daß zumindest eines von $R^4$ bis $R^8$ eine Hydroxy-Gruppe ist und daß dann, wenn $R^3$ Wasserstoffatom und $R^4$ bis $R^8$ jeweils unabhängig ein Wasserstoffatom, Hydroxy-Gruppe, $C_1$-$C_5$-Alkoxy-Gruppe, -$NR^{12}R^{13}$ (worin $R^{12}$ und $R^{13}$ wie oben definiert sind), $C_1$-$C_5$-Alkyl-Gruppe sind, $R^1$ eine Cyano-Gruppe, $R^2$ -$CONR^9R^{10}$ [worin $R^9$ Wasserstoffatom oder $C_1$-$C_5$-Alkyl-Gruppe, $R^{10}$

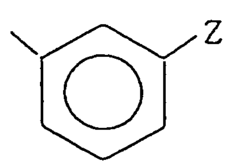

oder

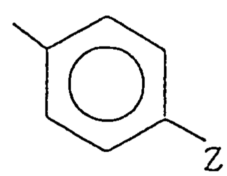

(worin Z eine $C_1$-$C_5$-Alkyl-Gruppe, Halogenatom, Nitro-Gruppe oder Cyano-Gruppe ist ) sind] oder -$COR^{11}$ ist [worin $R^{11}$ bedeutet:

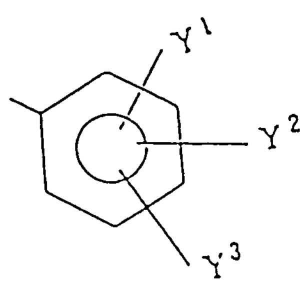

(worin $Y^1$ $Y^2$ und $Y^3$ wie oben definiert sind), vorausgesetzt, daß $R^{11}$ nicht:

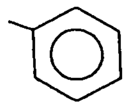
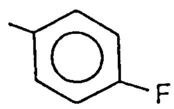
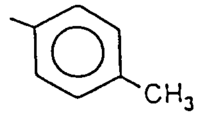
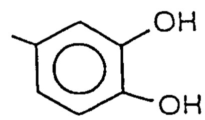

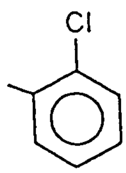

und

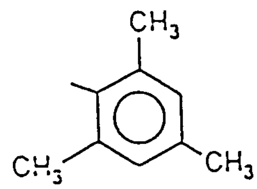

ist] bedeutet, und mit dem zweiten Vorbehalt, daß das Styrol der Formel I nicht N,N-Diethyl-2-cyano-3-(3,4-dihydroxy-5-nitrophenyl)-acrylamid ist, oder pharmazeutisch akzeptable Salze dafür.

**6.** Verwendung von Styrol-Derivaten der folgenden allgemeinen Formel (I)

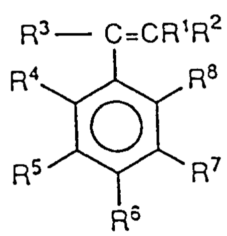

(I)

worin $R^1$ und $R^2$ jeweils unabhängig eine Cyano-Gruppe, $CONR^9R^{10}$ [worin $R^9$ und $R^{10}$ jeweils unabhängig Wasserstoffatom, $C_1$-$C_5$-Alkyl-Gruppe oder

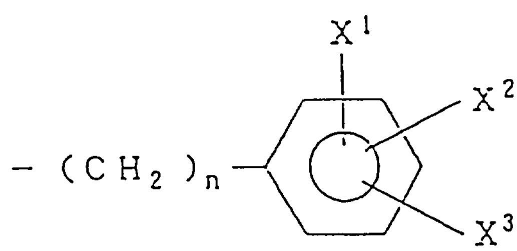

bedeuten, (worin n 0 oder eine ganze Zahl von 1 bis 5 und $X^1$, $X^2$ und $X^3$ jeweils unabhängig Wasserstoffatom, Halogenatom, $C_1$-$C_5$-Alkyl-Gruppe, $C_1$-$C_5$-Alkoxy-Gruppe, Hydroxy-Gruppe, Nitro-Gruppe oder Cyano-Gruppe sind), mit dem Vorbehalt, daß $R^9$ und $R^{10}$ nicht gleichzeitig Wasserstoffatom bedeuten] oder $-COR^{11}$ bedeuten [worin $R^{11}$ ist:

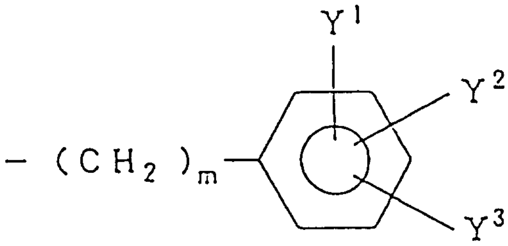

(worin m 0 oder eine ganze Zahl von 1 bis 5 und $Y^1$, $Y^2$ und $Y^3$ jeweils unabhängig Wasserstoffatom, $C_1$-$C_5$-Alkyl-Gruppe, $C_1$-$C_5$-Alkoxy-Gruppe, Hydroxy-Gruppe oder Halogenatom sind)], aber worin $R^1$ und $R^2$ nicht gleichzeitig eine Cyano-Gruppe sind;
$R^3$ Wasserstoffatom, $C_1$-$C_5$-Alkyl-Gruppe oder Hydroxy-Gruppe ist,
$R^4$, $R^5$, $R^6$, $R^7$ und $R^8$ jeweils unabhängig Wasserstoffatom, Hydroxy-Gruppe, Halogenatom, $C_1$-$C_5$-Alkoxy-Gruppe, Nitro-Gruppe, Cyano-Gruppe;
$-NR^{12}R^{13}$ [worin $R^{12}$ und $R^{13}$ jeweils unabhängig Wasserstoffatom, $C_1$-$C_5$-Alkyl-Gruppe oder Benzoyl-Gruppe sind];
$-SO_pR^{14}$ [worin p 0, 1 oder 2 und $R^{14}$ $C_1$-$C_5$-Alkyl-Gruppe oder Phenyl-Gruppe sind];
$-COR^{15}$ [worin $R^{15}$ Wasserstoffatom, $C_1$-$C_5$-Alkyl-Gruppe, $C_1$-$C_5$-Alkoxy-Gruppe, Hydroxy-Gruppe, Phenyl-Gruppe, Phenoxy-Gruppe oder $-NHR^{16}$ sind (worin $R^{16}$ $C_1$-$C_5$-Alkyl-Gruppe oder Phenyl-Gruppe ist)] oder $C_1$-$C_5$-Alkyl-Gruppe, wahlweise substituiert durch Halogenatom, bedeuten,
mit dem ersten Vorbehalt, daß zumindest eines von $R^4$ bis $R^8$ eine Hydroxy-Gruppe ist und daß dann, wenn $R^3$ Wasserstoffatom und $R^4$ bis $R^8$ jeweils unabhängig ein Wasserstoffatom, Hydroxy-Gruppe, $C_1$-$C_5$-Alkoxy-Gruppe, $-NR^{12}R^{13}$ (worin $R^{12}$ und $R^{13}$ wie oben definiert sind), $C_1$-$C_5$-Alkyl-Gruppe sind, $R^1$ eine Cyano-Gruppe, $R^2$ $-CONR^9R^{10}$ [worin $R^9$ Wasserstoffatom oder $C_1$-$C_5$-Alkyl-Gruppe, $R^{10}$

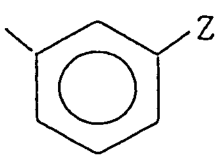

oder

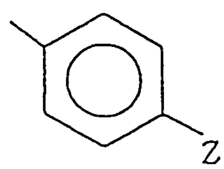

(worin Z eine $C_1$-$C_5$-Alkyl-Gruppe, Halogenatom, Nitro-Gruppe oder Cyano-Gruppe ist ) sind] oder $-COR^{11}$ sind [worin $R^{11}$ bedeutet:

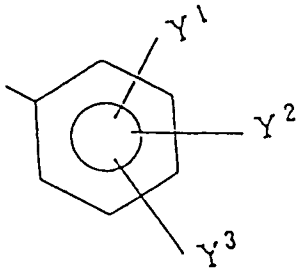

(worin $Y^1$, $Y^2$ und $Y^3$ wie oben definiert sind), vorausgesetzt, daß $R^{11}$ nicht:

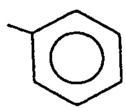 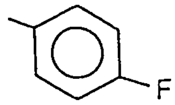 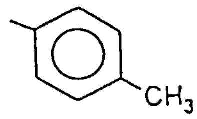 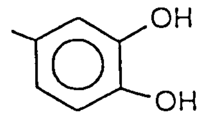

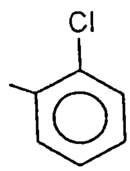 und 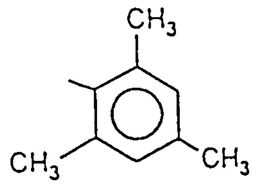

ist], oder von pharmazeutisch akzeptablen Salzen davon für die Herstellung eines Medikamentes, das bei der Behandlung von Krebs wirksam ist.

**7.** Verfahren zur Herstellung von Styrol-Derivaten der folgenden allgemeinen Formel (I)

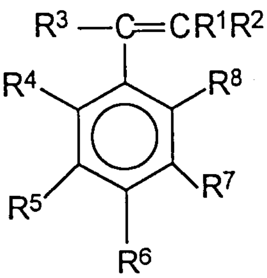

(I)

worin $R^1$ und $R^2$ jeweils unabhängig eine Cyano-Gruppe, $CONR^9R^{10}$ [worin $R^9$ und $R^{10}$ jeweils unabhängig Wasserstoffatom, $C_1$-$C_5$-Alkyl-Gruppe oder

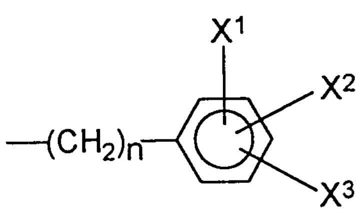

bedeuten (worin n 0 oder eine ganze Zahl von 1 bis 5 und $X^1$, $X^2$ und $X^3$ jeweils unabhängig Wasserstoffatom, Halogenatom, $C_1$-$C_5$-Alkyl-Gruppe, $C_1$-$C_5$-Alkoxy-Gruppe, Hydroxy-Gruppe, Nitro-Gruppe oder Cyano-Gruppe sind), mit dem Vorbehalt, daß $R^9$ und $R^{10}$ nicht gleichzeitig Wasserstoffatom bedeuten] oder $-COR^{11}$ bedeuten [worin $R^{11}$ ist:

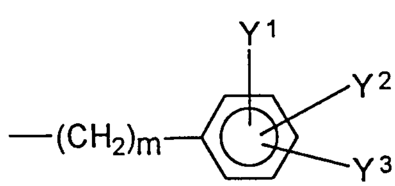

(worin m 0 oder eine ganze Zahl von 1 bis 5 und $Y^1$, $Y^2$ und $Y^3$ jeweils unabhängig Wasserstoffatom, $C_1$-$C_5$-Alkyl-

Gruppe, $C_1$-$C_5$-Alkoxy-Gruppe, Hydroxy-Gruppe oder Halogenatom sind)], aber worin $R^1$ und $R^2$ nicht gleichzeitig eine Cyano-Gruppe sind;

$R^3$ Wasserstoffatom oder $C_1$-$C_5$-Alkyl-Gruppe ist,

$R^4$, $R^5$, $R^6$, $R^7$ und $R^8$ jeweils unabhängig Wasserstoffatom, Hydroxy-Gruppe, Halogenatom, $C_1$-$C_5$-Alkoxy-Gruppe, Nitro-Gruppe, Cyano-Gruppe;

-$NR^{12}R^{13}$ [worin $R^{12}$ und $R^{13}$ jeweils unabhängig Wasserstoffatom, $C_1$-$C_5$-Alkyl-Gruppe oder Benzoyl-Gruppe sind];

-$SO_pR^{14}$ [worin p 0, 1 oder 2 und $R^{14}$ $C_1$-$C_5$-Alkyl-Gruppe oder Phenyl-Gruppe sind];

-$COR^{15}$ [worin $R^{15}$ Wasserstoffatom, $C_1$-$C_5$-Alkyl-Gruppe, $C_1$-$C_5$-Alkoxy-Gruppe, Hydroxy-Gruppe, Phenyl-Gruppe, Phenoxy-Gruppe oder -$NHR^{16}$ (worin $R^{16}$ $C_1$-$C_5$-Alkyl-Gruppe oder Phenyl-Gruppe ist)] oder $C_1$-$C_5$-Alkyl-Gruppe, wahlweise substituiert durch Halogenatom, bedeuten,

mit dem ersten Vorbehalt, daß zumindest eines von $R^4$ bis $R^8$ eine Hydroxy-Gruppe ist und daß dann, wenn $R^3$ Wasserstoffatom und $R^4$ bis $R^8$ jeweils unabhängig ein Wasserstoffatom, Hydroxy-Gruppe, $C_1$-$C_5$-Alkoxy-Gruppe, -$NR^{12}R^{13}$ (worin $R^{12}$ und $R^{13}$ wie oben definiert sind), $C_1$-$C_5$-Alkyl-Gruppe sind, $R^1$ eine Cyano-Gruppe, $R^2$ -$CONR^9R^{10}$ [worin $R^9$ Wasserstoffatom oder $C_1$-$C_5$-Alkyl-Gruppe, $R^{10}$

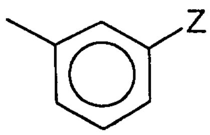

oder

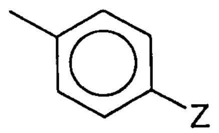

(worin Z eine $C_1$-$C_5$-Alkyl-Gruppe, Halogenatom, Nitro-Gruppe oder Cyano-Gruppe ist ) sind] oder -$COR^{11}$ ist [worin $R^{11}$ bedeutet:

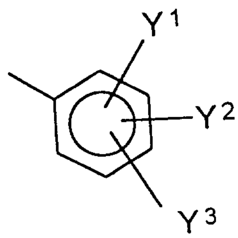

(worin $Y^1$, $Y^2$ und $Y^3$ wie oben definiert sind), vorausgesetzt, daß $R^{11}$ nicht:

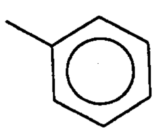
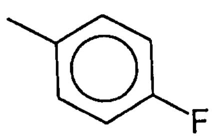
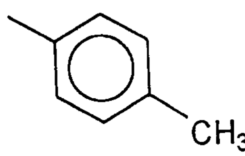
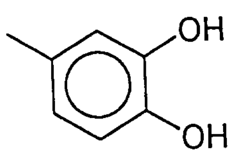

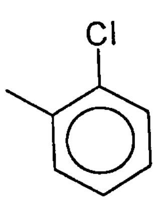

und

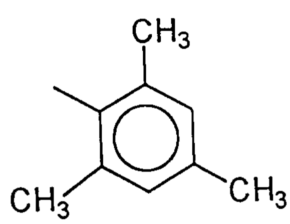

ist], mit dem zweiten Vorbehalt, daß dann, wenn $R^4$, $R^7$ und $R^8$ H sind, $R^6$ Hydroxy, $R^5$ $COR^{15}$, worin $R^{15}$ eine Hydroxy-Gruppe oder $C_1$-$C_5$-Alkoxy-Gruppe ist, $R^3$ Wasserstoff, $R^1$ Cyano und $R^2$ -$CONR^9R^{10}$ sind, worin $R^9$ Wasserstoff ist, $R^{10}$ nicht

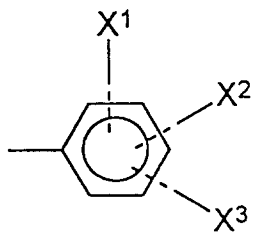

bedeutet, worin $X^1$, $X^2$ und $X^3$ jeweils unabhängig Chlor oder $C_1$-$C_5$-Alkyl-Gruppe bedeuten, und mit dem dritten Vorbehalt, daß das Styrol der Formel I nicht N,N-Diethyl-2-cyano-3-(3,4-dihydroxy-5-nitrophenyl)-acrylamid ist, durch Kondensation der Verbindung der Formel (II):

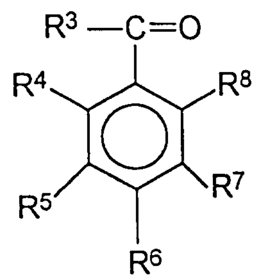

(II)

worin $R^3$ bis $R^8$ die gleiche Bedeutung wie oben haben, mit der Verbindung der Formel (III):

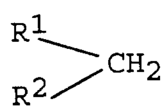

(III)

worin $R^1$ und $R^2$ die gleiche Bedeutung wie oben haben, oder durch Schützen der Hydroxygruppe(n) in der obigen Verbindung der Formel (II), Kondensation des dadurch erhaltenen Produktes mit der obigen Verbindung der Formel (III) und Abspaltung der Schutzgruppe(n) der Hydroxygruppe(n) des durch diesen Kondensationsschritt erhaltenen Produktes.

**8.** Verfahren zur Herstellung von Styrol-Derivaten der folgenden allgemeinen Formel (Ia):

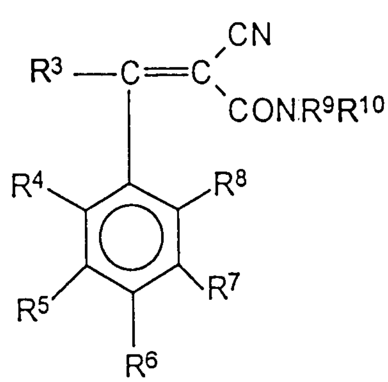

(Ia)

worin $R^9$ und $R^{10}$ jeweils unabhängig Wasserstoffatom, $C_1$-$C_5$-Alkyl-Gruppe oder

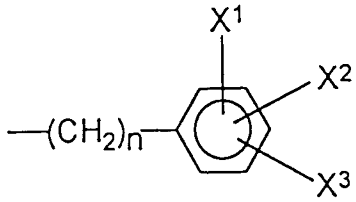

bedeuten (worin n 0 oder eine ganze Zahl von 1 bis 5 und $X^1$, $X^2$ und $X^3$ jeweils unabhängig Wasserstoffatom, Halogenatom, $C_1$-$C_5$-Alkyl-Gruppe, $C_1$-$C_5$-Alkoxy-Gruppe, Hydroxy-Gruppe, Nitro-Gruppe oder Cyano-Gruppe bedeuten) mit dem Vorbehalt, daß $R^9$ und $R^{10}$ nicht gleichzeitig Wasserstoffatome bedeuten
worin $R^3$ Wasserstoffatom oder $C_1$-$C_5$-Alkyl-Gruppe;
$R^4$, $R^5$, $R^6$, $R^7$ und $R^8$ jeweils unabhängig Wasserstoffatom, Hydroxy-Gruppe, Halogenatom, $C_1$-$C_5$-Alkoxy-Gruppe, Nitro-Gruppe, Cyano-Gruppe;
$-NR^{12}R^{13}$ [worin $R^{12}$ und $R^{13}$ jeweils unabhängig Wasserstoffatom, $C_1$-$C_5$-Alkyl-Gruppe oder Benzoyl-Gruppe sind];
$-SO_pR^{14}$ [worin p 0, 1 oder 2 und $R^{14}$ $C_1$-$C_5$-Alkyl-Gruppe oder Phenyl-Gruppe sind];
$-COR^{15}$ [worin $R^{15}$ Wasserstoffatom, $C_1$-$C_5$-Alkyl-Gruppe, $C_1$-$C_5$-Alkoxy-Gruppe, Hydroxy-Gruppe, Phenyl-Gruppe, Phenoxy-Gruppe oder $-NHR^{16}$ (worin $R^{16}$ $C_1$-$C_5$-Alkyl-Gruppe oder Phenyl-Gruppe ist) bedeutet] oder $C_1$-$C_5$-Alkyl-Gruppe, wahlweise substituiert durch Halogenatom, sind,
mit dem ersten Vorbehalt, daß zumindest eines von $R^4$ bis $R^8$ Hydroxy-Gruppe ist und daß dann, wenn $R^3$ Wasserstoffatom und $R^4$ bis $R^8$ jeweils unabhängig Wasserstoffatom, Hydroxy-Gruppe, $C_1$-$C_5$-Alkoxy-Gruppe, $-NR^{12}R^{13}$ (worin $R^{12}$ und $R^{13}$ wie oben definiert sind), $C_1$-$C_5$-Alkyl-Gruppe sind, $R^9$ Wasserstoffatom oder $C_1$-$C_5$-Alkyl-Gruppe und $R^{10}$

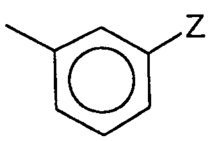

oder

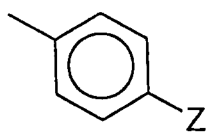

bedeuten (worin Z $C_1$-$C_5$-Alkyl-Gruppe, Halogenatom, Nitro-Gruppe oder Cyano-Gruppe ist)
mit dem zweiten Vorbehalt, daß dann, wenn $R^4$, $R^7$ und $R^8$ H, $R^6$ Hydroxy, $R^5$ $COR^{15}$, worin $R^{15}$ eine Hydroxy-Gruppe oder $C_1$-$C_5$-Alkoxy-Gruppe, $R^3$ Wasserstoff und $R^9$ Wasserstoff ist, bedeuten, $R^{10}$ nicht

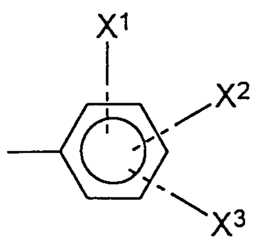

ist, worin $X^1$, $X^2$ und $X^3$ unabhängig Chlor oder $C_1$-$C_5$-Alkyl-Gruppe bedeuten,
und mit dem dritten Vorbehalt, daß das Styrol der Formel I nicht N,N-Diethyl-2-cyano-3-(3,4-dihydroxy-5-nitrophenyl)acrylamid ist,
umfassend die folgenden Schritte:
Kondensation der Verbindung der allgemeinen Formel (II):

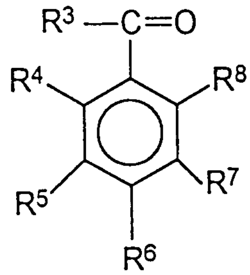

(II)

worin $R^3$ bis $R^8$ die gleiche Bedeutung wie oben haben, mit Cyanoessigsäure, unter Erhalt der Verbindung der folgenden allgemeinen Formel (IV)

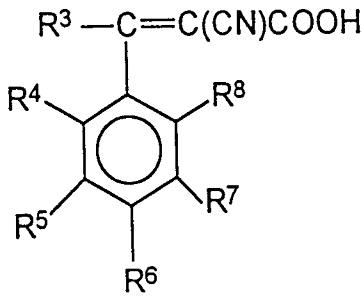

(IV)

worin $R^3$ bis $R^8$ die gleiche Bedeutung wie oben haben, und Kondensation der Verbindung (IV) mit dem Amin der folgenden allgemeinen Formel (V):

$$R^9R^{10}NH \quad (V)$$

worin $R^9$ bis $R^{10}$ die gleiche Bedeutung wie oben haben.

**9.** Verfahren zur Herstellung von Styrol-Derivaten der folgenden allgemeinen Formel (Ib):

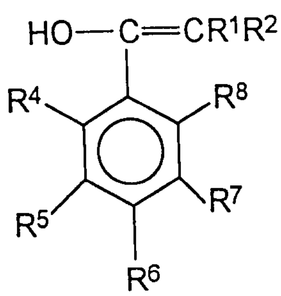

(Ib)

worin $R^1$ unf $R^2$ jeweils unabhängig Cyano-Gruppe, $CONR^9R^{10}$ [worin $R^9$ und $R^{10}$ jeweils unabhängig Wasserstoffatom, $C_1$-$C_5$-Alkyl-Gruppe oder

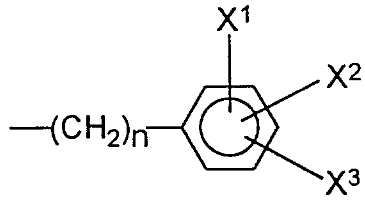

(worin n 0 oder eine ganze Zahl von 1 bis 5 und $X^1$, $X^2$ und $X^3$ jeweils unabhängig Wasserstoffatom, Halogenatom, $C_1$-$C_5$-Alkyl-Gruppe, $C_1$-$C_5$-Alkoxy, Hydroxy-Gruppe, Nitro-Gruppe oder Cyano-Gruppe sind) sind mit dem Vorbehalt, daß $R^9$ und $R^{10}$ nicht gleichzeitig Wasserstoffatom bedeuten] oder $-COR^{11}$ bedeuten [worin $R^{11}$

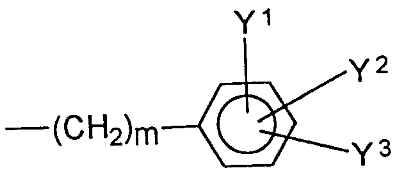

ist (worin m 0 oder eine ganze Zahl von 1 bis 5 und $Y^1$, $Y^2$ und $Y^3$ jeweils unabhängig Wasserstoffatom, $C_1$-$C_5$-Alkyl-Gruppe, $C_1$-$C_5$-Alkoxy-Gruppe, Hydroxy-Gruppe oder Halogenatom sind)], aber worin $R^1$ und $R^2$ nicht gleichzeitig Cyano-Gruppe bedeuten;
worin $R^4$, $R^5$, $R^6$, $R^7$ und $R^8$ jeweils unabhängig Wasserstoffatom, Hydroxy-Gruppe, Halogenatom, $C_1$-$C_5$-Alkoxy-Gruppe, Nitro-Gruppe, Cyano-Gruppe;
$-NR^{12}R^{13}$ [worin $R^{12}$ und $R^{13}$ jeweils unabhängig Wasserstoffatom, $C_1$-$C_5$-Alkyl-Gruppe oder Benzoyl-Gruppe bedeuten];
$-SO_pR^{14}$ [worin p 0, 1 oder 2 und $R^{14}$ $C_1$-$C_5$-Alkyl-Gruppe oder Phenyl-Gruppe sind];
$-COR^{15}$ [worin $R^{15}$ Wasserstoffatom, $C_1$-$C_5$-Alkyl-Gruppe, $C_1$-$C_5$-Alkoxy-Gruppe, Hydroxy-Gruppe, Phenyl-Gruppe, Phenoxy-Gruppe oder $-NHR^{16}$ (worin $R^{16}$ $C_1$-$C_5$-Alkyl-Gruppe oder Phenyl-Gruppe ist) bedeuten] oder $C_1$-$C_5$-Alkyl-Gruppe, wahlweise substituiert durch Halogenatom, sind, mit dem Vorbehalt, daß zumindest eines von $R^4$ bis $R^8$ Hydroxy-Gruppe ist,
umfassend die folgenden Schritte:
Schützen der Hydroxy-Gruppe in Verbindungen der allgemeinen Formel (IIa)

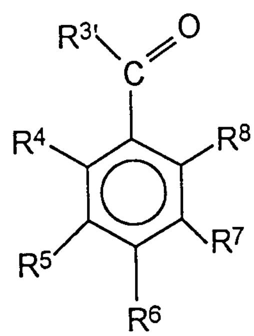

(IIa)

worin $R^4$ bis $R^8$ wie oben definiert sind und $R^{3'}$ Hydroxy ist; Reaktion mit Thionylchlorid oder Oxalylchlorid, unter Erhalt der Verbindung der Formel (IIa), worin $R^{3'}$ Chlor und zumindest eines von $R^4$ bis $R^8$ Acyloxy oder Trialkylsiloxy und die verbleibenden Substituenten wie oben definiert sind;
Reaktion des dadurch erhaltenen Produktes mit der Verbindung der allgemeinen Formel (III):

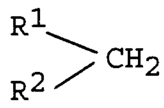

(III)

worin $R^1$ und $R^2$ wie oben definiert sind; und Abspaltung der Schutzgruppen von den Hydroxy-Gruppen, falls erforderlich.

**10.** Verfahren zur Herstellung von Styrol-Derivaten der folgenden allgemeinen Formel (Ic):

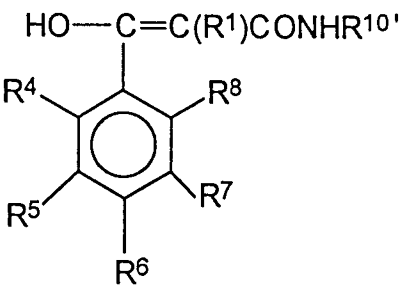

(Ic)

worin $R^1$ Cyano-Gruppe oder $CONR^9R^{10}$ [worin $R^9$ und $R^{10}$ jeweils unabhängig Wasserstoffatom, $C_1$-$C_5$-Alkyl-Gruppe oder

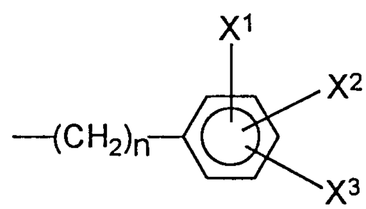

bedeuten (worin n 0 oder eine ganze Zahl von 1 bis 5 und $X^1$, $X^2$ und $X^3$ jeweils unabhängig Wasserstoffatom, Halogenatom, $C_1$-$C_5$-Alkyl-Gruppe, $C_1$-$C_5$-Alkoxy-Gruppe, Hydroxy-Gruppe, oder Cyano-Gruppe sind), mit dem Vorbehalt, daß $R^9$ und $R^{10}$ nicht gleichzeitig Wasserstoffatom bedeuten] oder $-COR^{11}$ bedeutet [worin $R^{11}$

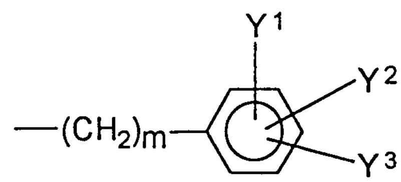

ist (worin m 0 oder eine ganze Zahl von 1 bis 5 und $Y^1$, $Y^2$ und $Y^3$ jeweils unabhängig Wasserstoffatom, $C_1$-$C_5$-Alkyl-Gruppe, $C_1$-$C_5$-Alkoxy-Gruppe, Hydroxy-Gruppe oder Halogenatom sind)];
$R^4$, $R^5$, $R^6$, $R^7$ und $R^8$ jeweils unabhängig Wasserstoffatom, Hydroxy-Gruppe, Halogenatom, $C_1$-$C_5$-Alkoxy-Gruppe, Nitro-Gruppe, Cyano-Gruppe;
$-NR^{12}R^{13}$ [worin $R^{12}$ und $R^{13}$ jeweils unabhängig Wasserstoffatom, $C_1$-$C_5$-Alkyl-Gruppe oder Benzoyl-Gruppe sind];
$-SO_pR^{14}$ [worin p 0, 1 oder 2 und $R^{14}$ $C_1$-$C_5$-Alkyl-Gruppe oder Phenyl-Gruppe sind];
$-COR^{15}$ [worin $R^{15}$ Wasserstoffatom, $C_1$-$C_5$-Alkyl-Gruppe, $C_1$-$C_5$-Alkoxy-Gruppe, Hydroxy-Gruppe, Phenyl-Gruppe, Phenoxy-Gruppe oder $-NHR^{16}$ (worin $R^{16}$ $C_1$-$C_5$-Alkyl-Gruppe oder Phenyl-Gruppe ist)] oder $C_1$-$C_5$-Alkyl-Gruppe, wahlweise substituiert durch Halogenatom, bedeuten und worin $R^{10'}$ bedeutet:

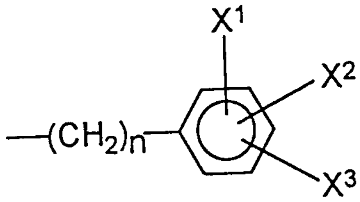

worin n, $X^1$, $X^2$ und $X^3$ wie oben definiert sind,
mit dem Vorbehalt, daß zumindest eines von $R^4$ bis $R^8$ Hydroxy-Gruppe ist,
umfassend die folgenden Schritte:
Schützen der Hydroxy-Gruppe in einer Verbindung der allgemeinen Formel (V):

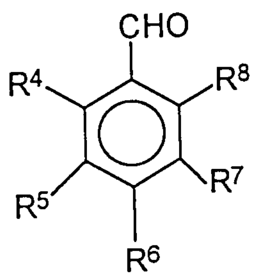

(V)

worin $R^4$ bis $R^8$ die gleiche Bedeutung wie oben haben;
Reaktion mit einer Verbindung der folgenden Formel (VI):

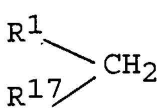

(VI)

worin $R^1$ die gleiche Bedeutung wie oben hat und $R^{17}$ Wasserstoffatom oder Carboxy-Gruppe bedeutet;
Oxidation des dadurch erhaltenen Produktes, unter Erhalt einer Verbindung der allgemeinen Formel (VII):

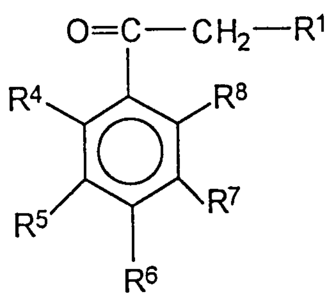

(VII)

worin $R^4$ bis $R^8$ wie oben definiert sind; Reaktion der Verbindung (VII) mit

$R^{10'}$-N=C=O

worin $R^{10'}$ wie oben definiert ist; und
Abspalten der Schutzgruppe an den Hydroxy-Gruppen, falls erforderlich.

**11.** Verfahren zur Herstellung von Styrol-Derivaten der folgenden allgemeinen Formel (Id):

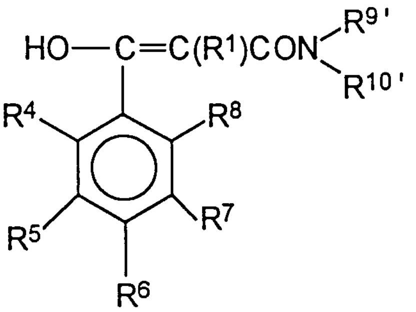

(Id)

worin $R^1$ Cyano-Gruppe oder $CONR^9R^{10}$ [worin $R^9$ und $R^{10}$ jeweils unabhängig Wasserstoffatom, $C_1$-$C_5$-Alkyl-Gruppe oder

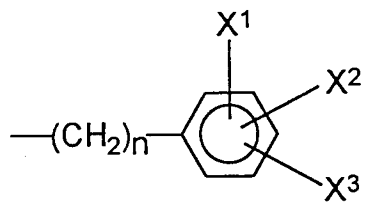

bedeuten (worin n 0 oder eine ganze Zahl von 1 bis 5 und $X^1$, $X^2$ und $X^3$ jeweils unabhängig Wasserstoffatom, Halogenatom, $C_1$-$C_5$-Alkyl-Gruppe, $C_1$-$C_5$-Alkoxy-Gruppe, Hydroxy-Gruppe, Nitro-Gruppe oder Cyano-Gruppe sind), mit dem Vorbehalt, daß $R^9$ und $R^{10}$ nicht gleichzeitig Wasserstoffatom bedeuten] oder $-COR^{11}$ ist [worin $R^{11}$ bedeutet

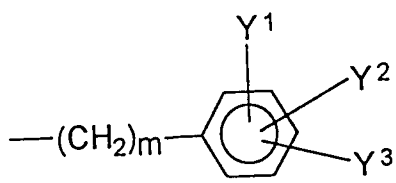

(worin m 0 oder eine ganze Zahl von 1 bis 5 und $Y^1$, $Y^2$ und $Y^3$ jeweils unabhängig Wasserstoffatom, $C_1$-$C_5$-Alkyl-Gruppe, $C_1$-$C_3$-Alkoxy-Gruppe, Hydroxy-Gruppe oder Halogenatom sind)],
$R^4$, $R^5$, $R^6$, $R^7$ und $R^8$ jeweils unabhängig Wasserstoffatom, Hydroxy-Gruppe, Halogenatom, $C_1$-$C_5$-Alkoxy-Gruppe, Nitro-Gruppe, Cyano-Gruppe;
$-NR^{12}R^{13}$ [worin $R^{12}$ und $R^{13}$ jeweils unabhängig Wasserstoffatom, $C_1$-$C_5$-Alkyl-Gruppe oder Benzoyl-Gruppe sind];
$-SO_pR^{14}$ [worin p 0, 1 oder 2 und $R^{14}$ $C_1$-$C_5$-Alkyl-Gruppe oder Phenyl-Gruppe sind];
$-COR^{15}$ [worin $R^{15}$ Wasserstoffatom, $C_1$-$C_5$-Alkyl-Gruppe, $C_1$-$C_5$-Alkoxy-Gruppe, Hydroxy-Gruppe, Phenyl-Gruppe, Phenoxy-Gruppe oder $-NHR^{16}$ ist (worin $R^{16}$ $C_1$-$C_5$-Alkyl-Gruppe oder Phenyl-Gruppe bedeutet)] oder $C_1$-$C_5$-Alkyl-Gruppe, wahlweise durch Halogenatom substituiert, bedeuten, und worin $R^{9'}$ $C_1$-$C_5$-Alkyl-Gruppe und $R^{10'}$

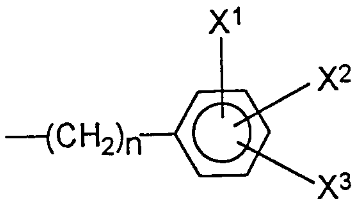

bedeuten, worin n, $X^1$, $X^2$ und $X^3$ wie oben definiert sind;
mit dem Vorbehalt, daß zumindest eines von $R^4$ bis $R^8$ Hydroxy-Gruppe ist,
umfassend die folgenden Schritte:
Schützen der Hydroxy-Gruppe in einer Verbindung der allgemeinen Formel (V):

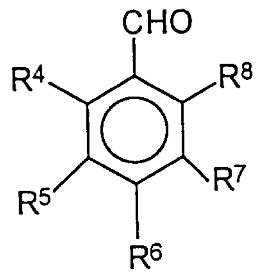

(V)

worin $R^4$ bis $R^8$ die gleiche Bedeutung wie oben haben;
Reaktion mit einer Verbindung der folgenden Formel (VI):

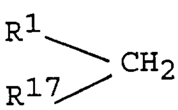

(VI)

worin $R^1$ die gleiche Bedeutung wie oben hat und $R^{17}$ Wasserstoff oder Carboxy-Gruppe bedeutet;
Oxidation des dadurch erhaltenen Produktes, unter Erhalt einer Verbindung mit der allgemeinen Formel (VII):

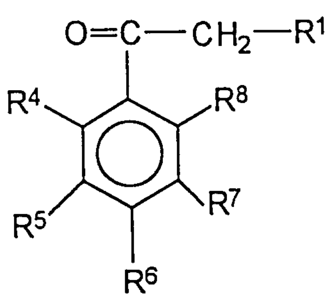

(VII)

worin $R^4$ bis $R^8$ wie oben definiert sind;
Reaktion der Verbindung (VII) mit:

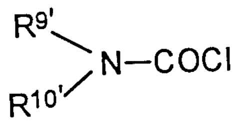

worin $R^{9'}$ und $R^{10'}$ wie oben definiert sind, und Abspaltung der Schutzgruppe an den Hydroxy-Gruppen, falls erforderlich.

**Revendications**

**Revendications pour les Etats contractants suivants : DE, FR, GB, IT**

1. Dérivés de styrène ayant la formule générale suivante (I) :

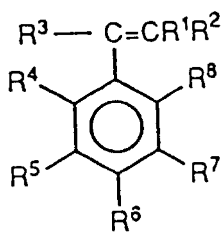

(I)

dans laquelle $R^1$ et $R^2$ représentent chacun indépendamment un groupe cyano, un groupe $CONR^9R^{10}$ [où $R^9$ et $R^{10}$ représentent chacun indépendamment un atome d'hydrogène, un groupe alkyle en $C_1$-$C_5$ ou un groupe de formule :

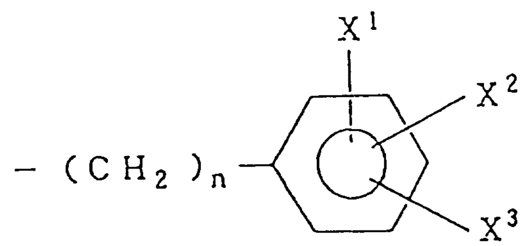

(dans laquelle n vaut 0 ou un nombre entier de 1 à 5, et $X^1$, $X^2$ et $X^3$ représentent chacun indépendamment un atome d'hydrogène, un atome d'halogène, un groupe alkyle en $C_1$-$C_5$, un groupe alcoxy en $C_1$-$C_5$, un groupe hydroxy, un groupe nitro ou un groupe cyano), à la condition que $R^9$ et $R^{10}$ ne représentent pas en même temps un atome d'hydrogène] ou un groupe $-COR^{11}$ [où $R^{11}$ représente un groupe de formule :

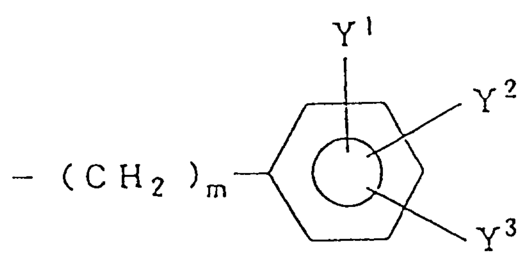

(dans laquelle m vaut 0 ou un nombre entier de 1 à 5, et $Y^1$, $Y^2$ et $Y^3$ représentent chacun indépendamment un atome d'hydrogène, un groupe alkyle en $C_1$-$C_5$, un groupe alcoxy en $C_1$-$C_5$, un groupe hydroxy ou un atome d'halogène)], mais $R^1$ et $R^2$ ne représentant pas en même temps un groupe cyano;

$R^3$ représente un atome d'hydrogène, un groupe alkyle en $C_1$-$C_5$ ou un groupe hydroxy ;
$R^4$, $R^5$, $R^6$, $R^7$ et $R^8$ représentent chacun indépendamment un atome d'hydrogène, un groupe hydroxy, un atome d'halogène, un groupe alcoxy en $C_1$-$C_5$, un groupe nitro, un groupe cyano ;
un groupe -$NR^{12}R^{13}$ [où $R^{12}$ et $R^{13}$ représentent chacun indépendamment un atome d'hydrogène, un groupe alkyle en $C_1$-$C_5$ ou un groupe benzoyle] ;
un groupe -$SO_pR^{14}$ [où p vaut 0, 1 ou 2, et $R^{14}$ représente un groupe alkyle en $C_1$-$C_5$ ou un groupe phényle] ;
un groupe -$COR^{15}$ [où $R^{15}$ représente un atome d'hydrogène, un groupe alkyle en $C_1$-$C_5$, un groupe alcoxy en $C_1$-$C_5$, un groupe hydroxy, un groupe phényle, un groupe phénoxy ou un groupe -$NHR^{16}$ (où $R^{16}$ représente un groupe alkyle en $C_1$-$C_5$ ou un groupe phényle)],
ou encore un groupe alkyle en $C_1$-$C_5$ éventuellement substitué par un atome d'halogène,
avec comme première condition qu'au moins l'un de $R^4$ à $R^8$ représente un groupe hydroxy, et que, lorsque $R^3$ est un atome d'hydrogène et $R^4$ à $R^8$ représentent chacun indépendamment un atome d'hydrogène, un groupe hydroxy, un groupe alcoxy en $C_1$-$C_5$, un groupe -$NR^{12}R^{13}$ (où $R^{12}$ et $R^{13}$ sont tels que définis ci-dessus) ou un groupe alkyle en $C_1$-$C_5$, alors $R^1$ représente un groupe cyano, $R^2$ représente un groupe -$CONR^9R^{10}$ [où $R^9$ représente un atome d'hydrogène ou un groupe alkyle en $C_1$-$C_5$ et $R^{10}$ représente un groupe de formule :

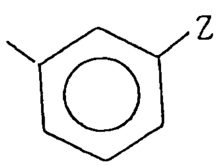 ou 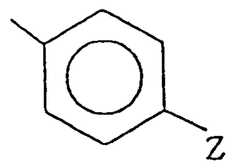

(dans laquelle Z représente un groupe alkyle en $C_1$-$C_5$, un atome d'halogène, un groupe nitro ou un groupe cyano)] ou un groupe -$COR^{11}$ [où $R^{11}$ représente un groupe de formule :

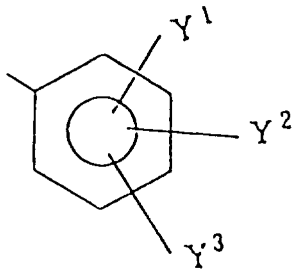

(dans laquelle $Y^1$, $Y^2$ et $Y^3$ sont tels que définis ci-dessus), à condition que $R^{11}$ ne soit pas

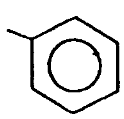 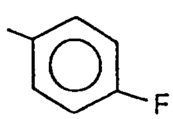 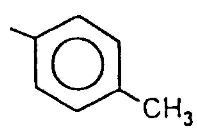 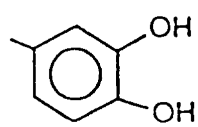

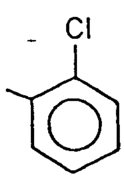 ni 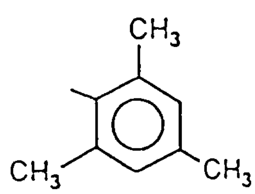 ]]

avec comme deuxième condition que lorsque $R^4$, $R^7$ et $R^8$ sont H, $R^6$ est un groupe hydroxy, $R^5$ est un groupe -$COR^{15}$ où $R^{15}$ représente un groupe hydroxy ou un groupe alcoxy en $C_1$-$C_5$, $R^3$ est l'hydrogène, $R^1$ est un groupe cyano et $R^2$ est un groupe -$CONR^9R^{10}$ où $R^9$ représente l'hydrogène, alors $R^{10}$ ne représente pas le groupe de formule :

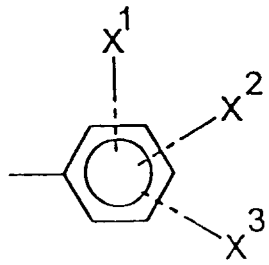

dans laquelle $X^1$, $X^2$ et $X^3$ représentent chacun indépendamment le chlore ou un groupe alkyle en $C_1$-$C_5$, et avec comme troisième condition que le styrène de la formule I ne soit pas le N,N-diéthyl-2-cyano-3-(3,4-dihydroxy-5-nitrophényl) acrylamide,
ou leurs sels pharmaceutiquement acceptables.

2. Composé selon la revendication 1, dans lequel $R^1$ représente un groupe cyano et $R^2$ représente un groupe -$CONR^9R^{10}$ [où $R^9$ est un atome d'hydrogène ou un groupe alkyle en $C_1$-$C_5$ et $R^{10}$ représente un groupe de formule :

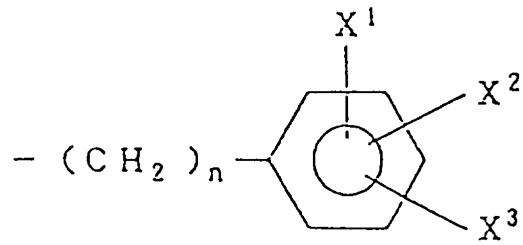

(dans laquelle $X^1$ représente un atome d'hydrogène, un atome d'halogène, un groupe alkyle en $C_1$-$C_5$, un groupe nitro ou un groupe cyano, $X^2$ et $X^3$ représentent un atome d'hydrogène, et n vaut 0)]
ou un groupe -$COR^{11}$ [où $R^{11}$ représente un groupe de formule :

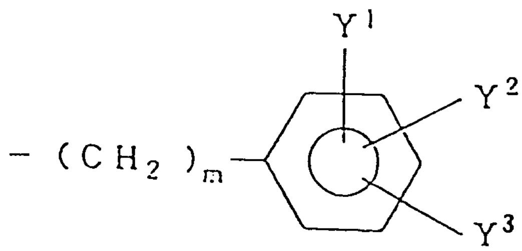

(dans laquelle $Y^1$ représente un atome d'hydrogène ou un atome d'halogène, $Y^2$ et $Y^3$ représentent un atome d'hydrogène et m vaut 0)],
$R^3$ représente un atome d'hydrogène, un groupe alkyle en $C_1$-$C_5$ ou un groupe hydroxy,
$R^4$, $R^5$, $R^6$, $R^7$ et $R^8$ représentent chacun indépendamment un atome d'hydrogène, un groupe hydroxy, un atome d'halogène, un groupe alcoxy en $C_1$-$C_5$, un groupe nitro, un groupe cyano, un groupe -$SO_2R^{14}$ (où $R^{14}$ représente un groupe alkyle en $C_1$-$C_5$), un groupe -$COR^{15}$ (où $R^{15}$ représente un groupe hydroxy), ou un groupe alkyle en $C_1$-$C_5$ éventuellement substitué par un atome d'halogène, et
au moins l'un de $R^4$ et $R^5$ est un groupe hydroxy, à condition que, lorsque $R^3$ est un atome d'hydrogène et $R^4$ à $R^8$ représentent chacun indépendamment un atome d'hydrogène, un groupe hydroxy, un groupe alcoxy en $C_1$-$C_5$ ou un groupe alkyle en $C_1$-$C_5$, alors $R^2$ est le groupe - $CONR^9R^{10}$ [où $R^9$ représente un atome d'hydrogène et $R^{10}$ représente un groupe de formule :

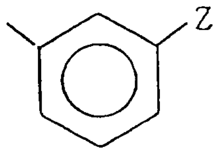

ou

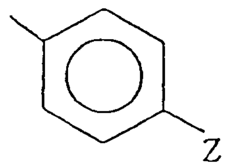

(dans laquelle Z représente un groupe alkyle en $C_1$-$C_5$, un atome d'halogène, un groupe nitro ou un groupe cyano)] ou un groupe -$COR^{11}$ [où $R^{11}$ est un groupe de formule :

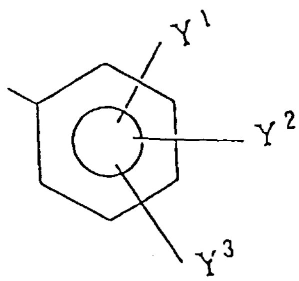

(dans laquelle $Y^1$ représente un atome d'halogène et $Y^2$ et $Y^3$ représentent un atome d'hydrogène) à condition que $R^{11}$ ne soit pas

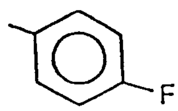

ni

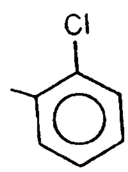

**3.** Composé selon la revendication 2, dans lequel :
$R^1$ représente un groupe cyano,
$R^2$ représente un groupe -$CONR^9R^{10}$ [où $R^9$ représente un atome d'hydrogène et $R^{10}$ représente un groupe de formule :

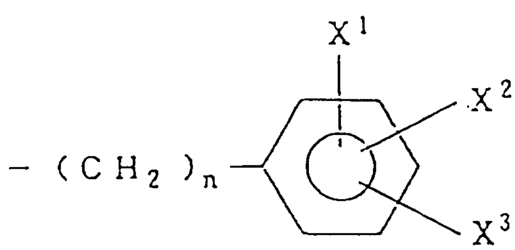

(dans laquelle $X^1$ représente un atome d'hydrogène, un atome d'halogène, un groupe alkyle en $C_1$-$C_5$, un groupe nitro ou un groupe cyano, $X^2$ et $X^3$ représentent un atome d'hydrogène et n vaut 0)]
ou un groupe -$COR^{11}$ [où $R^{11}$ représente un groupe de formule :

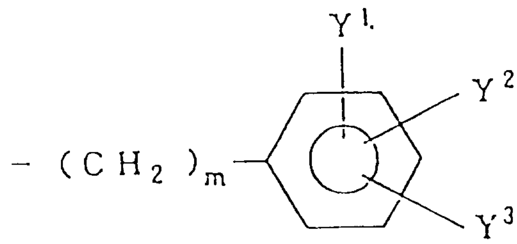

(dans laquelle $Y^1$, $Y^2$ et $Y^3$ représentent un atome d'hydrogène et m vaut 0)],
$R^3$ représente un atome d'hydrogène ou un groupe hydroxy,
$R^4$ et $R^8$ représentent chacun indépendamment un atome d'hydrogène, un atome d'halogène ou un groupe nitro,
$R^5$ et $R^7$ représentent un groupe hydroxy, un atome d'halogène, un groupe alcoxy en $C_1$-$C_5$, un groupe nitro, un groupe cyano, un groupe $SO_2R^{14}$ (où $R^{14}$ représente un groupe alkyle en $C_1$-$C_5$), un groupe $-COR^{15}$ (où $R^{15}$ représente un groupe hydroxy) ou un groupe alkyle en $C_1$-$C_5$ éventuellement substitué par un atome d'halogène,
$R^6$ représente un groupe hydroxy,
à condition que lorsque $R^3$, $R^4$ et $R^8$ sont un atome d'hydrogène et que $R^5$ et $R^7$ représentent chacun indépendamment un atome d'hydrogène, un groupe hydroxy, un groupe alcoxy en $C_1$-$C_5$ ou un groupe alkyle en $C_1$-$C_5$, alors $R^2$ est un groupe $-CONR^9R^{10}$ [où $R^9$ représente un atome d'hydrogène et $R^{10}$ représente un groupe de formule :

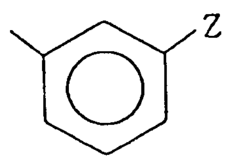

ou

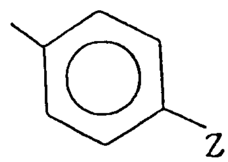

(dans laquelle Z représente un groupe alkyle en $C_1$-$C_5$, un atome d'halogène, un groupe nitro ou un groupe cyano)].

**4.** Composé selon la revendication 3, dans lequel :
$R^1$ représente un groupe cyano,
$R^2$ représente un groupe $-CONR^9R^{10}$ [où $R^9$ représente un atome d'hydrogène et $R^{10}$ représente un groupe de formule :

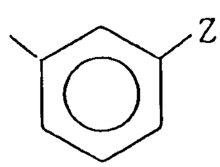

(dans laquelle Z représente un atome d'hydrogène ou un atome d'halogène)],
$R^3$ représente un atome d'hydrogène ou un groupe hydroxy,
$R^4$ représente un atome d'hydrogène,
$R^5$ représente un groupe hydroxy, un atome d'halogène, un groupe alcoxy en $C_1$-$C_5$ ou un groupe alkyle en $C_1$-$C_5$,
$R^6$ représente un groupe hydroxy,
$R^7$ représente un atome d'hydrogène, un atome d'halogène, un groupe cyano ou un groupe alkyle en $C_1$-$C_5$, et
$R^8$ représente un atome d'hydrogène, un atome d'halogène ou un groupe nitro,
à condition que lorsque $R^3$ et $R^8$ sont un atome d'hydrogène et que $R^5$ représente un groupe hydroxy, un groupe alcoxy en $C_1$-$C_5$ ou un groupe alkyle en $C_1$-$C_5$ et que $R^7$ représente un atome d'hydrogène ou un groupe alkyle en $C_1$-$C_5$, alors Z est un atome d'halogène.

**5.** Composition pharmaceutique comprenant des dérivés de styrène ayant la formule générale suivante (I) :

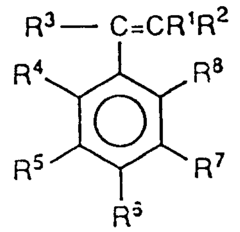

(I)

dans laquelle $R^1$ et $R^2$ représentent chacun indépendamment un groupe cyano, un groupe $CONR^9R^{10}$ [où $R^9$ et $R^{10}$ représentent chacun indépendamment un atome d'hydrogène, un groupe alkyle en $C_1$-$C_5$ ou un groupe de formule :

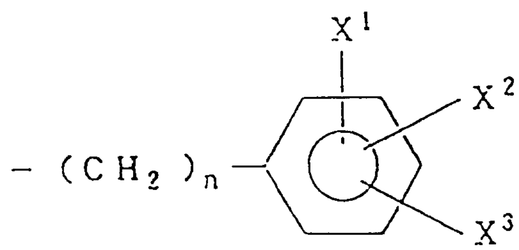

(dans laquelle n vaut 0 ou un nombre entier de 1 à 5, et $X^1$, $X^2$ et $X^3$ représentent chacun indépendamment un atome d'hydrogène, un atome d'halogène, un groupe alkyle en $C_1$-$C_5$, un groupe alcoxy en $C_1$-$C_5$, un groupe hydroxy, un groupe nitro ou un groupe cyano), à condition que $R^9$ et $R^{10}$ ne représentent pas en même temps un atome d'hydrogène] ou un groupe $-COR^{11}$ [où $R^{11}$ représente un groupe de formule :

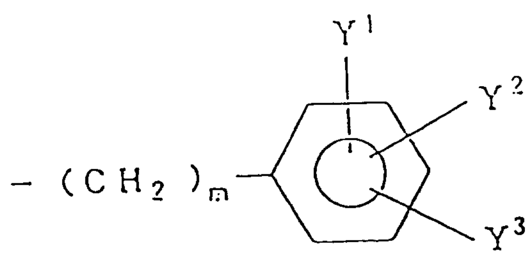

(dans laquelle m vaut 0 ou un nombre entier de 1 à 5, et $Y^1$, $Y^2$ et $Y^3$ représentent chacun indépendamment un atome d'hydrogène, un groupe alkyle en $C_1$-$C_5$, un groupe alcoxy en $C_1$-$C_5$, un groupe hydroxy ou un atome d'halogène)], mais $R^1$ et $R^2$ ne représentant pas en même temps un groupe cyano;

$R^3$ représente un atome d'hydrogène, un groupe alkyle en $C_1$-$C_5$ ou un groupe hydroxy ;

$R^4$, $R^5$, $R^6$, $R^7$ et $R^8$ représentent chacun indépendamment un atome d'hydrogène, un groupe hydroxy, un atome d'halogène, un groupe alcoxy en $C_1$-$C_5$, un groupe nitro, un groupe cyano ;

un groupe $-NR^{12}R^{13}$ [où $R^{12}$ et $R^{13}$ représentent chacun indépendamment un atome d'hydrogène, un groupe alkyle en $C_1$-$C_5$ ou un groupe benzoyle] ;

un groupe $-SO_pR^{14}$ [où p vaut 0, 1 ou 2, et $R^{14}$ représente un groupe alkyle en $C_1$-$C_5$ ou un groupe phényle] ;

un groupe $-COR^{15}$ [où $R^{15}$ représente un atome d'hydrogène, un groupe alkyle en $C_1$-$C_5$, un groupe alcoxy en $C_1$-$C_5$, un groupe hydroxy, un groupe phényle, un groupe phénoxy ou un groupe $-NHR^{16}$ (où $R^{16}$ représente un groupe alkyle en $C_1$-$C_5$ ou un groupe phényle)],

ou encore un groupe alkyle en $C_1$-$C_5$ éventuellement substitué par un atome d'halogène,

avec comme première condition qu'au moins l'un de $R^4$ à $R^8$ représente un groupe hydroxy, et que, lorsque $R^3$ est un atome d'hydrogène et $R^4$ à $R^8$ représentent chacun indépendamment un atome d'hydrogène, un groupe hydroxy, un groupe alcoxy en $C_1$-$C_5$, un groupe $-NR^{12}R^{13}$ (où $R^{12}$ et $R^{13}$ sont tels que définis ci-dessus) ou un groupe alkyle en $C_1$-$C_5$, alors $R^1$ représente un groupe cyano, $R^2$ représente un groupe $-CONR^9R^{10}$ [où $R^9$ représente un atome d'hydrogène ou un groupe alkyle en $C_1$-$C_5$ et $R^{10}$ représente un groupe de formule :

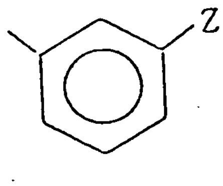

ou

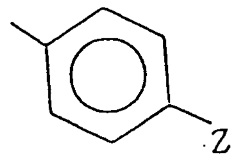

(dans laquelle Z représente un groupe alkyle en $C_1$-$C_5$, un atome d'halogène, un groupe nitro ou un groupe cyano)] ou un groupe -$COR^{11}$ [où $R^{11}$ représente un groupe de formule :

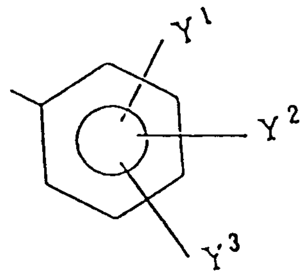

(dans laquelle $Y^1$, $Y^2$ et $R^3$ sont tels que définis ci-dessus), à condition que $R^{11}$ ne soit pas

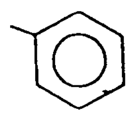

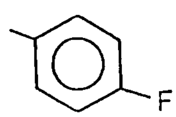

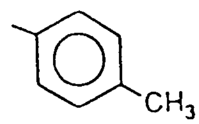

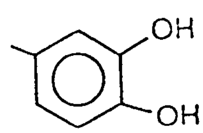

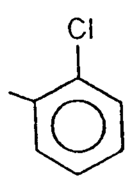

ni

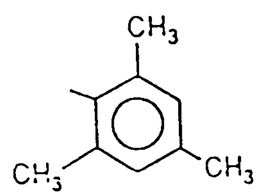

]]

et avec comme deuxième condition que le styrène de la formule I ne soit pas le N,N-diéthyl-2-cyano-3-(3,4-dihydroxy-5-nitrophényl)acrylamide,
ou leurs sels pharmaceutiquement acceptables.
et des véhicules pharmaceutiquement acceptables pour de tels composés.

6. Utilisation de dérivés de styrène de formule générale suivante (I) :

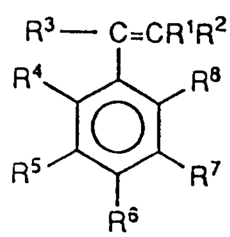

(I)

dans laquelle $R^1$ et $R^2$ représentent chacun indépendamment un groupe cyano un groupe $CONR^9R^{10}$ [où $R^9$ et $R^{10}$ représentent chacun indépendamment un atome d'hydrogène, un groupe alkyle en $C_1$-$C_5$ ou un groupe de formule :

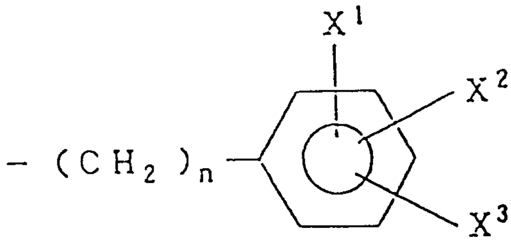

(dans laquelle n vaut 0 ou un nombre entier de 1 à 5, et $X^1$, $X^2$ et $X^3$ représentent chacun indépendamment un atome d'hydrogène, un atome d'halogène, un groupe alkyle en $C_1$-$C_5$, un groupe alcoxy en $C_1$-$C_5$, un groupe hydroxy, un groupe nitro ou un groupe cyano), à condition que $R^9$ et $R^{10}$ ne représentent pas en même temps un atome d'hydrogène] ou un groupe $-COR^{11}$ [où $R^{11}$ représente un groupe de formule :

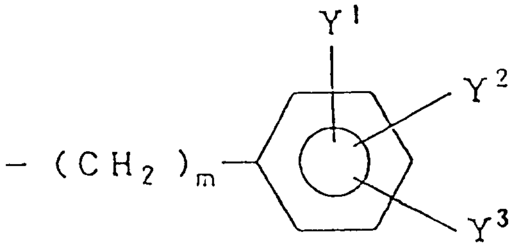

(dans laquelle m vaut 0 ou un nombre entier de 1 à 5, et $Y^1$, $Y^2$ et $Y^3$ représentent chacun indépendamment un atome d'hydrogène, un groupe alkyle en $C_1$-$C_5$, un groupe alcoxy en $C_1$-$C_5$, un groupe hydroxy ou un atome d'halogène)], mais $R^1$ et $R^2$ ne représentant pas en même temps un groupe cyano;

$R^3$ représente un atome d'hydrogène, un groupe alkyle en $C_1$-$C_5$ ou un groupe hydroxy ;

$R^4$, $R^5$, $R^6$, $R^7$ et $R^8$ représentent chacun indépendamment un atome d'hydrogène, un groupe hydroxy, un atome d'halogène, un groupe alcoxy en $C_1$-$C_5$, un groupe nitro, un groupe cyano ;

un groupe $-NR^{12}R^{13}$ [où $R^{12}$ et $R^{13}$ représentent chacun indépendamment un atome d'hydrogène, un groupe alkyle en $C_1$-$C_5$ ou un groupe benzoyle] ;

un groupe $-SO_pR^{14}$ [où p vaut 0, 1 ou 2, et $R^{14}$ représente un groupe alkyle en $C_1$-$C_5$ ou un groupe phényle] ;

un groupe $-COR^{15}$ [où $R^{15}$ représente un atome d'hydrogène, un groupe alkyle en $C_1$-$C_5$, un groupe alcoxy en $C_1$-$C_5$, un groupe hydroxy, un groupe phényle, un groupe phénoxy ou un groupe $-NHR^{16}$ (où $R^{16}$ représente un groupe alkyle en $C_1$-$C_5$ ou un groupe phényle)],

ou encore un groupe alkyle en $C_1$-$C_5$ éventuellement substitué par un atome d'halogène,

avec comme condition qu'au moins l'un de $R^4$ à $R^8$ représente un groupe hydroxy, et que, lorsque $R^3$ est un atome d'hydrogène et $R^4$ à $R^8$ représentent chacun indépendamment un atome d'hydrogène, un groupe hydroxy, un groupe alcoxy en $C_1$-$C_5$, un groupe $-NR^{12}R^{13}$ (où $R^{12}$ et $R^{13}$ sont tels que définis ci-dessus) ou un groupe alkyle en $C_1$-$C_5$, alors $R^1$ représente un groupe cyano, $R^2$ représente un groupe $-CONR^9R^{10}$ [où $R^9$ représente un atome d'hydrogène ou un groupe alkyle en $C_1$-$C_5$ et $R^{10}$ représente un groupe de formule :

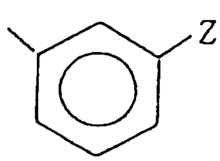

ou

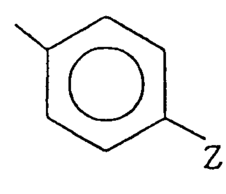

(dans laquelle Z représente un groupe alkyle en $C_1$-$C_5$, un atome d'halogène, un groupe nitro ou un groupe cyano)] ou un groupe $-COR^{11}$ [où $R^{11}$ représente un groupe de formule :

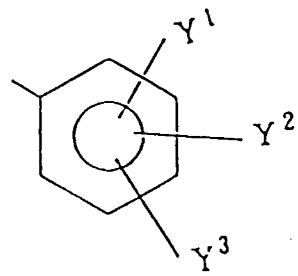

(dans laquelle $Y^1$, $Y^2$ et $Y^3$ sont tels que définis ci-dessus), à condition que $R^{11}$ ne soit pas

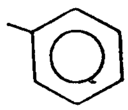 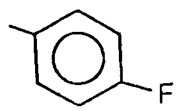 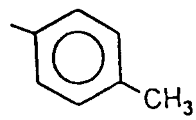 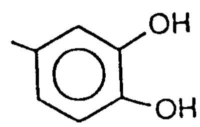

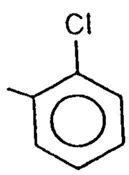 ni 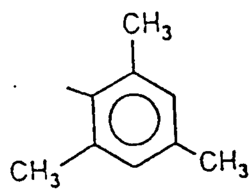

ou leurs sels pharmaceutiquement acceptables,
pour la fabrication d'un médicament efficace dans le traitement d'un cancer.

**Revendications pour l'Etat contractant suivant : ES**

**1.** Dérivés de styrène ayant la formule générale suivante (I) :

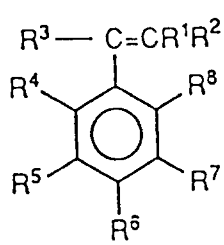

(I)

dans laquelle $R^1$ et $R^2$ représentent chacun indépendamment un groupe cyano, un groupe $CONR^9R^{10}$ [où $R^9$ et $R^{10}$ représentent chacun indépendamment un atome d'hydrogène, un groupe alkyle en $C_1$-$C_5$ ou un groupe de formule :

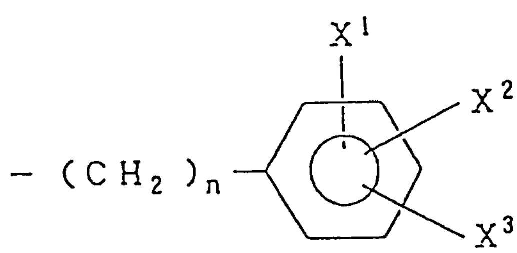

(dans laquelle n vaut 0 ou un nombre entier de 1 à 5, et $X^1$, $X^2$ et $X^3$ représentent chacun indépendamment un atome d'hydrogène, un atome d'halogène, un groupe alkyle en $C_1$-$C_5$, un groupe alcoxy en $C_1$-$C_5$, un groupe hydroxy, un groupe nitro ou un groupe cyano), à condition que $R^9$ et $R^{10}$ ne représentent pas en même temps un atome d'hydrogène] ou un groupe -$COR^{11}$ [où $R^{11}$ représente un groupe de formule :

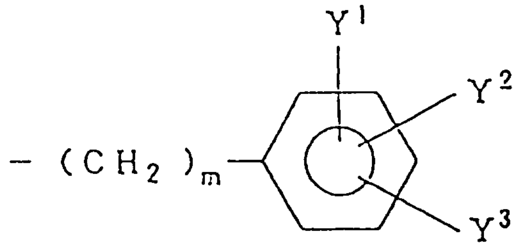

(dans laquelle m vaut 0 ou un nombre entier de 1 à 5, et $Y^1$, $Y^2$ et $Y^3$ représentent chacun indépendamment un atome d'hydrogène, un groupe alkyle en $C_1$-$C_5$, un groupe alcoxy en $C_1$-$C_5$, un groupe hydroxy ou un atome d'halogène)], mais $R^1$ et $R^2$ ne représentant pas en même temps un groupe cyano;

$R^3$ représente un atome d'hydrogène, un groupe alkyle en $C_1$-$C_5$ ou un groupe hydroxy ;

$R^4$, $R^5$, $R^6$, $R^7$ et $R^8$ représentent chacun indépendamment un atome d'hydrogène, un groupe hydroxy, un atome d'halogène, un groupe alcoxy en $C_1$-$C_5$, un groupe nitro, un groupe cyano ;

un groupe -$NR^{12}R^{13}$ [où $R^{12}$ et $R^{13}$ représentent chacun indépendamment un atome d'hydrogène, un groupe alkyle en $C_1$-$C_5$ ou un groupe benzoyle] ;

un groupe -$SO_pR^{14}$ [où p vaut 0, 1 ou 2, et $R^{14}$ représente un groupe alkyle en $C_1$-$C_5$ ou un groupe phényle] ;

un groupe -$COR^{15}$ [où $R^{15}$ représente un atome d'hydrogène, un groupe alkyle en $C_1$-$C_5$, un groupe alcoxy en $C_1$-$C_5$, un groupe hydroxy, un groupe phényle, un groupe phénoxy ou un groupe -$NHR^{16}$ (où $R^{16}$ représente un groupe alkyle en $C_1$-$C_5$ ou un groupe phényle)],

ou encore un groupe alkyle en $C_1$-$C_5$ éventuellement substitué par un atome d'halogène,

avec comme première condition qu'au moins l'un de $R^4$ à $R^8$ représente un groupe hydroxy, et que, lorsque $R^3$ est un atome d'hydrogène et $R^4$ à $R^8$ représentent chacun indépendamment un atome d'hydrogène, un groupe hydroxy,

un groupe alcoxy en $C_1$-$C_5$, un groupe -$NR^{12}R^{13}$ (où $R^{12}$ et $R^{13}$ sont tels que définis ci-dessus) ou un groupe alkyle en $C_1$-$C_5$, alors $R^1$ représente un groupe cyano, $R^2$ représente un groupe -$CONR^9R^{10}$ [où $R^9$ représente un atome d'hydrogène ou un groupe alkyle en $C_1$-$C_5$ et $R^{10}$ représente un groupe de formule :

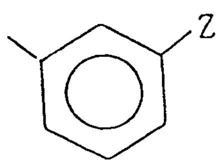

ou

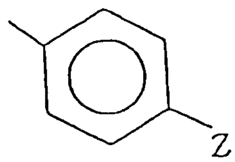

(dans laquelle Z représente un groupe alkyle en $C_1$-$C_5$, un atome d'halogène, un groupe nitro ou un groupe cyano)] ou un groupe -$COR^{11}$ [où $R^{11}$ représente un groupe de formule :

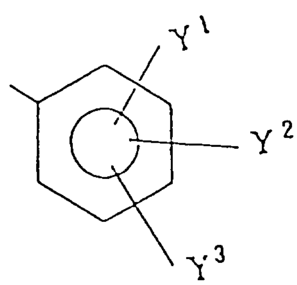

(dans laquelle $Y^1$, $Y^2$ et $Y^3$ sont tels que définis ci-dessus), à condition que $R^{11}$ ne soit pas

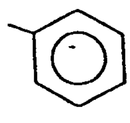 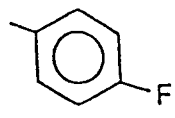 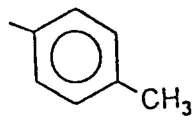 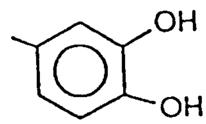

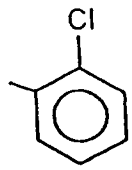 ni 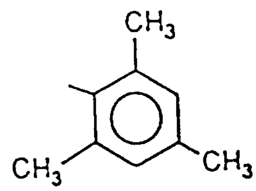 ]]

avec comme deuxième condition que lorsque $R^4$, $R^7$ et $R^8$ sont H, $R^6$ est un groupe hydroxy, $R^5$ est un groupe -$COR^{15}$ où $R^{15}$ représente un groupe hydroxy ou un groupe alcoxy en $C_1$-$C_5$, $R^3$ est l'hydrogène, $R^1$ est un groupe cyano et $R^2$ est un groupe -$CONR^9R^{10}$ où $R^9$ représente l'hydrogène, alors $R^{10}$ ne représente pas le groupe de formule :

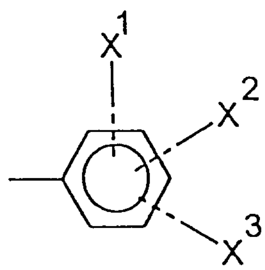

dans laquelle $X^1$, $X^2$ et $X^3$ représentent chacun indépendamment le chlore ou un groupe alkyle en $C_1$-$C_5$, et avec comme troisième condition que le styrène de la formule I ne soit pas le N,N-diéthyl-2-cyano-3-(3,4-dihydroxy-5-nitrophényl)acrylamide,
ou leurs sels pharmaceutiquement acceptables.

**2.** Composé selon la revendication 1, dans lequel $R^1$ représente un groupe cyano et $R^2$ représente un groupe -$CONR^9R^{10}$ [où $R^9$ est un atome d'hydrogène ou un groupe alkyle en $C_1$-$C_5$ et $R^{10}$ représente un groupe de formule :

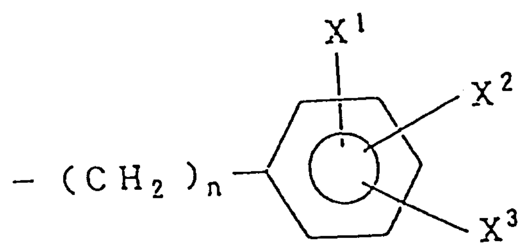

(dans laquelle $X^1$ représente un atome d'hydrogène, un atome d'halogène, un groupe alkyle en $C_1$-$C_5$, un groupe nitro ou un groupe cyano, $X^2$ et $X^3$ représentent un atome d'hydrogène, et n vaut 0)]
ou un groupe -$COR^{11}$ [où $R^{11}$ représente un groupe de formule :

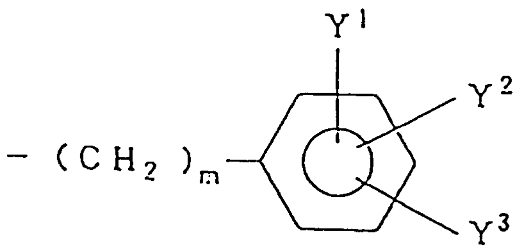

(dans laquelle $Y^1$ représente un atome d'hydrogène ou un atome d'halogène, $Y^2$ et $Y^3$ représentent un atome d'hydrogène et m vaut 0)],
$R^3$ représente un atome d'hydrogène, un groupe alkyle en $C_1$-$C_5$ ou un groupe hydroxy,
$R^4$, $R^5$, $R^6$, $R^7$ et $R^8$ représentent chacun indépendamment un atome d'hydrogène, un groupe hydroxy, un atome d'halogène, un groupe alcoxy en $C_1$-$C_5$, un groupe nitro, un groupe cyano un groupe $-SO_2R^{14}$ (où $R^{14}$ représente un groupe alkyle en $C_1$-$C_5$), un groupe $-COR^{15}$ (où $R^{15}$ représente un groupe hydroxy) ou un groupe alkyle en $C_1$-$C_5$ éventuellement substitué par un atome d'halogène, et
au moins l'un de $R^4$ et $R^5$ est un groupe hydroxy, à condition que, lorsque $R^3$ est un atome d'hydrogène et $R^4$ à $R^8$ représentent chacun indépendamment un atome d'hydrogène, un groupe hydroxy, un groupe alcoxy en $C_1$-$C_5$ ou un groupe alkyle en $C_1$-$C_5$, alors $R^2$ est le groupe - $CONR^9R^{10}$ [où $R^9$ représente un atome d'hydrogène et $R^{10}$ représente un groupe de formule :

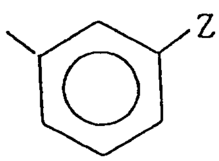

ou

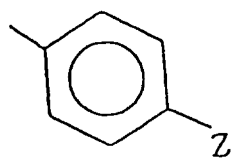

(dans laquelle Z représente un groupe alkyle en $C_1$-$C_5$, un atome d'halogène, un groupe nitro ou un groupe cyano)] ou un groupe $-COR^{11}$ [où $R^{11}$ est un groupe de formule :

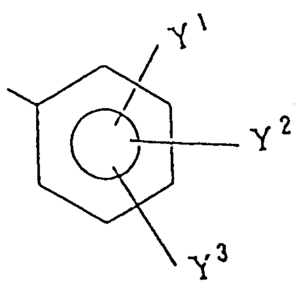

(dans laquelle $Y^1$ représente un atome d'halogène et $Y^2$ et $Y^3$ représentent un atome d'hydrogène), à condition que $R^{11}$ ne soit pas

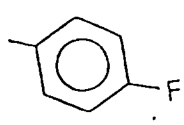

ni

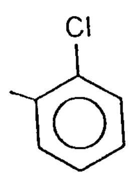

**3.** Composé selon la revendication 2, dans lequel :
$R^1$ représente un groupe cyano,
$R^2$ représente un groupe $-CONR^9R^{10}$ [où $R^9$ représente un atome d'hydrogène et $R^{10}$ représente un groupe de formule :

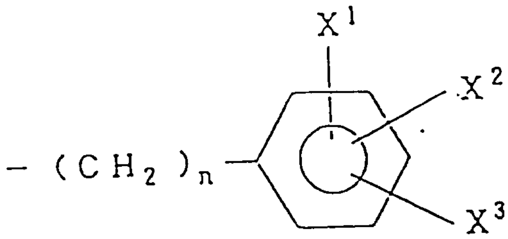

(dans laquelle $X^1$ représente un atome d'hydrogène, un atome d'halogène, un groupe alkyle en $C_1$-$C_5$, un groupe nitro ou un groupe cyano, $X^2$ et $X^3$ représentent un atome d'hydrogène et n vaut 0)]
ou un groupe $-COR^{11}$ [où $R^{11}$ représente un groupe de formule :

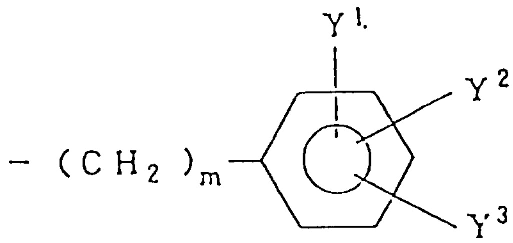

(dans laquelle $Y^1$, $Y^2$ et $Y^3$ représentent un atome d'hydrogène et m vaut 0)],
$R^3$ représente un atome d'hydrogène ou un groupe hydroxy,
$R^4$ et $R^8$ représentent chacun indépendamment un atome d'hydrogène, un atome d'halogène ou un groupe nitro,
$R^5$ et $R^7$ représentent un groupe hydroxy, un atome d'halogène, un groupe alcoxy en $C_1$-$C_5$, un groupe nitro, un groupe cyano, un groupe $SO_2R^{14}$ (où $R^{14}$ représente un groupe alkyle en $C_1$-$C_5$), un groupe $-COR^{15}$ (où $R^{15}$ représente un groupe hydroxy) ou un groupe alkyle en $C_1$-$C_5$ éventuellement substitué par un atome d'halogène,
$R^6$ représente un groupe hydroxy,
à condition que lorsque $R^3$, $R^4$ et $R^8$ sont un atome d'hydrogène et que $R^5$ et $R^7$ représentent chacun indépendamment un atome d'hydrogène, un groupe hydroxy, un groupe alcoxy en $C_1$-$C_5$ ou un groupe alkyle en $C_1$-$C_5$, alors $R^2$ est un groupe $-CONR^9R^{10}$ [où $R^9$ représente un atome d'hydrogène et $R^{10}$ représente un groupe de formule :

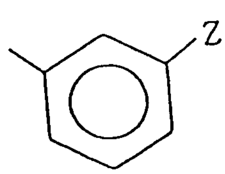

ou

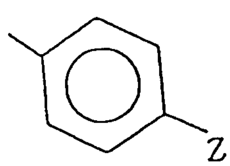

(dans laquelle Z représente un groupe alkyle en $C_1$-$C_5$, un atome d'halogène, un groupe nitro ou un groupe cyano)].

**4.** Composé selon la revendication 3, dans lequel :
$R^1$ représente un groupe cyano,
$R^2$ représente un groupe $-CONR^9R^{10}$ [où $R^9$ représente un atome d'hydrogène et $R^{10}$ représente un groupe de formule :

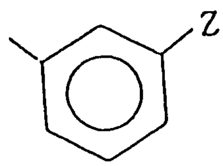

(dans laquelle Z représente un atome d'hydrogène ou un atome d'halogène)], $R^3$ représente un atome d'hydrogène ou un groupe hydroxy, $R^4$ représente un atome d'hydrogène, $R^5$ représente un groupe hydroxy, un atome d'halogène, un groupe alcoxy en $C_1$-$C_5$ ou un groupe alkyle en $C_1$-$C_5$, $R^6$ représente un groupe hydroxy, $R^7$ représente un atome d'hydrogène, un atome d'halogène, un groupe cyano ou un groupe alkyle en $C_1$-$C_5$, et $R^8$ représente un atome d'hydrogène, un atome d'halogène ou un groupe nitro, à condition que lorsque $R^3$ et $R^8$ sont un atome d'hydrogène et que $R^5$ représente un groupe hydroxy, un groupe alcoxy en $C_1$-$C_5$ ou un groupe alkyle en $C_1$-$C_5$ et que $R^7$ représente un atome d'hydrogène ou un groupe alkyle en $C_1$-$C_5$, alors Z est un atome d'halogène.

**5.** Composition pharmaceutique comprenant des dérivés de styrène ayant la formule générale suivante (I) :

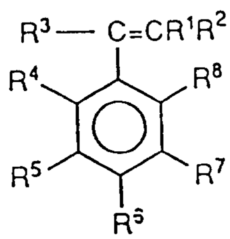

(I)

dans laquelle $R^1$ et $R^2$ représentent chacun indépendamment un groupe cyano, un groupe $CONR^9R^{10}$ [où $R^9$ et $R^{10}$ représentent chacun indépendamment un atome d'hydrogène, un groupe alkyle en $C_1$-$C_5$ ou un groupe de formule :

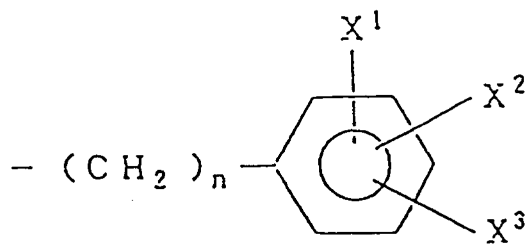

(dans laquelle n vaut 0 ou un nombre entier de 1 à 5, et $X^1$, $X^2$ et $X^3$ représentent chacun indépendamment un atome d'hydrogène, un atome d'halogène, un groupe alkyle en $C_1$-$C_5$, un groupe alcoxy en $C_1$-$C_5$, un groupe hydroxy, un groupe nitro ou un groupe cyano), à condition que $R^9$ et $R^{10}$ ne représentent pas en même temps un atome d'hydrogène] ou un groupe $-COR^{11}$ [où $R^{11}$ représente un groupe de formule :

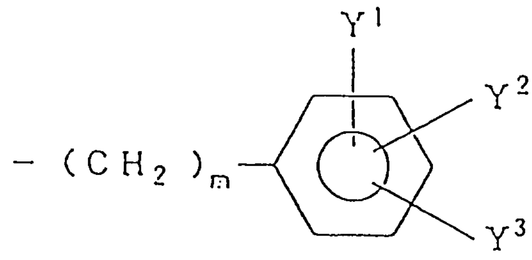

(dans laquelle m vaut 0 ou un nombre entier de 1 à 5, et $Y^1$, $Y^2$ et $Y^3$ représentent chacun indépendamment un atome d'hydrogène, un groupe alkyle en $C_1$-$C_5$, un groupe alcoxy en $C_1$-$C_5$, un groupe hydroxy ou un atome d'halogène)], mais $R^1$ et $R^2$ ne représentant pas en même temps un groupe cyano;
$R^3$ représente un atome d'hydrogène, un groupe alkyle en $C_1$-$C_5$ ou un groupe hydroxy ;
$R^4$, $R^5$, $R^6$, $R^7$ et $R^8$ représentent chacun indépendamment un atome d'hydrogène, un groupe hydroxy, un atome d'halogène, un groupe alcoxy en $C_1$-$C_5$, un groupe nitro, un groupe cyano ;
un groupe $-NR^{12}R^{13}$ [où $R^{12}$ et $R^{13}$ représentent chacun indépendamment un atome d'hydrogène, un groupe alkyle en $C_1$-$C_5$ ou un groupe benzoyle] ;
un groupe $-SO_pR^{14}$ [où p vaut 0, 1 ou 2, et $R^{14}$ représente un groupe alkyle en $C_1$-$C_5$ ou un groupe phényle] ;
un groupe $-COR^{15}$ [où $R^{15}$ représente un atome d'hydrogène,
un groupe alkyle en $C_1$-$C_5$, un groupe alcoxy en $C_1$-$C_5$, un groupe hydroxy, un groupe phényle, un groupe phénoxy ou un groupe $-NHR^{16}$ (où $R^{16}$ représente un groupe alkyle en $C_1$-$C_5$ ou un groupe phényle)],
ou encore un groupe alkyle en $C_1$-$C_5$ éventuellement substitué par un atome d'halogène,
avec comme première condition qu'au moins l'un de $R^4$ à $R^8$ représente un groupe hydroxy, et que, lorsque $R^3$ est un atome d'hydrogène et $R^4$ à $R^8$ représentent chacun indépendamment un atome d'hydrogène, un groupe hydroxy,
un groupe alcoxy en $C_1$-$C_5$, un groupe $-NR^{12}R^{13}$ (où $R^{12}$ et $R^{13}$ sont tels que définis ci-dessus) ou un groupe alkyle en $C_1$-$C_5$, alors $R^1$ représente un groupe cyano, $R^2$ représente un groupe $-CONR^9R^{10}$ [où $R^9$ représente un atome d'hydrogène ou un groupe alkyle en $C_1$-$C_5$ et $R^{10}$ représente un groupe de formule :

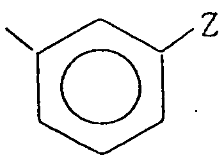

ou

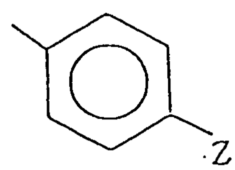

(dans laquelle Z représente un groupe alkyle en $C_1$-$C_5$, un atome d'halogène, un groupe nitro ou un groupe cyano)] ou un groupe $-COR^{11}$ [où $R^{11}$ représente un groupe de formule :

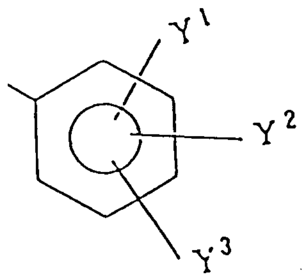

(dans laquelle $Y^1$, $Y^2$ et $Y^3$ sont tels que définis ci-dessus), à condition que $R^{11}$ ne soit pas

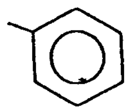 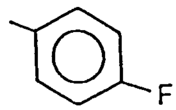 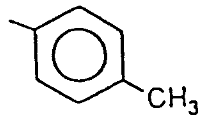 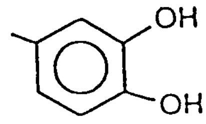

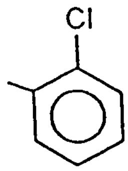 ni 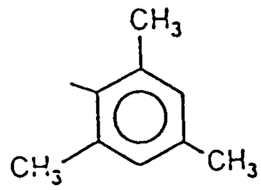 ]]

et avec comme deuxième condition que le styrène de la formule I ne soit pas le N,N-diéthyl-2-cyano-3-(3,4-dihydroxy-5-nitrophényl)acrylamide,
ou leurs sels pharmaceutiquement acceptables,
et des véhicules pharmaceutiquement acceptables pour de tels composés.

6. Utilisation de dérivés de styrène de formule générale suivante (I) :

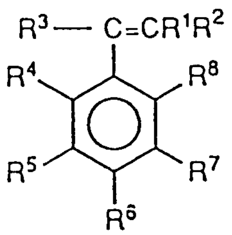

(I)

dans laquelle $R^1$ et $R^2$ représentent chacun indépendamment un groupe cyano, un groupe $CONR^9R^{10}$ [où $R^9$ et $R^{10}$ représentent chacun indépendamment un atome d'hydrogène, un groupe alkyle en $C_1$-$C_5$ ou un groupe de formule :

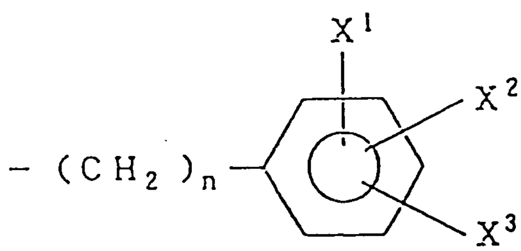

(dans laquelle n vaut 0 ou un nombre entier de 1 à 5, et $X^1$, $X^2$ et $X^3$ représentent chacun indépendamment un atome d'hydrogène, un atome d'halogène, un groupe alkyle en $C_1$-$C_5$, un groupe alcoxy en $C_1$-$C_5$, un groupe hydroxy, un groupe nitro ou un groupe cyano), à condition que $R^9$ et $R_{10}$ ne représentent pas en même temps un atome d'hydrogène] ou un groupe $-COR^{11}$ [où $R^{11}$ représente un groupe de formule :

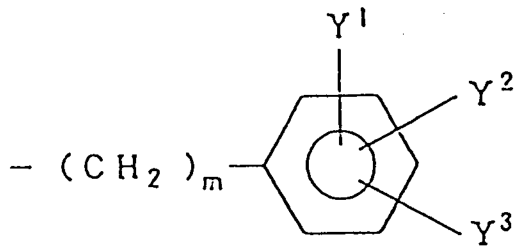

(dans laquelle m vaut 0 ou un nombre entier de 1 à 5, et $Y^1$, $Y^2$ et $Y^3$ représentent chacun indépendamment un atome d'hydrogène, un groupe alkyle en $C_1$-$C_5$, un groupe alcoxy en $C_1$-$C_5$, un groupe hydroxy ou un atome d'halogène)], mais $R^1$ et $R^2$ ne représentant pas en même temps un groupe cyano;

$R^3$ représente un atome d'hydrogène, un groupe alkyle en $C_1$-$C_5$ ou un groupe hydroxy ;

$R^4$, $R^5$, $R^6$, $R^7$ et $R^8$ représentent chacun indépendamment un atome d'hydrogène, un groupe hydroxy, un atome d'halogène, un groupe alcoxy en $C_1$-$C_5$, un groupe nitro, un groupe cyano ;

un groupe $-NR^{12}R^{13}$ [où $R^{12}$ et $R^{13}$ représentent chacun indépendamment un atome d'hydrogène, un groupe alkyle en $C_1$-$C_5$ ou un groupe benzoyle] ;

un groupe $-SO_pR^{14}$ [où p vaut 0, 1 ou 2 et $R^{14}$ représente un groupe alkyle en $C_1$-$C_5$ ou un groupe phényle] ;

un groupe $-COR^{15}$ [où $R^{15}$ représente un atome d'hydrogène, un groupe alkyle en $C_1$-$C_5$, un groupe alcoxy en $C_1$-$C_5$, un groupe hydroxy, un groupe phényle, un groupe phénoxy ou un groupe $-NHR^{16}$ (où $R^{16}$ représente un groupe alkyle en $C_1$-$C_5$ ou un groupe phényle)],

ou encore un groupe alkyle en $C_1$-$C_5$ éventuellement substitué par un atome d'halogène,

avec comme condition qu'au moins l'un de $R^4$ à $R^8$ représente un groupe hydroxy, et que, lorsque $R^3$ est un atome d'hydrogène et $R^4$ à $R^8$ représentent chacun indépendamment un atome d'hydrogène, un groupe hydroxy,

un groupe alcoxy en $C_1$-$C_5$, un groupe $-NR^{12}R^{13}$ (où $R^{12}$ et $R^{13}$ sont tels que définis ci-dessus) ou un groupe alkyle en $C_1$-$C_5$, alors $R^1$ représente un groupe cyano, $R^2$ représente un groupe $-CONR^9R^{10}$ [où $R^9$ représente un atome d'hydrogène ou un groupe alkyle en $C_1$-$C_5$ et $R^{10}$ représente un groupe de formule :

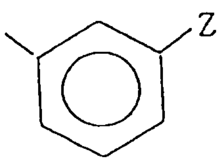

ou

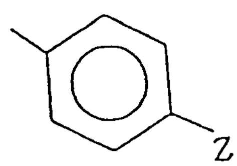

(dans laquelle Z représente un groupe alkyle en $C_1$-$C_5$, un atome d'halogène, un groupe nitro ou un groupe cyano)] ou un groupe $-COR^{11}$ [où $R^{11}$ représente un groupe de formule :

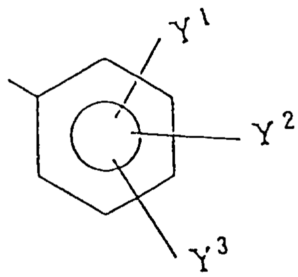

(dans laquelle $Y^1$, $Y^2$ et $Y^3$ sont tels que définis ci-dessus), à condition que $R^{11}$ ne soit pas

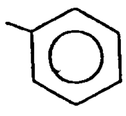 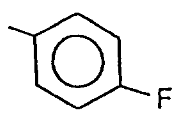 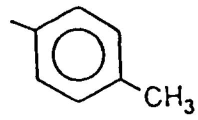 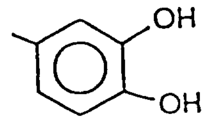

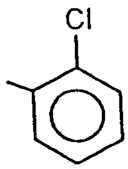 ni 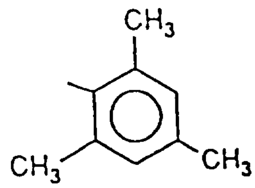 ]]

ou leurs sels pharmaceutiquement acceptables,
pour la fabrication d'un médicament efficace dans le traitement d'un cancer.

7. Procédé pour la préparation de dérivés de styrène ayant la formule générale suivante (I) :

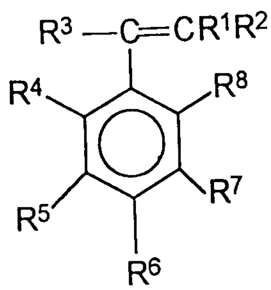

(I)

dans laquelle $R^1$ et $R^2$ représentent chacun indépendamment un groupe cyano, un groupe $CONR^9R^{10}$ [où $R^9$ et $R^{10}$ représentent chacun indépendamment un atome d'hydrogène, un groupe alkyle en $C_1$-$C_5$ ou un groupe de formule :

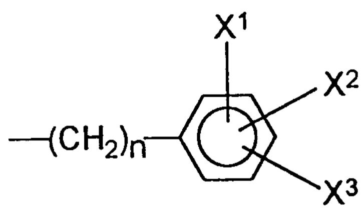

(dans laquelle n vaut 0 ou un nombre entier de 1 à 5, et $X^1$, $X^2$ et $X^3$ représentent chacun indépendamment un atome d'hydrogène, un atome d'halogène, un groupe alkyle en $C_1$-$C_5$, un groupe alcoxy en $C_1$-$C_5$, un groupe hydroxy, un groupe nitro ou un groupe cyano), à condition que $R^9$ et $R^{10}$ ne représentent pas en même temps un atome d'hydrogène] ou un groupe $-COR^{11}$ [où $R^{11}$ représente un groupe de formule :

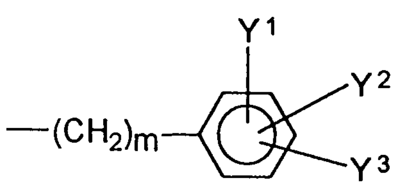

(dans laquelle m vaut 0 ou un nombre entier de 1 à 5, et $Y^1$, $Y^2$ et $Y^3$ représentent chacun indépendamment un atome d'hydrogène, un groupe alkyle en $C_1$-$C_5$, un groupe alcoxy en $C_1$-$C_5$, un groupe hydroxy ou un atome d'halogène)], mais $R^1$ et $R^2$ ne représentant pas en même temps un groupe cyano ;

$R^3$ représente un atome d'hydrogène ou un groupe alkyle en $C_1$-$C_5$ ;
$R^4$, $R^5$, $R^6$, $R^7$ et $R^8$ représentent chacun indépendamment un atome d'hydrogène, un groupe hydroxy, un atome d'halogène, un groupe alcoxy en $C_1$-$C_5$, un groupe nitro, un groupe cyano ;
un groupe -$NR^{12}R^{13}$ [où $R^{12}$ et $R^{13}$ représentent chacun indépendamment un atome d'hydrogène, un groupe alkyle en $C_1$-$C_5$ ou un groupe benzoyle] ;
un groupe -$SO_pR^{14}$ [où p vaut 0, 1 ou 2, et $R^{14}$ représente un groupe alkyle en $C_1$-$C_5$ ou un groupe phényle] ;
un groupe -$COR^{15}$ [où $R^{15}$ représente un atome d'hydrogène,
un groupe alkyle en $C_1$-$C_5$, un groupe alcoxy en $C_1$-$C_5$, un groupe hydroxy, un groupe phényle, un groupe phénoxy ou un groupe -$NHR^{16}$ (où $R^{16}$ représente un groupe alkyle en $C_1$-$C_5$ ou un groupe phényle)],
ou encore un groupe alkyle en $C_1$-$C_5$ éventuellement substitué par un atome d'halogène,
avec comme première condition qu'au moins l'un de $R^4$ à $R^8$ représente un groupe hydroxy, et que, lorsque $R^3$ est un atome d'hydrogène et $R^4$ à $R^8$ représentent chacun indépendamment un atome d'hydrogène, un groupe hydroxy, un groupe alcoxy en $C_1$-$C_5$, un groupe -$NR^{12}R^{13}$ (où $R^{12}$ et $R^{13}$ sont tels que définis ci-dessus) ou un groupe alkyle en $C_1$-$C_5$, alors $R^1$ représente un groupe cyano, $R^2$ représente un groupe -$CONR^9R^{10}$ [où $R^9$ représente un atome d'hydrogène ou un groupe alkyle en $C_1$-$C_5$ et $R^{10}$ représente un groupe de formule :

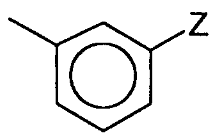

ou

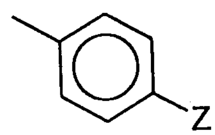

(dans laquelle Z représente un groupe alkyle en $C_1$-$C_5$, un atome d'halogène, un groupe nitro ou un groupe cyano)] ou un groupe -$COR^{11}$ [où $R^{11}$ représente un groupe de formule :

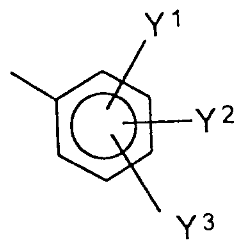

(dans laquelle $Y^1$, $Y^2$ et $Y^3$ sont tels que définis ci-dessus), à condition que $R^{11}$ ne soit pas

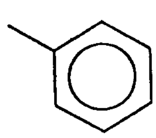
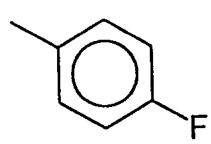
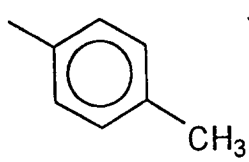
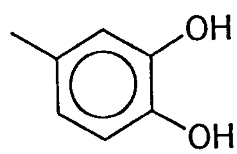
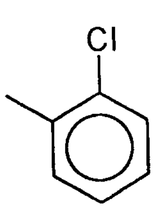

ni

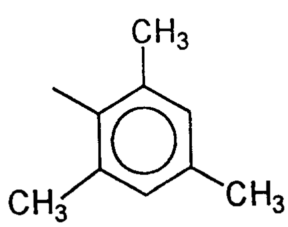

avec comme deuxième condition que lorsque $R^4$, $R^7$ et $R^8$ sont H, $R^6$ est un groupe hydroxy, $R^5$ est un groupe -$COR^{15}$ où $R^{15}$ représente un groupe hydroxy ou un groupe alcoxy en $C_1$-$C_5$, $R^3$ est l'hydrogène, $R^1$ est un groupe cyano et $R^2$ est un groupe -$CONR^9R^{10}$ où $R^9$ représente l'hydrogène, alors $R^{10}$ ne représente pas le groupe de formule :

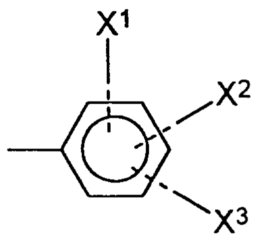

dans laquelle $X^1$, $X^2$ et $X^3$ représentent chacun indépendamment le chlore ou un groupe alkyle en $C_1$-$C_5$, et avec comme troisième condition que le styrène de la formule (I) ne soit pas le N,N-diéthyl-2-cyano-3-(3,4-dihydroxy-5-nitrophényl)acrylamide, par condensation du composé de formule (II) :

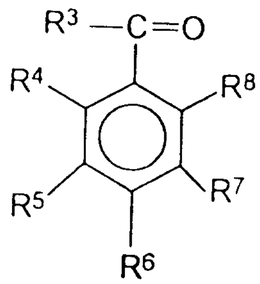

(II)

dans laquelle $R^3$ à $R^8$ ont les mêmes significations que ci-dessus, avec le composé de formule (III) :

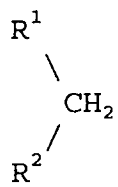

(III)

dans laquelle $R^1$ et $R^2$ ont les mêmes significations que ci-dessus; ou par protection du ou des groupes hydroxy dans le composé de formule (I) ci-dessus, condensation du produit ainsi obtenu avec le composé de formule (III) ci-dessus et déprotection du ou desdits groupes hydroxy du produit obtenu à l'étape de condensation.

**8.** Procédé pour la préparation de dérivés de styrène ayant la formule générale suivante (Ia) :

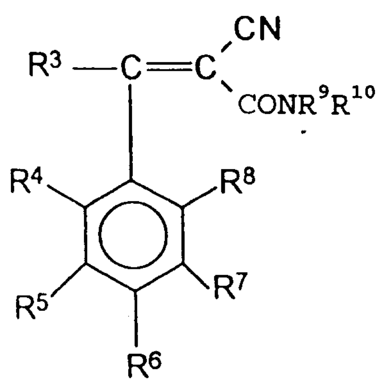

(Ia)

dans laquelle $R^9$ et $R^{10}$ représentent chacun indépendamment un atome d'hydrogène, un groupe alkyle en $C_1$-$C_5$

ou un groupe de formule :

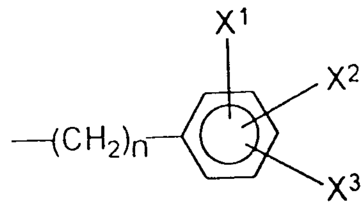

(dans laquelle n vaut 0 ou un nombre entier de 1 à 5, et $X^1$, $X^2$ et $X^3$ représentent chacun indépendamment un atome d'hydrogène, un atome d'halogène, un groupe alkyle en $C_1$-$C_5$, un groupe alcoxy en $C_1$-$C_5$, un groupe hydroxy, un groupe nitro ou un groupe cyano), à condition que $R^9$ et $R^{10}$ ne représentent pas en même temps un atome d'hydrogène

$R^3$ représente un atome d'hydrogène ou un groupe alkyle en $C_1$-$C_5$ ;

$R^4$, $R^5$, $R^6$, $R^7$ et $R^8$ représentent chacun indépendamment un atome d'hydrogène, un groupe hydroxy, un atome d'halogène, un groupe alcoxy en $C_1$-$C_5$, un groupe nitro, un groupe cyano ;

un groupe $-NR^{12}R^{13}$ [où $R^{12}$ et $R^{13}$ représentent chacun indépendamment un atome d'hydrogène, un groupe alkyle en $C_1$-$C_5$ ou un groupe benzoyle] ;

un groupe $-SO_pR^{14}$ [où p vaut 0, 1 ou 2, et $R^{14}$ représente un groupe alkyle en $C_1$-$C_5$ ou un groupe phényle] ;

un groupe $-COR^{15}$ [où $R^{15}$ représente un atome d'hydrogène,

un groupe alkyle en $C_1$-$C_5$, un groupe alcoxy en $C_1$-$C_5$, un groupe hydroxy, un groupe phényle, un groupe phénoxy ou un groupe $-NHR^{16}$ (où $R^{16}$ représente un groupe alkyle en $C_1$-$C_5$ ou un groupe phényle)],

ou encore un groupe alkyle en $C_1$-$C_5$ éventuellement substitué par un atome d'halogène,

avec comme première condition qu'au moins l'un de $R^4$ à $R^8$ représente un groupe hydroxy, et que, lorsque $R^3$ est un atome d'hydrogène et $R^4$ à $R^8$ représentent chacun indépendamment un atome d'hydrogène, un groupe hydroxy,

un groupe alcoxy en $C_1$-$C_5$, un groupe $-NR^{12}R^{13}$ (où $R^{12}$ et $R^{13}$ sont tels que définis ci-dessus) ou un groupe alkyle en $C_1$-$C_5$, alors $R^9$ représente un atome d'hydrogène ou un groupe alkyle en $C_1$-$C_5$ et $R^{10}$ représente un groupe de formule :

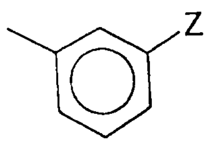

ou

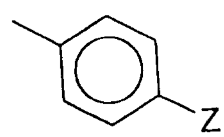

(dans laquelle Z représente un groupe alkyle en $C_1$-$C_5$, un atome d'halogène, un groupe nitro ou un groupe cyano) avec comme deuxième condition que lorsque $R^4$, $R^7$ et $R^8$ sont H, $R^6$ est un groupe hydroxy, $R^5$ est un groupe -$COR^{15}$ où $R^{15}$ représente un groupe hydroxy ou un groupe alcoxy en $C_1$-$C_5$, $R^3$ est l'hydrogène, et $R^9$ représente l'hydrogène, alors $R^{10}$ ne représente pas le groupe de formule :

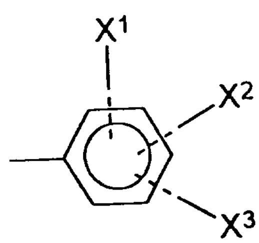

dans laquelle $X^1$, $X^2$ et $X^3$ représentent chacun indépendamment le chlore ou un groupe alkyle en $C_1$-$C_5$, et avec comme troisième condition que le styrène de la formule I ne soit pas le N,N-diéthyl-2-cyano-3-(3,4-dihydroxy-5-nitrophényl)acrylamide,

qui comprend les étapes consistant à :

condenser le composé de formule générale (II) :

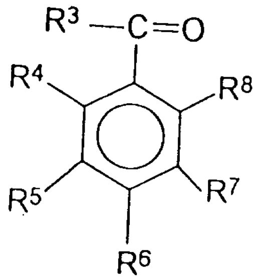

(II)

dans laquelle $R^3$ à $R^8$ ont les mêmes significations que ci-dessus, avec de l'acide cyanoacétique, pour obtenir le composé de formule générale suivante (IV) :

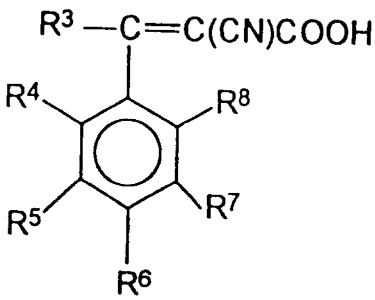

(IV)

dans laquelle $R^3$ à $R^8$ ont les mêmes significations que ci-dessus, et condenser ledit composé (IV) avec l'amine de formule générale suivante (V) :

$$R^9R^{10}NH \qquad (V)$$

dans laquelle $R^9$ et $R^{10}$ ont les mêmes significations que ci-dessus.

**9.** Procédé pour la préparation de dérivés de styrène ayant la formule générale suivante (Ib) :

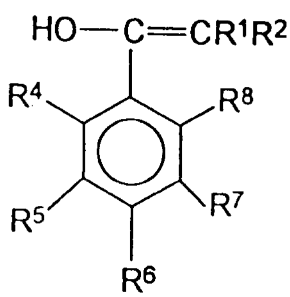

(Ib)

[dans laquelle $R^1$ et $R^2$ représentent chacun indépendamment un groupe cyano, un groupe $CONR^9R^{10}$ [où $R^9$ et $R^{10}$ représentent chacun indépendamment un atome d'hydrogène, un groupe alkyle en $C_1$-$C_5$ ou un groupe de formule :

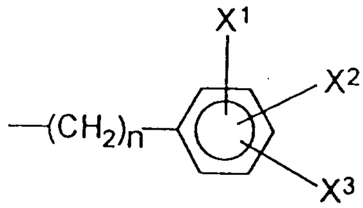

(dans laquelle n vaut 0 ou un nombre entier de 1 à 5, et $X^1$, $X^2$ et $X^3$ représentent chacun indépendamment un atome d'hydrogène, un atome d'halogène, un groupe alkyle en $C_1$-$C_5$, un groupe alcoxy en $C_1$-$C_5$, un groupe hydroxy, un groupe nitro ou un groupe cyano), à condition que $R^9$ et $R^{10}$ ne représentent pas en même temps un atome d'hydrogène] ou un groupe $-COR^{11}$ [où $R^{11}$ représente un groupe de formule :

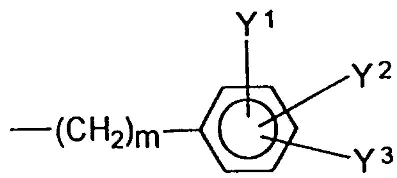

(dans laquelle m vaut 0 ou un nombre entier de 1 à 5, et $Y^1$, $Y^2$ et $Y^3$ représentent chacun indépendamment un atome d'hydrogène, un groupe alkyle en $C_1$-$C_5$, un groupe alcoxy en $C_1$-$C_5$, un groupe hydroxy ou un atome d'halogène)], mais $R^1$ et $R^2$ ne représentant pas en même temps un groupe cyano ;

$R^4$, $R^5$, $R^6$, $R^7$ et $R^8$ représentent chacun indépendamment un atome d'hydrogène, un groupe hydroxy, un atome d'halogène, un groupe alcoxy en $C_1$-$C_5$, un groupe nitro, un groupe cyano ;

un groupe $-NR^{12}R^{13}$ [où $R^{12}$ et $R^{13}$ représentent chacun indépendamment un atome d'hydrogène, un groupe alkyle en $C_1$-$C_5$ ou un groupe benzoyle] ;

un groupe $-SO_pR^{14}$ [où p vaut 0, 1 ou 2, et $R^{14}$ représente un groupe alkyle en $C_1$-$C_5$ ou un groupe phényle] ;

un groupe $-COR^{15}$ [où $R^{15}$ représente un atome d'hydrogène,

un groupe alkyle en $C_1$-$C_5$, un groupe alcoxy en $C_1$-$C_5$, un groupe hydroxy, un groupe phényle, un groupe phénoxy ou un groupe $-NHR^{16}$ (où $R^{16}$ représente un groupe alkyle en $C_1$-$C_5$ ou un groupe phényle)],

ou encore un groupe alkyle en $C_1$-$C_5$ éventuellement substitué par un atome d'halogène,

à condition qu'au moins l'un de $R^4$ à $R^8$ représente un groupe hydroxy,

comprenant les étapes consistant à :

protéger le groupe hydroxy dans des composés de formule générale (IIa) :

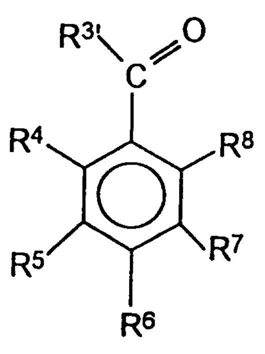

(IIa)

dans laquelle $R^4$ à $R^8$ sont tels que définis ci-dessus et $R^{3'}$ est un groupe hydroxy,

faire réagir avec du chlorure de thionyle ou du chlorure d'oxalyle pour obtenir le composé de formule (IIa) dans lequel $R^{3'}$ est le chlore et au moins l'un de $R^4$ à $R^8$ est un groupe acyloxy ou trialkylsiloxy, les autres substituants étant tels que définis ci-dessus,

faire réagir le produit ainsi obtenu avec le composé de formule générale (III) :

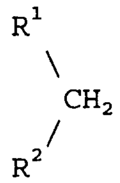

(III)

dans laquelle $R^1$ et $R^2$ sont tels que définis ci-dessus ; et si nécessaire, déprotéger les groupes hydroxy.

**10.** Procédé pour la préparation de dérivés de styrène ayant la formule générale suivante (Ic) :

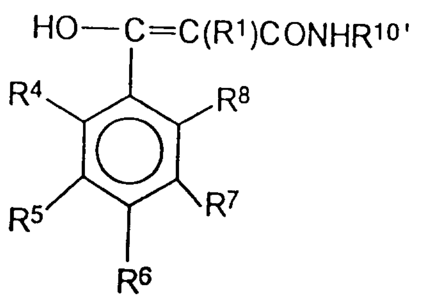

(Ic)

dans laquelle $R^1$ représente un groupe cyano ou un groupe $CONR^9R^{10}$ [où $R^9$ et $R^{10}$ représentent chacun indépendamment un atome d'hydrogène, un groupe alkyle en $C_1$-$C_5$ ou un groupe de formule :

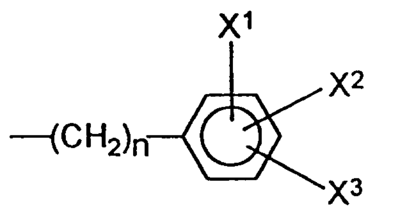

(dans laquelle n vaut 0 ou un nombre entier de 1 à 5, et $X^1$, $X^2$ et $X^3$ représentent chacun indépendamment un atome d'hydrogène, un atome d'halogène, un groupe alkyle en $C_1$-$C_5$, un groupe alcoxy en $C_1$-$C_5$, un groupe hydroxy, un groupe nitro ou un groupe cyano), à condition que $R^9$ et $R^{10}$ ne représentent pas en même temps un atome d'hydrogène] ou un groupe $-COR^{11}$ [où $R^{11}$ représente un groupe de formule :

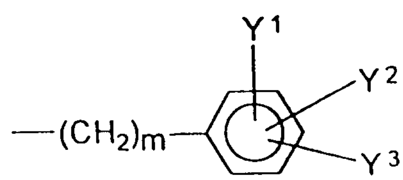

(dans laquelle m vaut 0 ou un nombre entier de 1 à 5, et $Y^1$, $Y^2$ et $Y^3$ représentent chacun indépendamment un atome d'hydrogène, un groupe alkyle en $C_1$-$C_5$, un groupe alcoxy en $C_1$-$C_5$, un groupe hydroxy ou un atome d'halogène)] ;

$R^4$, $R^5$, $R^6$, $R^7$ et $R^8$ représentent chacun indépendamment un atome d'hydrogène, un groupe hydroxy, un atome d'halogène, un groupe alcoxy en $C_1$-$C_5$, un groupe nitro, un groupe cyano ;

un groupe $-NR^{12}R^{13}$ [où $R^{12}$ et $R^{13}$ représentent chacun indépendamment un atome d'hydrogène, un groupe alkyle en $C_1$-$C_5$ ou un groupe benzoyle] ;

un groupe $-SO_pR^{14}$ [où p vaut 0, 1 ou 2, et $R^{14}$ représente un groupe alkyle en $C_1$-$C_5$ ou un groupe phényle] ;

un groupe $-COR^{15}$ [où $R^{15}$ représente un atome d'hydrogène, un groupe alkyle en $C_1$-$C_5$, un groupe alcoxy en $C_1$-$C_5$, un groupe hydroxy, un groupe phényle, un groupe phénoxy ou un groupe $-NHR^{16}$ (où $R^{16}$ représente un groupe

alkyle en $C_1$-$C_5$ ou un groupe phényle)],
ou encore un groupe alkyle en $C_1$-$C_5$ éventuellement substitué par un atome d'halogène, et $R^{10'}$ représente un groupe de formule :

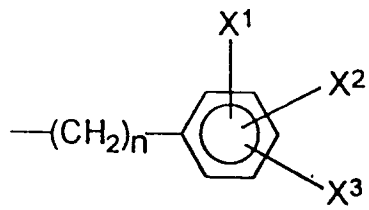

dans laquelle n, $X^1$, $X^2$ et $X^3$ sont tels que définis ci-dessus, à condition qu'au moins l'un de $R^4$ à $R^8$ représente un groupe hydroxy,
comprenant les étapes consistant à :
protéger le groupe hydroxy dans un composé de formule générale (V) :

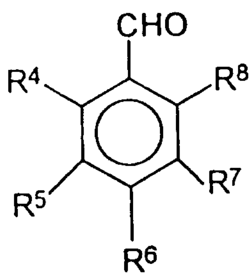

(V)

dans laquelle $R^4$ à $R^8$ ont les mêmes significations que ci-dessus ;
faire réagir avec un composé de formule suivante (VI) :

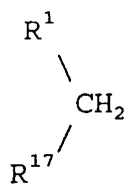

(VI)

dans laquelle $R^1$ a les mêmes significations que ci-dessus et $R^{17}$ représente l'hydrogène ou un groupe carboxy ;
oxyder le produit ainsi obtenu pour obtenir un composé de formule générale (VII) :

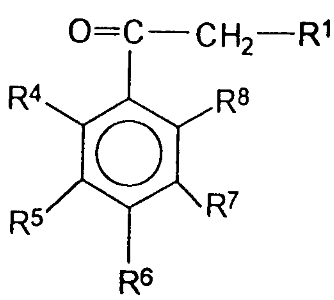

(VII)

dans laquelle $R^4$ à $R^8$ sont tels que définis ci-dessus ;
faire réagir le composé (VII) avec un composé de formule :

$R^{10'}$-N=C=O

dans laquelle $R^{10'}$ est tel que défini ci-dessus ; et
si nécessaire, déprotéger les groupes hydroxy.

11. Procédé pour la préparation de dérivés du styrène de formule générale suivante (Id) :

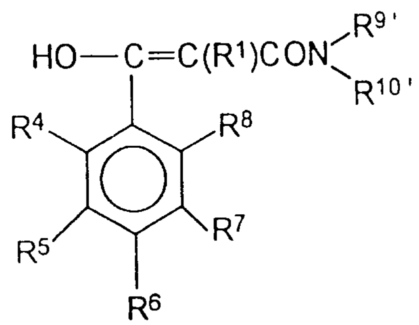

(Id)

dans laquelle $R^1$ représente un groupe cyano, un groupe $CONR^9R^{10}$ [où $R^9$ et $R^{10}$ représentent chacun indépendamment un atome d'hydrogène, un groupe alkyle en $C_1$-$C_5$ ou un groupe de formule :

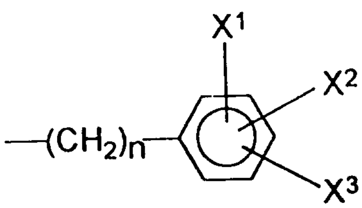

(dans laquelle n vaut 0 ou un nombre entier de 1 à 5, et $X^1$, $X^2$ et $X^3$ représentent chacun indépendamment un atome d'hydrogène, un atome d'halogène, un groupe alkyle en $C_1$-$C_5$, un groupe alcoxy en $C_1$-$C_5$, un groupe hydroxy, un groupe nitro ou un groupe cyano), à condition que $R^9$ et $R^{10}$ ne représentent pas en même temps un atome d'hydrogène] ou un groupe $-COR^{11}$ [où $R^{11}$ représente un groupe de formule :

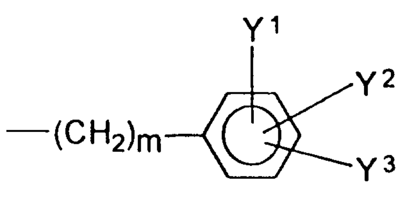

(dans laquelle m vaut 0 ou un nombre entier de 1 à 5, et $Y^1$, $Y^2$ et $Y^3$ représentent chacun indépendamment un atome d'hydrogène, un groupe alkyle en $C_1$-$C_5$, un groupe alcoxy en $C_1$-$C_5$, un groupe hydroxy ou un atome d'halogène)] ;
$R^4$, $R^5$, $R^6$, $R^7$ et $R^8$ représentent chacun indépendamment un atome d'hydrogène, un groupe hydroxy, un atome d'halogène, un groupe alcoxy en $C_1$-$C_5$, un groupe nitro, un groupe cyano ;
un groupe $-NR^{12}R^{13}$ [où $R^{12}$ et $R^{13}$ représentent chacun indépendamment un atome d'hydrogène, un groupe alkyle en $C_1$-$C_5$ ou un groupe benzoyle] ;
un groupe $-SO_pR^{14}$ [où p vaut 0, 1 ou 2, et $R^{14}$ représente un groupe alkyle en $C_1$-$C_5$ ou un groupe phényle] ;
un groupe $-COR^{15}$ [où $R^{15}$ représente un atome d'hydrogène, un groupe alkyle en $C_1$-$C_5$, un groupe alcoxy en $C_1$-$C_5$, un groupe hydroxy, un groupe phényle, un groupe phénoxy ou un groupe $-NHR^{16}$ (où $R^{16}$ représente un groupe alkyle en $C_1$-$C_5$ ou un groupe phényle)],
ou encore un groupe alkyle en $C_1$-$C_5$ éventuellement substitué par un atome d'halogène, et $R^{9'}$ représente un groupe alkyle en $C_1$-$C_5$ et $R^{10'}$ représente un groupe de formule :

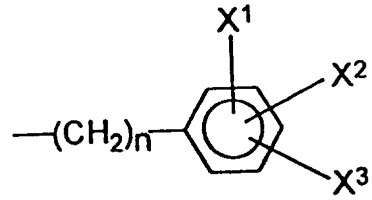

dans laquelle n, $X^1$, $X^2$ et $X^3$ sont tels que définis ci-dessus à condition qu'au moins l'un de $R^4$ à $R^8$ représente un groupe hydroxy,
comprenant les étapes consistant à :
protéger le groupe hydroxy dans un composé de formule générale (V) :

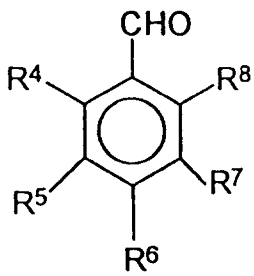

(V)

dans laquelle $R^4$ à $R^8$ ont les mêmes significations que ci-dessus ;
faire réagir avec un composé de formule suivante (VI) :

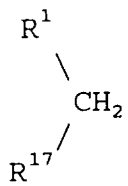

(VI)

dans laquelle $R^1$ a la même signification que ci-dessus et $R^{17}$ représente l'hydrogène ou un groupe carboxy,
oxyder le produit ainsi obtenu pour obtenir un composé de formule générale (VII) :

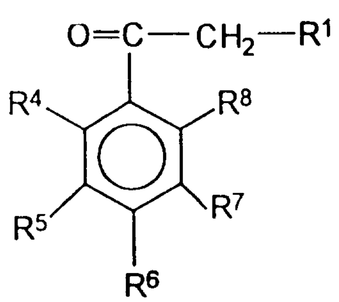

(VII)

dans laquelle $R^4$ à $R^8$ sont tels que définis ci-dessus ;
faire réagir le composé (VII) avec un composé de formule :

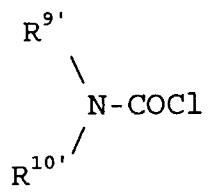

dans laquelle $R^{9'}$ et $R^{10'}$ sont tels que définis ci-dessus ; et si nécessaire, déprotéger les groupes hydroxy.